# EUROPEAN PATENT APPLICATION

(11) **EP 3 438 091 A1**
(43) Date of publication of application: **06.02.2019**
(21) Application number: 17775360.5
(22) Date of filing: 30.03.2017
(51) Int. Cl.: C07C 311/21, A61K 31/18, A61K 31/275, A61K 31/341, A61K 31/381, A61K 31/4045, A61K 31/4196, A61K 31/4245, A61K 45/00, A61P 3/04, A61P 3/10, A61P 43/00, C07C 321/14, C07C 335/20, C07D 209/20, C07D 213/50, C07D 213/74, C07D 249/12, C07D 271/06, C07D 295/135, C07D 307/52

(54) **COMPOUND HAVING ENHANCING ACTIVITY FOR GLUCAGON-LIKE PEPTIDE-1 RECEPTOR ACTIONS**

(30) Priority: 30.03.2016 JP 2016067130
(71) Applicant: AJINOMOTO CO., INC., Chuo-ku Tokyo 104-8315 (JP)
(72) Inventor: FUJII, Tomohiro, Kawasaki-shi Kanagawa 210-8681 (JP); OHSUMI, Koji, Kawasaki-shi Kanagawa 210-8681 (JP); KITAJIMA, Seiji, Kawasaki-shi Kanagawa 210-8681 (JP); KITAHARA, Yoshiro, Kawasaki-shi Kanagawa 210-8681 (JP)
(74) Representative: Mewburn Ellis LLP
(86) International application number: PCT/JP2017/013158
(87) International publication number: WO 2017/170826

(57) **Abstract**

The present invention aims to provide a compound useful for the prophylaxis or treatment of diabetes and obesity, and diseases related thereto. The present invention relates to a glucagon-like peptide-1 receptor action enhancer containing a compound represented by the formula (I): 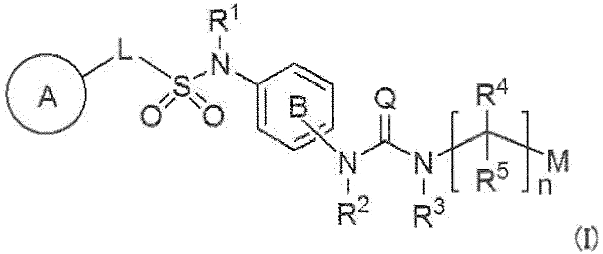 wherein each symbol is as defined in the present specification, or a salt thereof.

## Description

### [Technical Field]

The present invention relates to a compound having a glucagon-like peptide -1 (hereinafter sometimes to be referred to as "GLP-1") receptor action enhancing activity. The present invention also relates to a pharmaceutical composition (prophylactic or therapeutic agent) containing the compound and useful for the prophylaxis or treatment of diabetes or obesity, or a disease related thereto.

### [Background Art]

GLP-1 is an incretin hormone released from L cells in the lower small intestine after food intake. GLP-1 shows an action to increase pancreatic β cell amount by promoting insulin secretion from pancreatic B cells in a glucose dependent manner. GLP-1 also shows an action to promote satiety by decreasing the stomach content discharge rate. On the other hand, GLP-1 is inactivated by being decomposed rapidly by dipeptidyl peptidase-4 (DPP-4). Therefore, GLP-1 receptor agonists and DPP-4 inhibitors are useful as antidiabetic drugs or antiobesity agents.

However, patients with metabolic syndrome are often GLP-1 secretion deficient and sufficient clinical effect may not be achieved due to DPP-4 inhibition. In addition, GLP-1 receptor agonists are useful as antidiabetic drugs or antiobesity agents. Being peptide preparations, however, they are injections and have low compliance.

Non-patent document 1 discloses (4-(3-benzyloxyphenyl)-2-ethylsulfinyl-6-(trifluoromethyl)pyrimidine (BETP) as a compound enhancing a GLP-1 receptor action.

Compounds having structures similar to the structure of the compound described in the present specification are disclosed in patent documents 1 and 2. However, the both documents do not describe that the compounds are GLP-1 receptor action enhancers.

### [Document List]

### [Patent documents]

Patent document 1: WO 2008/109991
Patent document 2: WO 2008/113760

### [Non-patent document]

Non-patent document 1: Molecular Pharmacology, vol.82, p.281-290 (2012)

### [SUMMARY OF THE INVENTION]

### [Problems to be Solved by the Invention]

An object of the present invention is to provide an orally-ingestible prophylactic or therapeutic agent for diabetes or obesity, or a disease related thereto, which is capable of improving a GLP-1 deficiency state due to secretion insufficiency.

### [Means of Solving the Problems]

The present inventors have conducted intensive studies in an attempt to solve the above-mentioned problems and found that a compound represented by the following formula (I) or a salt thereof (hereinafter sometimes to be referred to as "compound (I)" or "the compound of the present invention") has a superior GLP-1 receptor action enhancing activity and is useful for the prophylaxis or treatment of diabetes and/or obesity, which resulted in the completion of the present invention.

Therefore, the present invention provides the following.
[1] A compound represented by the formula (I): 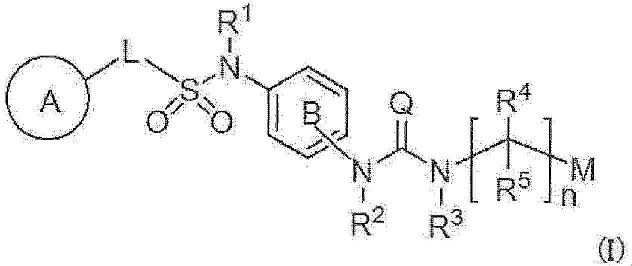 wherein
   A is an optionally further substituted cyclic group;
   B is an optionally further substituted benzene ring;
   L is a bond or an optionally substituted C₁₋₆ alkylene group;
   R¹ is a hydrogen atom, a hydroxy group, an optionally substituted C₁₋₆ alkoxy group, an optionally substituted amino group or an optionally substituted C₁₋₆ alkyl group;
   R² and R³ are the same or different and are each independently a hydrogen atom, a carboxy group, a hydroxy group, an optionally substituted C₁₋₁₀ alkoxy group, an optionally substituted amino group or an optionally substituted C₁₋₁₀ alkyl group;
   Q is an oxygen atom or a sulfur atom;
   R⁴ and R⁵ are the same or different and are each independently
   a hydrogen atom or an optionally substituted C₁₋₆ alkyl group, or R⁴ and R⁵ form a ring together with the carbon atom bonded thereto;
   n is an integer of 1 - 6; and
   M is an optionally substituted C₁₋₁₂ alkoxy-carbonyl group, an optionally substituted carbamoyl group or an optionally substituted heterocyclic group, excluding 3-(3-(3-(phenylsulfonamide)phenyl)ureido)-4-(trimethylammonio)butanoate, or a salt thereof;
[2] the compound of the above-mentioned [1], wherein
   A is a C₆₋₁₄ aryl group or a 5- or 6-membered monocyclic aromatic heterocyclic group, each of which is optionally further substituted,
   L is a bond or a methylene group,
   Q is an oxygen atom,
   R⁴ and R⁵ are the same or different and are each independently
   a hydrogen atom or an optionally substituted C₁₋₆ alkyl group, and
   n is 1 or 2, or a salt thereof;
[3] the compound of the above-mentioned [1], wherein
   A is an optionally further substituted C₆₋₁₄ aryl group,
   L is a bond or a methylene group,
   Q is an oxygen atom,
   R⁴ and R⁵ are the same or different and are each independently
   a hydrogen atom or an optionally substituted C₁₋₆ alkyl group, and
   n is 1 or 2, or a salt thereof;
[4] the compound of the above-mentioned [1], wherein
   A is a phenyl group optionally further substituted by the same or different 1 to 5 substituents selected from the group consisting of a halogen atom, a hydroxy group, a carboxy group, a sulfanyl group, an optionally substituted C₁₋₆ alkyl group, an optionally substituted C₁₋₆ alkoxy group, an optionally substituted amino group, a C₁₋₆ alkylthio group optionally substituted by a halogen atom, and a C₁₋₃ alkylenedioxy group, or a pyridyl group optionally further substituted by the same or different 1 to 4 substituents selected from the group consisting of a halogen atom, a hydroxy group, a carboxy group, a sulfanyl group, an optionally substituted C₁₋₆ alkyl group, an optionally substituted C₁₋₆ alkoxy group, an optionally substituted amino group, a 4- to 7-membered monocyclic non-aromatic heterocyclic group, a C₁₋₆ alkylthio group optionally substituted by a halogen atom, and a C₁₋₃ alkylenedioxy group,
   B is a benzene ring optionally further substituted by the same or different 1 to 4 substituents selected from the group consisting of a halogen atom, a hydroxy group, a sulfanyl group, an optionally substituted C₁₋₆ alkyl group, an optionally substituted C₁₋₆ alkoxy group, an optionally substituted amino group, a C₁₋₆ alkylthio group optionally substituted by a halogen atom, a C₁₋₃ alkylenedioxy group, and a 4- to 7-membered monocyclic non-aromatic heterocyclic group,
   L is a bond or a methylene group,
   R¹ is a hydrogen atom, or a C₁₋₆ alkyl group optionally substituted by a substituent selected from the group consisting of a halogen atom, a hydroxy group, a sulfanyl group, a carboxy group, a C₁₋₆ alkoxy group, an amino group optionally mono- or di-substituted by a C₁₋₆ alkyl group, and a C₁₋₆ alkylthio group optionally substituted by a halogen atom,
   R² and R³ are the same or different and are each independently a hydrogen atom; a carboxy group; a hydroxy group; or a C₁₋₁₀ alkyl group optionally substituted by a substituent selected from the group consisting of a halogen atom, a hydroxy group, a sulfanyl group, a carboxy group, a C₁₋₆ alkoxy group, an amino group optionally mono- or di-substituted by a C₁₋₆ alkyl group, and a C₁₋₆ alkylthio group optionally substituted by a halogen atom,
   Q is an oxygen atom,
   R⁴ is a hydrogen atom,
   R⁵ is a C₁₋₆ alkyl group optionally substituted by a substituent selected from the group consisting of a halogen atom, a hydroxy group, a carboxy group, a guanidino group, a cyano group, a sulfanyl group, an optionally substituted amino group, an optionally substituted carbamoyl group, a C₁₋₆ alkoxy-carbonyl group optionally substituted by a halogen atom, an optionally substituted C₁₋₆ alkoxy group, a C₁₋₆ alkylthio group optionally substituted by a halogen atom, an optionally substituted C₃₋₁₀ cycloalkyl group, and an optionally substituted aromatic ring group,
   n is 1, and
   M is an optionally substituted C₁₋₁₂ alkoxy-carbonyl group, an optionally substituted carbamoyl group or an optionally substituted 5- or 6-membered monocyclic aromatic heterocyclic group, or a salt thereof;
[5] the compound of the above-mentioned [1], wherein A is a phenyl group optionally further substituted by the same or different 1 to 5 substituents selected from the group consisting of a halogen atom, a hydroxy group, a carboxy group, a sulfanyl group, an optionally substituted C₁₋₆ alkyl group, an optionally substituted C₁₋₆ alkoxy group, an optionally substituted amino group, a C₁₋₆ alkylthio group optionally substituted by a halogen atom, and a C₁₋₃ alkylenedioxy group, B is a benzene ring optionally further substituted by the same or different 1 to 4 substituents selected from the group consisting of a halogen atom, a hydroxy group, a sulfanyl group, an optionally substituted C₁₋₆ alkyl group, an optionally substituted C₁₋₆ alkoxy group, an optionally substituted amino group, a C₁₋₆ alkylthio group optionally substituted by a halogen atom, and a C₁₋₃ alkylenedioxy group,
   L is a bond,
   R¹ is a hydrogen atom, or a C₁₋₆ alkyl group optionally substituted by a substituent selected from the group consisting of a halogen atom, a hydroxy group, a sulfanyl group, a carboxy group, a C₁₋₆ alkoxy group, an amino group optionally mono- or di-substituted by a C₁₋₆ alkyl group, and a C₁₋₆ alkylthio group optionally substituted by a halogen atom,
   R² and R³ are the same or different and are each independently a hydrogen atom; a carboxy group; a hydroxy group; or a C₁₋₁₀ alkyl group optionally substituted by a substituent selected from the group consisting of a halogen atom, a hydroxy group, a sulfanyl group, a carboxy group, a C₁₋₆ alkoxy group, an amino group optionally mono- or di-substituted by a C₁₋₆ alkyl group, and a C₁₋₆ alkylthio group optionally substituted by a halogen atom,
   Q is an oxygen atom,
   R⁴ is a hydrogen atom,
   R⁵ is a C₁₋₆ alkyl group optionally substituted by a substituent selected from the group consisting of a halogen atom, a hydroxy group, a carboxy group, a guanidino group, a cyano group, a sulfanyl group, an optionally substituted amino group, an optionally substituted carbamoyl group, a C₁₋₆ alkoxy-carbonyl group optionally substituted by a halogen atom, an optionally substituted C₃₋₁₀ cycloalkyl group, an optionally substituted C₁₋₆ alkoxy group, an optionally substituted aromatic ring group, and a C₁₋₆ alkylthio group optionally substituted by a halogen atom,
   n is 1, and
   M is an optionally substituted C₁₋₁₂ alkoxy-carbonyl group, an optionally substituted carbamoyl group or an optionally substituted 5- or 6-membered monocyclic aromatic heterocyclic group, or a salt thereof;
[6] the compound of the above-mentioned [1], wherein
   A is a phenyl group optionally further substituted by a C₁₋₆ alkyl group optionally substituted by 1 to 3 substituents selected from the group consisting of a halogen atom, a carboxy group, a hydroxy group, a C₁₋₆ alkoxy-carbonyl group, a C₁₋₆ alkoxy group, an amino group optionally mono- or di-substituted by a C₁₋₆ alkyl group, and a cyano group, or a pyridyl group optionally further substituted by the same or different 1 to 3 substituents selected from the group consisting of an amino group optionally mono- or di-substituted by a C₁₋₆ alkyl group and a 4- to 7-membered monocyclic non-aromatic nitrogen-containing heterocyclic group,
   B is a benzene ring optionally further substituted by the same or different 1 to 4 substituents selected from the group consisting of a halogen atom, a C₁₋₆ alkoxy group, and a 4- to 7-membered monocyclic non-aromatic heterocyclic group,
   L is a bond,
   R¹ is a hydrogen atom, or a C₁₋₆ alkyl group substituted by a C₁₋₆ alkylthio group optionally substituted by a halogen atom,
   R² and R³ are the same or different and are each independently a hydrogen atom, or a C₁₋₁₀ alkyl group optionally substituted by a carboxy group,
   Q is an oxygen atom,
   R⁴ is a hydrogen atom,
   R⁵ is a C₁₋₆ alkyl group optionally substituted by a substituent selected from the group consisting of a halogen atom, a hydroxy group, a carboxy group, a guanidino group, a cyano group, a sulfanyl group, an optionally substituted amino group, an optionally substituted carbamoyl group, a C₁₋₆ alkoxy-carbonyl group, an optionally substituted C₁₋₆ alkoxy group, a C₁₋₆ alkylthio group optionally substituted by a halogen atom, an optionally substituted C₃₋₁₀ cycloalkyl group, and an optionally substituted aromatic ring group,
   n is 1, and
   M is an optionally substituted C₁₋₁₂ alkoxy-carbonyl group; a carbamoyl group optionally mono- or di-substituted by a substituent selected from the group consisting of an optionally substituted C₁₋₈ alkyl group and a C₃₋₁₀ cycloalkyl group; or an optionally substituted 5- or 6-membered monocyclic aromatic heterocyclic group, or a salt thereof;
[7] the compound of the above-mentioned [1], wherein
   A is a phenyl group optionally further substituted by a C₁₋₆ alkyl group optionally substituted by 1 to 3 halogen atoms, B is a benzene ring optionally further substituted by the same or different 1 to 4 substituents selected from the group consisting of a halogen atom and a C₁₋₆ alkoxy group,
   L is a bond,
   R¹ is a hydrogen atom,
   R² and R³ are the same or different and are each independently a hydrogen atom or a C₁₋₁₀ alkyl group,
   Q is an oxygen atom,
   R⁴ is a hydrogen atom,
   R⁵ is a C₁₋₆ alkyl group optionally substituted by a substituent selected from the group consisting of a halogen atom, a hydroxy group, a carboxy group, a guanidino group, a cyano group, a sulfanyl group, an optionally substituted amino group, an optionally substituted carbamoyl group, a C₁₋₆ alkoxy-carbonyl group, an optionally substituted C₃₋₁₀ cycloalkyl group, an optionally substituted C₁₋₆ alkoxy group, an optionally substituted aromatic ring group, and a C₁₋₆ alkylthio group optionally substituted by a halogen atom,
   n is 1, and
   M is an optionally substituted C₁₋₁₂ alkoxy-carbonyl group, an optionally substituted carbamoyl group, an optionally substituted triazolyl group, an optionally substituted isoxazolyl group, an optionally substituted oxazolyl group, an optionally substituted oxadiazolyl group or an optionally substituted tetrazolyl group, or a salt thereof;
[8] the compound of the above-mentioned [1], wherein
   A is a phenyl group optionally further substituted by a C₁₋₆ alkyl group optionally substituted by 1 to 3 halogen atoms,
   B is a benzene ring without a further substituent,
   L is a bond,
   R¹ is a hydrogen atom,
   R² and R³ are the same or different and are each independently
   a hydrogen atom or a C₁₋₁₀ alkyl group,
   Q is an oxygen atom,
   R⁴ is a hydrogen atom,
   R⁵ is a C₁₋₄ alkyl group optionally substituted by a substituent selected from the group consisting of an optionally substituted C₃₋₁₀ cycloalkyl group, an optionally substituted phenyl group, a C₁₋₆ alkylthio group, a 5- or 6-membered monocyclic aromatic nitrogen-containing heterocyclic group, and a 8- to 14-membered fused aromatic nitrogen-containing heterocyclic group,
   n is 1, and
   M is an optionally substituted C₁₋₁₂ alkoxy-carbonyl group, an optionally substituted carbamoyl group, an optionally substituted triazolyl group, an optionally substituted isoxazolyl group, an optionally substituted oxazolyl group, an optionally substituted oxadiazolyl group or an optionally substituted tetrazolyl group, or a salt thereof;
[9] the compound of the above-mentioned [1], wherein
   A is a phenyl group optionally further substituted by 1 to 3 C₁₋₆ alkyl groups,
   B is a benzene ring without a further substituent,
   L is a bond,
   R¹ is a hydrogen atom,
   R² and R³ are the same or different and are each independently
   a hydrogen atom or a C₁₋₁₀ alkyl group,
   Q is an oxygen atom,
   R⁴ is a hydrogen atom,
   R⁵ is a C₁₋₄ alkyl group optionally substituted by a substituent selected from the group consisting of an optionally substituted C₃₋₁₀ cycloalkyl group, an optionally substituted phenyl group,
   a C₁₋₆ alkylthio group, a 5- or 6-membered monocyclic aromatic nitrogen-containing heterocyclic group, and a 8- to 14-membered fused aromatic nitrogen-containing heterocyclic group,
   n is 1, and
   M is a carbamoyl group mono-substituted by a C₁₋₁₂ alkyl group or an optionally substituted oxadiazolyl group, or a salt thereof;
[10] the compound of the above-mentioned [1], wherein
   A is a phenyl group optionally further substituted by 1 to 3 C₁₋₆ alkyl groups,
   B is a benzene ring without a further substituent,
   L is a bond,
   R¹ is a hydrogen atom,
   R² and R³ are the same or different and are each independently
   a hydrogen atom, or a C₁₋₁₀ alkyl group,
   Q is an oxygen atom,
   R⁴ is a hydrogen atom,
   R⁵ is a C₁₋₄ alkyl group optionally substituted by a substituent selected from the group consisting of an optionally substituted C₃₋₁₀ cycloalkyl group, a C₁₋₆ alkylthio group, an optionally substituted phenyl group, and an optionally substituted aromatic heterocyclic group,
   n is 1, and
   M is a carbamoyl group optionally mono- or di-substituted by a C₁₋₁₂ alkyl group, or an optionally substituted oxadiazolyl group, or a salt thereof;
[11] the compound of any of the above-mentioned [1] to [10], wherein the group -N(R²)- binds to B at a para-position relative to the binding position of the group -N(R¹)-, or a salt thereof;
[12] a glucagon-like peptide-1 receptor action enhancer comprising the compound of any of the above-mentioned [1] to [11] or a salt thereof;
[13] a pharmaceutical composition comprising the compound of any of the above-mentioned [1] to [11] or a salt thereof, and a pharmaceutically acceptable carrier;
[14] the pharmaceutical composition of the above-mentioned [13], wherein the composition is a prophylactic or therapeutic agent for diabetes, obesity and/or a complication thereof;
[15] the pharmaceutical composition of the above-mentioned [14], further comprising at least one kind of medicament selected from the group consisting of the dipeptidyl peptidase-4 inhibitor, insulin secretagogue, α-glucosidase inhibitor, insulin resistance improving agent, sodium.glucose conjugated transporter-2 inhibitor, glucagon-like peptide-1 receptor agonist and lipase inhibitor;
   and the like.

### [Effect of the Invention]

According to the present invention, a prophylactic or therapeutic agent for diabetes and/or obesity, which has a superior GLP-1 receptor action enhancing activity, is applicable to a wide range of patient groups, and can be administered orally, can be provided.

### [Brief Description of the Drawings]

Fig. 1 shows the results of the glucose-stimulated insulin secretion promoting action of the compound of the present invention on isolated rat islets by GLP-1.
Fig. 2A shows the results of the blood glucose elevation suppressive action of the compound of the present invention using diabetes model mouse.
Fig. 2B shows the results of the blood glucose AUC value suppressive action of the compound of the present invention using diabetes model mouse for 180 min after glucose administration.
Fig. 3 shows the results of the food intake-suppressive effects of the compound of the present invention using diabetes model mouse.

### [Description of Embodiments]

Each symbol in the formula (I) is defined in detail below.

The "halogen atom" in the present specification means, unless otherwise specified, fluorine atom, chlorine atom, bromine atom or iodine atom.

The "C₁₋₆ alkyl (group)" in the present specification means, unless otherwise specified, a linear or branched alkyl group having 1 - 6 carbon atoms such as methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl and the like.

The "C₁₋₁₀ alkyl (group)" in the present specification means, unless otherwise specified, a linear or branched alkyl group having 1 - 10 carbon atoms such as methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, heptyl, octyl, nonyl, decyl and the like.

The "C₁₋₁₂ alkyl (group)" in the present specification means, unless otherwise specified, a linear or branched alkyl group having 1 - 12 carbon atoms such as methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, heptyl, octyl, nonyl, decyl, undecyl, dodecyl and the like.

The "C₁₋₆ alkylene (group)" in the present specification means, unless otherwise specified, a linear or branched alkylene group having 1 - 6 carbon atoms such as methylene, ethylene, propylene, butylene, pentylene, neopentylene, hexylene and the like.

The "C₂₋₆ alkenyl (group)" in the present specification means, unless otherwise specified, a linear or branched alkenyl group having 2 - 6 carbon atoms such as ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, 5-hexenyl and the like.

The "C₂₋₁₀ alkenyl (group)" in the present specification means, unless otherwise specified, a linear or branched alkenyl group having 2 - 10 carbon atoms such as ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, 5-hexenyl, 1-heptenyl, 1-octenyl and the like.

The "C₂₋₆ alkynyl (group)" in the present specification means, unless otherwise specified, a linear or branched alkynyl group having 2 - 6 carbon atoms such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl and the like.

The "C₁₋₆ alkoxy (group)" in the present specification means, unless otherwise specified, a linear or branched alkoxy group having 1 - 6 carbon atoms such as methoxy, ethoxy, propoxy, isopropoxy, butoxy, isobutoxy, sec-butoxy, tert-butoxy and the like.

The "C₁₋₁₂ alkoxy-carbonyl (group)" in the present specification means, unless otherwise specified, a group in which a linear or branched alkoxy group having 1 - 12 carbon atoms is bonded to a carbonyl group, such as methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, hexyloxycarbonyl, heptyloxycarbonyl, octyloxycarbonyl, nonyloxycarbonyl, decyloxycarbonyl, dodecyloxycarbonyl and the like.

The "C₁₋₆ alkylthio (group)" in the present specification means, unless otherwise specified, a linear or branched alkylthio group having 1 - 6 carbon atoms such as methylthio, ethylthio, propylthio, isopropylthio, butylthio, isobutylthio, sec-butylthio, tert-butylthio and the like.

Examples of the "cyclic group" of the "optionally substituted cyclic group" for A include C₃₋₁₀ cycloalkyl group, C₃₋₁₀ cycloalkenyl group, C₄₋₁₀ cycloalkadienyl group, C₆₋₁₄ aryl group, heterocyclic group and the like, each of which optionally has the same or different one or more substituents at substitutable position(s).

In the present specification, examples of the "C₃₋₁₀ cycloalkyl (group)" include cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl, adamantyl and the like.

In the present specification, examples of the "C₃₋₁₀ cycloalkenyl (group)" include cyclopropenyl (e.g., 2-cyclopropen-1-yl), cyclobutenyl (e.g., 2-cyclobuten-1-yl), cyclopentenyl (e.g., 2-cyclopenten-1-yl, 3-cyclopenten-1-yl), cyclohexenyl (e.g., 2-cyclohexen-1-yl, 3-cyclohexen-1-yl) and the like.

In the present specification, examples of the "C₄₋₁₀ cycloalkadienyl (group)" include 2,4-cyclopentadien-1-yl, 2,4-cyclohexadien-1-yl, 2,5-cyclohexadien-1-yl and the like.

In the present specification, examples of the "C₆₋₁₄ aryl (group)" include phenyl, naphthyl, anthryl, phenanthryl, acenaphthyl, biphenylyl and the like. The "C₆₋₁₄ aryl (group)" may be condensed with other ring and, for example, fluorenyl, dihydronaphthyl, tetrahydronaphthyl and the like can be mentioned. Of these, C₆₋₁₀ aryl group is preferable, and phenyl is particularly preferable.

In the present specification, examples of the "C₇₋₁₆ aralkyl (group)" include benzyl, phenethyl, naphthylmethyl, biphenylylmethyl and the like. Of these, C₇₋₁₀ aralkyl group is preferable, and benzyl group is particularly preferable.

In the present specification, examples of the "heterocycle (group)" include aromatic heterocyclic group and non-aromatic heterocyclic group.

In the present specification, examples of the "aromatic heterocyclic group" include a 4- to 7-membered (preferably 5- or 6-membered) monocyclic aromatic heterocyclic group and a fused aromatic heterocyclic group, each containing, as a ring-constituting atom besides carbon atom, 1 to 4 hetero atoms selected from an oxygen atom, a sulfur atom and a nitrogen atom. Examples of the fused aromatic heterocyclic group include a group induced from a ring in which a ring corresponding to these 4- to 7-membered monocyclic aromatic heterocyclic groups is condensed with 1 or 2 selected from a 5- or 6-membered monocyclic aromatic heterocycle containing 1 or 2 nitrogen atoms (e.g., pyrrole, imidazole, pyrazole, pyrazine, pyridine, pyrimidine etc.), a 5-membered aromatic heterocycle containing one sulfur atom (e.g., thiophene) and a benzene ring and the like.

Preferable examples of the aromatic heterocyclic group include 5- or 6-membered monocyclic aromatic heterocyclic groups such as furyl, thienyl, pyridyl, pyrimidinyl, pyridazinyl, pyrazinyl, pyrrolyl, imidazolyl, pyrazolyl, thiazolyl, isothiazolyl, oxazolyl, isoxazolyl, oxadiazolyl, thiadiazolyl, triazolyl, tetrazolyl, triazinyl and the like; 8- to 14-membered fused aromatic heterocyclic groups such as quinolyl, isoquinolyl, quinazolyl, quinoxalyl, benzofuranyl, benzothienyl, benzoxazolyl, benzisoxazolyl, benzothiazolyl, benzimidazolyl, benzotriazolyl, indolyl, indazolyl, carbazolyl, pyrrolopyrazinyl, imidazopyridyl, thienopyridyl, imidazopyrazinyl, pyrazolopyridyl, pyrazolothienyl, pyrazolotriazinyl, pyridopyridyl, thienopyridyl and the like; and the like.

In the present specification, examples of the "non-aromatic heterocyclic group" include a 4- to 7-membered (preferably 5- or 6-membered) monocyclic non-aromatic heterocyclic group and a fused non-aromatic heterocyclic group, and 7- to 10-membered bridged heterocyclic group, each containing, as a ring constituting atom besides carbon atom, 1 to 4 hetero atoms selected from an oxygen atom, a sulfur atom and a nitrogen atom. Examples of the fused non-aromatic heterocyclic group include a group induced from a ring in which a ring corresponding to these 4- to 7-membered monocyclic non-aromatic heterocyclic groups is condensed with 1 or 2 rings selected from a 5- or 6-membered monocyclic aromatic heterocycle containing 1 or 2 nitrogen atoms (e.g., pyrrole, imidazole, pyrazole, pyrazine, pyridine, pyrimidine etc.), a 5-membered monocyclic aromatic heterocycle containing one sulfur atom (e.g., thiophene) and a benzene ring, a group obtained by partial saturation of the above group, and the like.

Preferable examples of the non-aromatic heterocyclic group include
4- to 7-membered monocyclic non-aromatic heterocyclic groups such as azetidinyl, pyrrolidinyl, piperidyl, morpholinyl, thiomorpholinyl, piperazinyl, hexamethyleniminyl, oxazolidinyl, thiazolidinyl, imidazolidinyl, oxazolinyl, thiazolinyl, imidazolinyl, dioxolyl, dioxolanyl, dihydrooxadiazolyl, pyranyl, tetrahydropyranyl, thiopyranyl, tetrahydrothiopyranyl, tetrahydrofuryl, pyrazolidinyl, pyrazolinyl, tetrahydropyrimidinyl, dihydrotriazolyl, tetrahydrotriazolyl and the like;
9- to 14-membered fused non-aromatic heterocyclic groups such as dihydroindolyl, dihydroisoindolyl, dihydrobenzofuranyl, dihydrobenzodioxinyl, dihydrobenzodioxepinyl, tetrahydrobenzofuranyl, chromenyl, dihydrochromenyl, dihydroquinolyl, tetrahydroquinolyl, dihydroisoquinolyl, tetrahydroisoquinolyl, dihydrophthalazinyl and the like; and the like.

In the present specification, preferable examples of the "7- to 10-membered bridged heterocyclic group" include quinuclidinyl and 7-azabicyclo[2.2.1]heptanyl.

In the present specification, examples of the "nitrogen-containing heterocyclic group" include a "heterocyclic group" containing at least one nitrogen atom as a ring-constituting atom.

In the present specification, examples of the "aromatic ring (group)" include the aforementioned C₆₋₁₄ aryl group and aromatic heterocyclic group.

In the present specification, examples of the "optionally substituted amino (group)" include an amino group optionally mono- or di-substituted by substituent(s) selected from C₁₋₁₀ alkyl group, C₂₋₁₀ alkenyl group, C₃₋₁₀ cycloalkyl group, C₃₋₁₀ cycloalkenyl group, C₆₋₁₄ aryl group, C₇₋₁₆ aralkyl group, heterocyclic group, acyl group and the like, each of which is optionally substituted, and the like.

In the present specification, examples of the "acyl group" exemplified as the substituent of the "optionally substituted amino (group)" include linear or branched C₁₋₁₂ alkanoyl group, C₇₋₁₃ aroyl group, C₁₋₆ alkoxy-carbonyl group, C₃₋₁₀ cycloalkyl-carbonyl group, C₃₋₁₀ cycloalkyloxy-carbonyl group, C₇₋₁₆ aralkyl-carbonyl group, C₇₋₁₆ aralkyloxy-carbonyl group, C₆₋₁₀ aryloxy-carbonyl group, heterocyclylcarbonyl group, carbamoyl group (-CONH₂), mono or di-C₁₋₆ alkyl-carbamoyl group, mono or di-C₃₋₁₀ cycloalkyl-carbamoyl group, mono or di-heterocyclylcarbamoyl group, C₁₋₁₂ alkylsulfonyl group, C₆₋₁₀ arylsulfonyl group, heterocyclylsulfonyl group, thiocarbamoyl group (-CSNH₂), mono or di-C₁₋₆ alkyl-thiocarbamoyl group, mono or di-C₃₋₁₀ cycloalkyl-thiocarbamoyl group, sulfamoyl group (-S(O)₂NH₂), mono or di-C₁₋₆ alkylsulfamoyl group, mono or di-C₃₋₁₀ cycloalkylsulfamoyl group and the like. These are each optionally further substituted by C₁₋₆ alkyl group, C₂₋₆ alkenyl group, C₂₋₆ alkynyl group, C₃₋₈ cycloalkyl group, C₃₋₈ cycloalkenyl group, C₄₋₈ cycloalkadienyl group, C₆₋₁₄ aryl group, C₇₋₁₆ aralkyl group, heterocyclic group, halogen atom, hydroxy group, carboxy group, amino group, carbamoyl group, cyano group, nitro group, oxo group or the like.

Preferable examples of the "acyl group" include optionally substituted C₁₋₆ alkanoyl groups (e.g., formyl, acetyl, trifluoroacetyl, n-propionyl, isopropionyl, n-butyryl, isobutyryl, pivaloyl, valeryl, hexanoyl etc.), optionally substituted C₁₋₆ alkoxy-carbonyl groups (e.g., methoxycarbonyl, ethoxycarbonyl etc.), optionally substituted C₃₋₁₀ cycloalkyl-carbonyl groups (e.g., cyclopentylcarbonyl, cyclohexylcarbonyl etc.), optionally substituted C₃₋₁₀ cycloalkyloxy-carbonyl group, optionally substituted C₇₋₁₃ aroyl groups (e.g., benzoyl, 1-naphthoyl, 2-naphthoyl etc.), optionally substituted C₇₋₁₆ aralkyloxy-carbonyl groups (e.g., benzyloxycarbonyl, 1-naphthylmethyloxycarbonyl, 2-naphthylmethyloxycarbonyl etc.), optionally substituted di(C₁₋₆ alkyl)carbamoyl groups (e.g., dimethylcarbamoyl etc.), optionally substituted C₁₋₆ alkylsulfonyl groups (e.g., methanesulfonyl, trifluoromethanesulfonyl etc.), optionally substituted C₆₋₁₀ arylsulfonyl groups (e.g., benzenesulfonyl, toluenesulfonyl etc.), optionally substituted di(C₁₋₆ alkyl)sulfamoyl groups (e.g., dimethylsulfamoyl etc.), optionally substituted heterocyclylcarbonyl groups (e.g., pyrrolidylcarbonyl, piperidylcarbonyl, morpholinylcarbonyl, pyridylcarbonyl etc.), optionally substituted heterocyclylsulfonyl groups (e.g., pyrrolidylsulfonyl, piperidylsulfonyl, morpholinylsulfonyl etc.) and the like.

The C₃₋₁₀ cycloalkyl group, C₃₋₁₀ cycloalkenyl group, C₄₋₁₀ cycloalkadienyl group, C₆₋₁₄ aryl group and heterocyclic group exemplified as the aforementioned "cyclic group" for A in the formula (I) optionally have 1 to 5 substituents at substitutable position(s).

Examples of such substituent include
(1) a C₁₋₆ alkyl group optionally substituted by 1 to 3 substituents selected from
   (a) a halogen atom,
   (b) a carboxy group,
   (c) a hydroxy group,
   (d) a C₁₋₆ alkoxy-carbonyl group optionally substituted by a halogen atom,
   (e) a C₁₋₆ alkoxy group optionally substituted by a halogen atom,
   (f) an amino group optionally mono- or di-substituted by a C₁₋₆ alkyl group optionally substituted by a halogen atom, and
   (g) a cyano group;
(2) a C₇₋₁₆ aralkyl group optionally substituted by a halogen atom;
(3) an amino group optionally mono- or di-substituted by a substituent selected from
   (a) a C₁₋₆ alkyl group optionally substituted by a halogen atom,
   (b) a C₁₋₆ alkanoyl group optionally substituted by a halogen atom,
   (c) a C₇₋₁₃ aroyl group optionally substituted by a halogen atom,
   (d) a C₁₋₆ alkylsulfonyl group optionally substituted by a halogen atom,
   (e) a C₆₋₁₀ arylsulfonyl group optionally substituted by a substituent selected from
      (i) a halogen atom, and
      (ii) a C₁₋₆ alkyl group optionally substituted by a halogen atom, and
   (f) a C₁₋₆ alkoxy-carbonyl group optionally substituted by a halogen atom;
(4) a C₁₋₆ alkanoyl group optionally substituted by a halogen atom;
(5) a C₁₋₆ alkoxy-carbonyl group optionally substituted by one or more substituents selected from
   (a) a halogen atom, and
   (b) a C₁₋₆ alkoxy group optionally substituted by a halogen atom;
(6) a C₁₋₆ alkylsulfonyl group optionally substituted by a halogen atom;
(7) a carbamoyl group optionally mono- or di-substituted by a C₁₋₆ alkyl group optionally substituted by a halogen atom;
(8) a carboxy group;
(9) a hydroxy group;
(10) a C₁₋₆ alkoxy group optionally substituted by one or more substituents selected from
   (a) a halogen atom,
   (b) a carboxy group,
   (c) a hydroxy group,
   (d) a C₁₋₆ alkoxy group optionally substituted by a halogen atom,
   (e) a C₁₋₆ alkoxy-carbonyl group optionally substituted by a halogen atom, and
   (f) an amino group optionally mono- or di-substituted by a C₁₋₆ alkyl group optionally substituted by a halogen atom;
(11) a C₇₋₁₆ aralkyloxy group optionally substituted by a halogen atom;
(12) a C₆₋₁₄ aryloxy group optionally substituted by a halogen atom;
(13) a C₁₋₆ alkanoyloxy group optionally substituted by a halogen atom;
(14) a C₆₋₁₄ aroyl group optionally substituted by one or more substituents selected from
   (a) a halogen atom, and
   (b) a C₁₋₆ alkyl group optionally substituted by a halogen atom;
(15) a sulfanyl (SH) group;
(16) a C₁₋₆ alkylthio group optionally substituted by a halogen atom;
(17) a C₇₋₁₆ aralkylthio group (e.g., benzylthio etc.) optionally substituted by a halogen atom;
(18) a C₆₋₁₄ arylthio group (e.g., phenylthio, naphthylthio etc.) optionally substituted by a halogen atom;
(19) a cyano group;
(20) a nitro group;
(21) a halogen atom;
(22) a C₁₋₃ alkylenedioxy group (e.g., methylenedioxy, ethylenedioxy etc.);
(23) a tri C₁₋₆ alkylsilyl group;
(24) a 4- to 7-membered monocyclic non-aromatic heterocyclic group (e.g., monocyclic non-aromatic nitrogen-containing heterocyclic group such as 4-morpholinyl and the like); and the like. When two or more substituents are present, the respective substituents may be the same or different.

The benzene ring for B in the formula (I) optionally has 1 to 4 substituents at substitutable position(s). As such substituent, the groups exemplified as the substituents for the aforementioned "cyclic group" and the like can be mentioned. When two or more substituents are present, the respective substituents may be the same or different.

Examples of the optionally substituted "substituent" of the "optionally substituted C₁₋₆ alkyl group", "optionally substituted C₁₋₆ alkoxy group", "optionally substituted C₁₋₁₀ alkyl group" and "optionally substituted C₁₋₁₀ alkoxy group" in the respective definitions of L, R¹, R² and R³ in the formula (I) include
(1) a C₁₋₆ alkoxy-carbonyl group optionally substituted by a halogen atom;
(2) an amino group optionally mono- or di-substituted by a substituent selected from
   (a) a C₁₋₆ alkyl group optionally substituted by a halogen atom,
   (b) a C₁₋₆ alkanoyl group optionally substituted by a halogen atom,
   (c) a C₇₋₁₃ aroyl group optionally substituted by a halogen atom,
   (d) a C₁₋₆ alkylsulfonyl group optionally substituted by a halogen atom,
   (e) a C₆₋₁₀ arylsulfonyl group optionally substituted by a substituent selected from
      (i) a halogen atom, and
      (ii) a C₁₋₆ alkyl group, and
   (f) a C₁₋₆ alkoxy-carbonyl group optionally substituted by a halogen atom;
(3) a carbamoyl group optionally mono- or di-substituted by a C₁₋₆ alkyl group optionally substituted by a halogen atom;
(4) a carboxy group;
(5) a hydroxy group;
(6) a sulfanyl (SH) group;
(7) a C₁₋₆ alkylthio group optionally substituted by a halogen atom;
(8) a C₁₋₆ alkoxy group optionally substituted by one or more substituents selected from
   (a) a halogen atom,
   (b) a carboxy group,
   (c) a hydroxy group,
   (d) a C₁₋₆ alkoxy group optionally substituted by a halogen atom,
   (e) a C₁₋₆ alkoxy-carbonyl group optionally substituted by a halogen atom, and
   (f) an amino group optionally mono- or di-substituted by a C₁₋₆ alkyl group optionally substituted by a halogen atom;
(9) a cyano group;
(10) a halogen atom;
(11) a C₆₋₁₄ aryl group optionally substituted by one or more substituents selected from
   (a) a C₁₋₆ alkyl group optionally substituted by one or more substituents selected from
      (i) a halogen atom,
      (ii) a carboxy group,
      (iii) a hydroxy group,
      (iv) a C₁₋₆ alkoxy group optionally substituted by a halogen atom,
      (v) an amino group optionally mono- or di-substituted by a C₁₋₆ alkyl group optionally substituted by a halogen atom, and
      (vi) a cyano group;
   (b) an amino group optionally mono- or di-substituted by a substituent selected from
      (i) a C₁₋₆ alkyl group optionally substituted by a halogen atom,
      (ii) a C₁₋₆ alkanoyl group optionally substituted by a halogen atom,
      (iii) a C₇₋₁₃ aroyl group optionally substituted by a halogen atom,
      (iv) a C₁₋₆ alkylsulfonyl group optionally substituted by a halogen atom,
      (v) a C₆₋₁₀ arylsulfonyl group optionally substituted by a substituent selected from
         (I) a halogen atom, and
         (II) a C₁₋₆ alkyl group optionally substituted by a halogen atom, and
      (vi) a C₁₋₆ alkoxy-carbonyl group optionally substituted by a halogen atom,
   (c) a C₁₋₆ alkoxy group optionally substituted by one or more substituents selected from
      (i) a halogen atom,
      (ii) a C₁₋₆ alkoxy group optionally substituted by a halogen atom,
      (iii) a carboxy group, and
      (iv) a hydroxy group,
   (d) a carbamoyl group optionally mono- or di-substituted by a C₁₋₆ alkyl group optionally substituted by a halogen atom,
   (e) a carboxy group,
   (f) a hydroxy group,
   (g) a halogen atom,
   (h) a sulfanyl (SH) group, and
      (i) a C₁₋₆ alkylthio group optionally substituted by a halogen atom,
   and the like. When two or more substituents are present, the respective substituents may be the same or different.

Examples of the optionally substituted "substituent" of the "optionally substituted C₁₋₆ alkyl group" in the respective definitions of R⁴ and R⁵ in the formula (I) include
(1) a halogen atom;
(2) a hydroxy group;
(3) a carboxy group;
(4) a guanidino group;
(5) a cyano group;
(6) a sulfanyl (SH) group;
(7) an amino group optionally mono- or di-substituted by a substituent selected from
   (a) a C₁₋₆ alkyl group optionally substituted by a halogen atom,
   (b) a C₁₋₆ alkanoyl group optionally substituted by a halogen atom,
   (c) a C₇₋₁₃ aroyl group optionally substituted by a halogen atom,
   (d) a C₁₋₆ alkylsulfonyl group optionally substituted by a halogen atom,
   (e) a C₆₋₁₀ arylsulfonyl group optionally substituted by a substituent selected from
      (i) a halogen atom, and
      (ii) a C₁₋₆ alkyl group optionally substituted by a halogen atom, and
   (f) a C₁₋₆ alkoxy-carbonyl group optionally substituted by a halogen atom;
(8) a carbamoyl group optionally mono- or di-substituted by a C₁₋₆ alkyl group optionally substituted by a halogen atom;
(9) a C₁₋₆ alkoxy-carbonyl group optionally substituted by a halogen atom;
(10) a C₁₋₆ alkoxy group optionally substituted by one or more substituents selected from
   (a) a halogen atom,
   (b) a carboxy group,
   (c) a hydroxy group,
   (d) a C₁₋₆ alkoxy group optionally substituted by a halogen atom,
   (e) a C₁₋₆ alkoxy-carbonyl group optionally substituted by a halogen atom, and
   (f) an amino group optionally mono- or di-substituted by a C₁₋₆ alkyl group optionally substituted by a halogen atom;
(11) a C₁₋₆ alkylthio group optionally substituted by a halogen atom;
(12) a C₃₋₁₀ cycloalkyl group (e.g., cyclopropyl, cyclobutyl, cyclopentyl, cyclohexyl etc.) optionally substituted by a substituent selected from
   (a) a halogen atom,
   (b) a hydroxy group,
   (c) a C₁₋₆ alkoxy group optionally substituted by a halogen atom, and
   (d) a C₁₋₆ alkyl group optionally substituted by a halogen atom;
(13) an aromatic ring group (e.g., C₆₋₁₄ aryl group such as phenyl group, naphthyl group and the like; 5- or 6-membered monocyclic aromatic nitrogen-containing heterocyclic group such as imidazolyl group, pyridyl group and the like; 8- to 14-membered fused aromatic nitrogen-containing heterocyclic group such as indolyl group and the like, etc.) optionally substituted by one or more substituents selected from
   (a) a C₁₋₆ alkyl group optionally substituted by one or more substituents selected from
      (i) a halogen atom,
      (ii) a carboxy group,
      (iii) a hydroxy group,
      (iv) a C₁₋₆ alkoxy group optionally substituted by a halogen atom,
      (v) an amino group optionally mono- or di-substituted by a C₁₋₆ alkyl group optionally substituted by a halogen atom, and
      (vi) a cyano group;
   (b) an amino group optionally mono- or di-substituted by a substituent selected from
      (i) a C₁₋₆ alkyl group optionally substituted by a halogen atom,
      (ii) a C₁₋₆ alkanoyl group optionally substituted by a halogen atom,
      (iii) a C₇₋₁₃ aroyl group optionally substituted by a halogen atom,
      (iv) a C₁₋₆ alkylsulfonyl group optionally substituted by a halogen atom,
      (v) a C₆₋₁₀ arylsulfonyl group optionally substituted by a substituent selected from
         (I) a halogen atom, and
         (II) a C₁₋₆ alkyl group optionally substituted by a halogen atom, and
      (vi) a C₁₋₆ alkoxy-carbonyl group optionally substituted by a halogen atom,
   (c) a C₁₋₆ alkoxy group optionally substituted by one or more substituents selected from
      (i) a halogen atom,
      (ii) a C₁₋₆ alkoxy group optionally substituted by a halogen atom,
      (iii) a carboxy group, and
      (iv) a hydroxy group,
   (d) a carbamoyl group optionally mono- or di-substituted by a C₁₋₆ alkyl group optionally substituted by a halogen atom,
   (e) a carboxy group,
   (f) a hydroxy group,
   (g) a halogen atom,
   (h) a sulfanyl (SH) group, and
      (i) a C₁₋₆ alkylthio group optionally substituted by a halogen atom; and the like. When two or more substituents are present, the respective substituents may be the same or different.

In the definition of R⁴ and R⁵ in the formula (I), examples of the ring formed by "R⁴ and R⁵ together with the carbon atom bonded thereto" include optionally substituted C₃₋₁₀ cycloalkyl group and the like. Examples of the optionally substituted "substituent" in the ring include groups similar to the substituents of the "optionally substituted C₁₋₆ alkyl group" in the aforementioned respective definitions of R⁴ and R⁵, and a C₁₋₆ alkyl group optionally substituted by one or more substituents selected from
(1) a halogen atom,
(2) a carboxy group,
(3) a hydroxy group,
(4) a C₁₋₆ alkoxy-carbonyl group optionally substituted by a halogen atom,
(5) a C₁₋₆ alkoxy group optionally substituted by a halogen atom,
(6) an amino group optionally mono- or di-substituted by a C₁₋₆ alkyl group optionally substituted by a halogen atom, and
(7) a cyano group.

Examples of the optionally substituted "substituent" of the "optionally substituted C₁₋₁₂ alkoxy-carbonyl group" in the definition of M in the formula (I) include
(1) a halogen atom;
(2) a hydroxy group;
(3) a carboxy group;
(4) a cyano group;
(5) a sulfanyl (SH) group;
(6) an amino group optionally mono- or di-substituted by a substituent selected from
   (a) a C₁₋₆ alkyl group optionally substituted by a halogen atom,
   (b) a C₁₋₆ alkanoyl group optionally substituted by a halogen atom,
   (c) a C₇₋₁₃ aroyl group optionally substituted by a halogen atom,
   (d) a C₁₋₆ alkylsulfonyl group optionally substituted by a halogen atom,
   (e) a C₆₋₁₀ arylsulfonyl group optionally substituted by a substituent selected from
      (i) a halogen atom, and
      (ii) a C₁₋₆ alkyl group optionally substituted by a halogen atom, and
   (f) a C₁₋₆ alkoxy-carbonyl group optionally substituted by a halogen atom;
(7) a C₁₋₆ alkoxy-carbonyl group optionally substituted by a halogen atom;
(8) a C₁₋₆ alkoxy group optionally substituted by one or more substituents selected from
   (a) a halogen atom,
   (b) a carboxy group,
   (c) a hydroxy group,
   (d) a C₁₋₆ alkoxy group optionally substituted by a halogen atom,
   (e) a C₁₋₆ alkoxy-carbonyl group optionally substituted by a halogen atom, and
   (f) an amino group optionally mono- or di-substituted by a C₁₋₆ alkyl group optionally substituted by a halogen atom;
(9) a C₁₋₆ alkylthio group optionally substituted by a halogen atom;
(10) a C₃₋₁₀ cycloalkyl group (e.g., cyclopropyl, cyclobutyl, cyclopentyl, cyclohexyl, bicyclo[2.2.1]heptyl, bicyclo[2.2.2]octyl, bicyclo[3.2.1]octyl, adamantyl etc.) optionally substituted by one or more substituents selected from
   (a) a halogen atom,
   (b) a hydroxy group,
   (c) a C₁₋₆ alkoxy group optionally substituted by a halogen atom, and
   (d) a C₁₋₆ alkyl group optionally substituted by a halogen atom;
(11) a C₆₋₁₄ aryl group (e.g., phenyl group, naphthyl group etc.) optionally substituted by one or more substituents selected from
   (a) a C₁₋₆ alkyl group optionally substituted by one or more substituents selected from
      (i) a halogen atom,
      (ii) a carboxy group,
      (iii) a hydroxy group,
      (iv) a C₁₋₆ alkoxy group optionally substituted by a halogen atom,
      (v) an amino group optionally mono- or di-substituted by a C₁₋₆ alkyl group optionally substituted by a halogen atom, and
      (vi) a cyano group,
   (b) an amino group optionally mono- or di-substituted by a substituent selected from
      (i) a C₁₋₆ alkyl group optionally substituted by a halogen atom,
      (ii) a C₁₋₆ alkanoyl group optionally substituted by a halogen atom,
      (iii) a C₇₋₁₃ aroyl group optionally substituted by a halogen atom,
      (iv) a C₁₋₆ alkylsulfonyl group optionally substituted by a halogen atom,
      (v) a C₆₋₁₀ arylsulfonyl group optionally substituted by a substituent selected from
         (I) a halogen atom, and
         (II) a C₁₋₆ alkyl group optionally substituted by a halogen atom, and
      (vi) a C₁₋₆ alkoxy-carbonyl group optionally substituted by a halogen atom,
   (c) a C₁₋₆ alkoxy group optionally substituted by one or more substituents selected from
      (i) a halogen atom,
      (ii) a C₁₋₆ alkoxy group optionally substituted by a halogen atom,
      (iii) a carboxy group, and
      (iv) a hydroxy group,
   (d) a carbamoyl group optionally mono- or di-substituted by a C₁₋₆ alkyl group optionally substituted by a halogen atom,
   (e) a carboxy group,
   (f) a hydroxy group,
   (g) a halogen atom,
   (h) a sulfanyl (SH) group, and
      (i) a C₁₋₆ alkylthio group optionally substituted by a halogen atom;
(12) a heterocyclic group optionally substituted by one or more substituents selected from
   (a) a C₁₋₆ alkyl group optionally substituted by one or more substituents selected from
      (i) a halogen atom,
      (ii) a carboxy group,
      (iii) a hydroxy group,
      (iv) a C₁₋₆ alkoxy group optionally substituted by a halogen atom,
      (v) an amino group optionally mono- or di-substituted by a C₁₋₆ alkyl group optionally substituted by a halogen atom, and
      (vi) a cyano group;
   (b) an amino group optionally mono- or di-substituted by a substituent selected from
      (i) a C₁₋₆ alkyl group optionally substituted by a halogen atom,
      (ii) a C₁₋₆ alkanoyl group optionally substituted by a halogen atom,
      (iii) a C₇₋₁₃ aroyl group optionally substituted by a halogen atom,
      (iv) a C₁₋₆ alkylsulfonyl group optionally substituted by a halogen atom,
      (v) a C₆₋₁₀ arylsulfonyl group optionally substituted by a substituent selected from
         (I) a halogen atom, and
         (II) a C₁₋₆ alkyl group optionally substituted by a halogen atom, and
      (vi) a C₁₋₆ alkoxy-carbonyl group optionally substituted by a halogen atom,
   (c) a C₁₋₆ alkoxy group optionally substituted by one or more substituents selected from
      (i) a halogen atom,
      (ii) a C₁₋₆ alkoxy group optionally substituted by a halogen atom,
      (iii) a carboxy group, and
      (iv) a hydroxy group;
   (d) a carbamoyl group optionally mono- or di-substituted by a C₁₋₆ alkyl group optionally substituted by a halogen atom,
   (e) a carboxy group,
   (f) a hydroxy group,
   (g) a halogen atom,
   (h) a sulfanyl (SH) group, and
   (i) a C₁₋₆ alkylthio group optionally substituted by a halogen atom
and the like. When two or more substituents are present, the respective substituents may be the same or different.

Examples of the optionally substituted "substituent" of the "optionally substituted carbamoyl group" in the definition of M in the formula (I) include
(1) a C₁₋₁₂ alkyl group optionally substituted by one or more substituents selected from
   (a) a halogen atom,
   (b) a carboxy group,
   (c) a hydroxy group,
   (d) a C₁₋₆ alkoxy-carbonyl group optionally substituted by a halogen atom,
   (e) a C₁₋₆ alkoxy group optionally substituted by a halogen atom,
   (f) a C₁₋₆ alkylthio group optionally substituted by a halogen atom,
   (g) a C₃₋₁₀ cycloalkyl group optionally substituted by one or more substituents selected from
      (i) a halogen atom,
      (ii) a hydroxy group,
      (iii) a C₁₋₆ alkoxy group optionally substituted by a halogen atom, and
      (iv) a C₁₋₆ alkyl group optionally substituted by a halogen atom
   (h) an amino group optionally mono- or di-substituted by a C₁₋₆ alkyl group optionally substituted by a halogen atom,
   (i) a cyano group,
   (j) a C₆₋₁₄ aryl group (e.g., phenyl group, naphthyl group etc.) optionally substituted by one or more substituents selected from
      (i) a C₁₋₆ alkyl group optionally substituted by one or more substituents selected from
         (I) a halogen atom,
         (II) a carboxy group,
         (III) a hydroxy group,
         (IV) a C₁₋₆ alkoxy group optionally substituted by a halogen atom,
         (V) an amino group optionally mono- or di-substituted by a C₁₋₆ alkyl group optionally substituted by a halogen atom, and
         (VI) a cyano group,
      (ii) an amino group optionally mono- or di-substituted by a substituent selected from
         (I) a C₁₋₆ alkyl group optionally substituted by a halogen atom,
         (II) a C₁₋₆ alkanoyl group optionally substituted by a halogen atom,
         (III) a C₇₋₁₃ aroyl group optionally substituted by a halogen atom,
         (IV) a C₁₋₆ alkylsulfonyl group optionally substituted by a halogen atom,
         (V) a C₆₋₁₀ arylsulfonyl group optionally substituted by a substituent selected from a halogen atom and a C₁₋₆ alkyl group, and
         (VI) a C₁₋₆ alkoxy-carbonyl group optionally substituted by a halogen atom,
      (iii) a C₁₋₆ alkoxy group optionally substituted by one or more substituents selected from
         (I) a halogen atom,
         (II) a C₁₋₆ alkoxy group optionally substituted by a halogen atom,
         (III) a carboxy group, and
         (IV) a hydroxy group,
      (iv) a carbamoyl group optionally mono- or di-substituted by a C₁₋₆ alkyl group optionally substituted by a halogen atom,
      (v) a carboxy group,
      (vi) a hydroxy group,
      (vii) a halogen atom,
      (viii) a sulfanyl (SH) group, and
      (ix) a C₁₋₆ alkylthio group optionally substituted by a halogen atom, and
   (k) a 5- or 6-membered monocyclic aromatic heterocyclic group (e.g., thienyl group, furyl group, etc.) optionally substituted by one or more substituents selected from
      (i) a C₁₋₆ alkyl group optionally substituted by one or more substituents selected from
         (I) a halogen atom,
         (II) a carboxy group,
         (III) a hydroxy group,
         (IV) a C₁₋₆ alkoxy group optionally substituted by a halogen atom,
         (V) an amino group optionally mono- or di-substituted by a C₁₋₆ alkyl group optionally substituted by a halogen atom, and
         (VI) a cyano group,
      (ii) an amino group optionally mono- or di-substituted by a substituent selected from
         (I) a C₁₋₆ alkyl group optionally substituted by a halogen atom,
         (II) a C₁₋₆ alkanoyl group optionally substituted by a halogen atom,
         (III) a C₇₋₁₃ aroyl group optionally substituted by a halogen atom,
         (IV) a C₁₋₆ alkylsulfonyl group optionally substituted by a halogen atom,
         (V) a C₆₋₁₀ arylsulfonyl group optionally substituted by a substituent selected from a halogen atom and a C₁₋₆ alkyl group, and
         (VI) a C₁₋₆ alkoxy-carbonyl group optionally substituted by a halogen atom,
      (iii) a C₁₋₆ alkoxy group optionally substituted by one or more substituents selected from
         (I) a halogen atom,
         (II) a C₁₋₆ alkoxy group optionally substituted by a halogen atom,
         (III) a carboxy group, and
         (IV) a hydroxy group,
      (iv) a carbamoyl group optionally mono- or di-substituted by a C₁₋₆ alkyl group optionally substituted by a halogen atom,
      (v) a carboxy group,
      (vi) a hydroxy group,
      (vii) a halogen atom,
      (viii) a sulfanyl (SH) group, and
      (ix) a C₁₋₆ alkylthio group optionally substituted by a halogen atom;
(2) a C₃₋₁₀ cycloalkyl group optionally substituted by one or more substituents selected from
   (a) a halogen atom,
   (b) a hydroxy group,
   (c) a C₁₋₆ alkoxy group optionally substituted by a halogen atom, and
   (d) a C₁₋₆ alkyl group optionally substituted by a halogen atom;
and the like. When two or more substituents are present, the respective substituents may be the same or different. The carbamoyl group is optionally mono- or di-substituted by the aforementioned optionally substituted C₁₋₁₂ alkyl group.

When the "optionally substituted carbamoyl group" is di-substituted, the two substituents may form an optionally substituted 4- to 7-membered non-aromatic nitrogen-containing heterocycle (e.g., azetidinyl, pyrrolidinyl, pyrrolinyl, piperidyl, azepanyl, morpholinyl, thiomorpholinyl etc.) together with the carbamoyl nitrogen atom bonded thereto. As the optionally substituted "substituent", those similar to the optionally substituted "substituent" of the "optionally substituted carbamoyl group" in the aforementioned definition of M can be mentioned.

Examples of the optionally substituted "substituent" of the "optionally substituted heterocyclic group" in the definition of M in the formula (I) include
(1) a C₁₋₆ alkyl group optionally substituted by one or more substituents selected from
   (a) a halogen atom,
   (b) a carboxy group,
   (c) a hydroxy group,
   (d) a C₁₋₆ alkoxy group optionally substituted by a halogen atom,
   (e) an amino group optionally mono- or di-substituted by a C₁₋₆ alkyl group optionally substituted by a halogen atom, and
   (f) a cyano group;
(2) a C₇₋₁₆ aralkyl group optionally substituted by a halogen atom;
(3) an amino group optionally mono- or di-substituted by a substituent selected from
   (a) a C₁₋₆ alkyl group optionally substituted by a halogen atom,
   (b) a C₁₋₆ alkanoyl group optionally substituted by a halogen atom,
   (c) a C₇₋₁₃ aroyl group optionally substituted by a halogen atom,
   (d) a C₁₋₆ alkylsulfonyl group optionally substituted by a halogen atom,
   (e) a C₆₋₁₀ arylsulfonyl group optionally substituted by a substituent selected from
      (i) a halogen atom, and
      (ii) a C₁₋₆ alkyl group optionally substituted by a halogen atom, and
   (f) a C₁₋₆ alkoxy-carbonyl group optionally substituted by a halogen atom;
(4) a C₁₋₆ alkoxy group optionally substituted by one or more substituents selected from
   (a) a halogen atom,
   (b) a C₁₋₆ alkoxy group optionally substituted by a halogen atom,
   (c) a carboxy group, and
   (d) a hydroxy group;
(5) a C₇₋₁₆ aralkyloxy group optionally substituted by a halogen atom;
(6) a carbamoyl group optionally mono- or di-substituted by a C₁₋₆ alkyl group optionally substituted by a halogen atom;
(7) a carboxy group;
(8) a hydroxy group;
(9) a halogen atom,
(10) a sulfanyl (SH) group;
(11) a C₁₋₆ alkylthio group optionally substituted by a halogen atom;
(12) a C₇₋₁₆ aralkylthio group optionally substituted by a halogen atom;
(13) a C₆₋₁₄ aryl group optionally substituted by one or more substituents selected from
   (a) a C₁₋₆ alkyl group optionally substituted by one or more substituents selected from
      (i) a halogen atom,
      (ii) a carboxy group,
      (iii) a hydroxy group,
      (iv) a C₁₋₆ alkoxy group optionally substituted by a halogen atom,
      (v) an amino group optionally mono- or di-substituted by a C₁₋₆ alkyl group optionally substituted by a halogen atom, and
      (vi) a cyano group,
   (b) an amino group optionally mono- or di-substituted by a substituent selected from
      (i) a C₁₋₆ alkyl group optionally substituted by a halogen atom,
      (ii) a C₁₋₆ alkanoyl group optionally substituted by a halogen atom,
      (iii) a C₇₋₁₃ aroyl group optionally substituted by a halogen atom,
      (iv) a C₁₋₆ alkylsulfonyl group optionally substituted by a halogen atom,
      (v) a C₆₋₁₀ arylsulfonyl group optionally substituted by a substituent selected from
         (I) a halogen atom, and
         (II) a C₁₋₆ alkyl group optionally substituted by a halogen atom, and
      (vi) a C₁₋₆ alkoxy-carbonyl group optionally substituted by a halogen atom,
   (c) a C₁₋₆ alkoxy group optionally substituted by one or more substituents selected from
      (i) a halogen atom,
      (ii) a C₁₋₆ alkoxy group optionally substituted by a halogen atom,
      (iii) a carboxy group, and
      (iv) a hydroxy group,
   (d) a carbamoyl group optionally mono- or di-substituted by a C₁₋₆ alkyl group optionally substituted by a halogen atom,
   (e) a carboxy group,
   (f) a hydroxy group,
   (g) a halogen atom,
   (h) a sulfanyl (SH) group, and
      (i) a C₁₋₆ alkylthio group optionally substituted by a halogen atom
      and the like. When two or more substituents are present, the respective substituents may be the same or different.

Each substituent of the formula (I) is explained below.

A in the formula (I) is an optionally further substituted cyclic group.

Examples of the "cyclic group" of the "optionally further substituted cyclic group" for A include C₃₋₁₀ cycloalkyl group, C₃₋₁₀ cycloalkenyl group, C₄₋₁₀ cycloalkadienyl group, C₆₋₁₄ aryl group, heterocyclic group and the like, each of which optionally has the aforementioned substituent(s) at substitutable position(s). While the number of the substituents is not particularly limited as long as it is a substitutable number, it is preferably 1 to 5, more preferably 1 to 3. When plural substituents are present, the respective substituents may be the same or different. As the optionally further substituted "substituent", the substituents exemplified as the optionally substituted "substituent" in the aforementioned A and the like can be mentioned.

A is preferably an optionally further substituted C₆₋₁₄ aryl group.

Specific preferable examples of A include a C₆₋₁₄ aryl group optionally further substituted by a substituent selected from
(1) a halogen atom;
(2) a hydroxy group;
(3) a carboxy group;
(4) a sulfanyl (SH) group;
(5) a C₁₋₆ alkyl group optionally substituted by 1 to 3 substituents selected from
   (a) a halogen atom,
   (b) a carboxy group,
   (c) a hydroxy group,
   (d) a C₁₋₆ alkoxy-carbonyl group,
   (e) a C₁₋₆ alkoxy group,
   (f) an amino group optionally mono- or di-substituted by a C₁₋₆ alkyl group, and
   (g) a cyano group;
(6) a C₁₋₆ alkoxy group optionally substituted by 1 to 3 substituents selected from
   (a) a halogen atom,
   (b) a carboxy group,
   (c) a hydroxy group,
   (d) a C₁₋₆ alkoxy group,
   (e) a C₁₋₆ alkoxy-carbonyl group, and
   (f) an amino group optionally mono- or di-substituted by a C₁₋₆ alkyl group;
(7) an amino group optionally mono- or di-substituted by a substituent selected from
   (a) a C₁₋₆ alkyl group optionally substituted by a halogen atom,
   (b) a C₁₋₆ alkanoyl group optionally substituted by a halogen atom,
   (c) a C₇₋₁₃ aroyl group optionally substituted by a halogen atom,
   (d) a C₁₋₆ alkylsulfonyl group optionally substituted by a halogen atom,
   (e) a C₆₋₁₀ arylsulfonyl group optionally substituted by a substituent selected from
      (i) a halogen atom, and
      (ii) a C₁₋₆ alkyl group, and
   (f) a C₁₋₆ alkoxy-carbonyl group optionally substituted by a halogen atom;
(8) a C₁₋₆ alkylthio group optionally substituted by a halogen atom; and
(9) a C₁₋₃ alkylenedioxy group.

A is more preferably an optionally further substituted phenyl group.

A specific more preferable example of A is a phenyl group optionally further substituted by a C₁₋₆ alkyl group optionally substituted by 1 to 3 substituents selected from
(1) a halogen atom;
(2) a carboxy group;
(3) a hydroxy group;
(4) a C₁₋₆ alkoxy-carbonyl group;
(5) a C₁₋₆ alkoxy group;
(6) an amino group optionally mono- or di-substituted by a C₁₋₆ alkyl group; and
(7) a cyano group.

A is further preferably, a phenyl group substituted by 1 to 3 C₁₋₆ alkyl groups (e.g., 4-tert-butylphenyl, 4-propylphenyl, 4-methylphenyl, 4-isopropylphenyl, 4-ethylphenyl etc.), and 4-tert-butylphenyl is particularly preferable.

Another preferable embodiment of A is an optionally further substituted heterocyclic group.

Another specific preferable example of A is a 5- or 6-membered monocyclic aromatic heterocyclic group (preferably, a 5- or 6-membered monocyclic aromatic nitrogen-containing heterocyclic group) optionally further substituted by a substituent selected from
(1) a halogen atom;
(2) a hydroxy group;
(3) a carboxy group;
(4) a sulfanyl (SH) group;
(5) a C₁₋₆ alkyl group optionally substituted by 1 to 3 substituents selected from
   (a) a halogen atom,
   (b) a carboxy group,
   (c) a hydroxy group,
   (d) a C₁₋₆ alkoxy-carbonyl group,
   (e) a C₁₋₆ alkoxy group,
   (f) an amino group optionally mono- or di-substituted by a C₁₋₆ alkyl group, and
   (g) a cyano group;
(6) a C₁₋₆ alkoxy group optionally substituted by 1 to 3 substituents selected from
   (a) a halogen atom,
   (b) a carboxy group,
   (c) a hydroxy group,
   (d) a C₁₋₆ alkoxy group,
   (e) a C₁₋₆ alkoxy-carbonyl group, and
   (f) an amino group optionally mono- or di-substituted by a C₁₋₆ alkyl group;
(7) an amino group optionally mono- or di-substituted by a substituent selected from
   (a) a C₁₋₆ alkyl group optionally substituted by a halogen atom,
   (b) a C₁₋₆ alkanoyl group optionally substituted by a halogen atom,
   (c) a C₇₋₁₃ aroyl group optionally substituted by a halogen atom,
   (d) a C₁₋₆ alkylsulfonyl group optionally substituted by a halogen atom,
   (e) a C₆₋₁₀ arylsulfonyl group optionally substituted by a substituent selected from
      (i) a halogen atom, and
      (ii) a C₁₋₆ alkyl group, and
   (f) a C₁₋₆ alkoxy-carbonyl group optionally substituted by a halogen atom;
(8) a 4- to 7-membered monocyclic non-aromatic heterocyclic group;
(9) a C₁₋₆ alkylthio group optionally substituted by a halogen atom; and
(10) a C₁₋₃ alkylenedioxy group.

Another more preferable embodiment of A is a pyridyl group optionally further substituted by the same or different 1 to 4 substituents.

Another specific more preferable example of A is a pyridyl group (e.g., 2-pyridyl, 3-pyridyl, 4-pyridyl) optionally further substituted by 1 to 3 of
(1) amino group optionally mono- or di-substituted by a C₁₋₆ alkyl group; or
(2) 4- to 7-membered monocyclic non-aromatic nitrogen-containing heterocyclic group.

Another particularly preferable embodiment of A is a pyridyl group substituted by an n-hexylamino group or a 4-morpholinyl group (e.g., 2-n-hexylaminopyridin-3-yl, 2-(morpholin-4-yl)pyridin-3-yl etc.).

B in the formula (I) is an optionally further substituted benzene ring.

A specific preferable example of B is a benzene ring optionally further substituted by 1 to 4 substituents selected from
(1) a halogen atom;
(2) a hydroxy group;
(3) a sulfanyl (SH) group;
(4) an amino group optionally mono- or di-substituted by a substituent selected from
   (a) a C₁₋₆ alkyl group optionally substituted by a halogen atom,
   (b) a C₁₋₆ alkanoyl group optionally substituted by a halogen atom,
   (c) a C₇₋₁₃ aroyl group optionally substituted by a halogen atom,
   (d) a C₁₋₆ alkylsulfonyl group optionally substituted by a halogen atom,
   (e) a C₆₋₁₀ arylsulfonyl group optionally substituted by a substituent selected from
      (i) a halogen atom, and
      (ii) a C₁₋₆ alkyl group optionally substituted by a halogen atom, and
   (f) a C₁₋₆ alkoxy-carbonyl group optionally substituted by a halogen atom;
(5) a C₁₋₆ alkyl group optionally substituted by 1 to 3 substituents selected from
   (a) a halogen atom,
   (b) a carboxy group,
   (c) a hydroxy group,
   (d) a C₁₋₆ alkoxy-carbonyl group,
   (e) a C₁₋₆ alkoxy group,
   (f) an amino group optionally mono- or di-substituted by a C₁₋₆ alkyl group, and
   (g) a cyano group;
(6) a C₁₋₆ alkoxy group optionally substituted by 1 to 3 substituents selected from
   (a) a halogen atom,
   (b) a carboxy group,
   (c) a hydroxy group,
   (d) a C₁₋₆ alkoxy group,
   (e) a C₁₋₆ alkoxy-carbonyl group, and
   (f) an amino group optionally mono- or di-substituted by a C₁₋₆ alkyl group;
(7) a C₁₋₆ alkylthio group optionally substituted by a halogen atom; and
(8) a C₁₋₃ alkylenedioxy group.

A more specific preferable example of B is a benzene ring optionally further substituted by 1 to 4 substituents selected from
(1) a halogen atom (e.g., fluorine atom etc.); and
(2) a C₁₋₆ alkoxy group (e.g., methoxy etc.).

B is particularly preferably a benzene ring without a further substituent.

Another specific preferable example of B is a benzene ring optionally further substituted by 1 to 4 substituents selected from
(1) a halogen atom;
(2) a hydroxy group;
(3) a sulfanyl (SH) group;
(4) an amino group optionally mono- or di-substituted by a substituent selected from
   (a) a C₁₋₆ alkyl group optionally substituted by a halogen atom,
   (b) a C₁₋₆ alkanoyl group optionally substituted by a halogen atom,
   (c) a C₇₋₁₃ aroyl group optionally substituted by a halogen atom,
   (d) a C₁₋₆ alkylsulfonyl group optionally substituted by a halogen atom,
   (e) a C₆₋₁₀ arylsulfonyl group optionally substituted by a substituent selected from
      (i) a halogen atom, and
      (ii) a C₁₋₆ alkyl group optionally substituted by a halogen atom, and
   (f) a C₁₋₆ alkoxy-carbonyl group optionally substituted by a halogen atom;
(5) a C₁₋₆ alkyl group optionally substituted by 1 to 3 substituents selected from
   (a) a halogen atom,
   (b) a carboxy group,
   (c) a hydroxy group,
   (d) a C₁₋₆ alkoxy-carbonyl group,
   (e) a C₁₋₆ alkoxy group,
   (f) an amino group optionally mono- or di-substituted by a C₁₋₆ alkyl group, and
   (g) a cyano group;
(6) a C₁₋₆ alkoxy group optionally substituted by 1 to 3 substituents selected from
   (a) a halogen atom,
   (b) a carboxy group,
   (c) a hydroxy group,
   (d) a C₁₋₆ alkoxy group,
   (e) a C₁₋₆ alkoxy-carbonyl group, and
   (f) an amino group optionally mono- or di-substituted by a C₁₋₆ alkyl group;
(7) a C₁₋₆ alkylthio group optionally substituted by a halogen atom;
(8) a C₁₋₃ alkylenedioxy group; and
(9) a 4- to 7-membered monocyclic non-aromatic heterocyclic group (e.g., 4-morpholinyl).

Another specific more preferable example of B is a benzene ring optionally further substituted by 1 to 4 substituents selected from
(1) a halogen atom (e.g., fluorine atom etc.);
(2) a C₁₋₆ alkoxy group (e.g., methoxy etc.); and
(3) a 4- to 7-membered monocyclic non-aromatic heterocyclic group (e.g., 4-morpholinyl).

The position of the group -N(R²)- bonded to B in the formula (I) is not particularly limited. It binds to B preferably at a meta-position or para-position, more preferably, para-position, relative to the binding position of the group-N(R¹)-.

L in the formula (I) is a bond or an optionally substituted C₁₋₆ alkylene group.

L is preferably a bond or a methylene group, more preferably, a bond.

R¹ in the formula (I) is a hydrogen atom, a hydroxy group, an optionally substituted C₁₋₆ alkoxy group, an optionally substituted amino group or an optionally substituted C₁₋₆ alkyl group. As the optionally substituted "substituent", the substituents exemplified as the optionally substituted "substituent" in the aforementioned R¹ and the like can be mentioned.

R¹ is preferably a hydrogen atom, or a C₁₋₆ alkyl group optionally substituted by one or more substituents selected from
(1) a halogen atom;
(2) a hydroxy group;
(3) a sulfanyl group;
(4) a carboxy group;
(5) a C₁₋₆ alkoxy group;
(6) an amino group optionally mono- or di-substituted by a C₁₋₆ alkyl group; and
(7) a C₁₋₆ alkylthio group optionally substituted by a halogen atom, more preferably, a hydrogen atom, or a C₁₋₆ alkyl group substituted by a C₁₋₆ alkylthio group optionally substituted by a halogen atom, particularly preferably, a hydrogen atom.

Q in the formula (I) is an oxygen atom or a sulfur atom.

Q is preferably an oxygen atom.

R² and R³ in the formula (I) are the same or different and are each independently a hydrogen atom, a carboxy group, a hydroxy group, an optionally substituted C₁₋₁₀ alkoxy group, an optionally substituted amino group or an optionally substituted C₁₋₁₀ alkyl group. As the optionally substituted "substituent", the substituents exemplified as the optionally substituted "substituent" in the aforementioned R² and R³ and the like can be mentioned.

R² and R³ are preferably the same or different and are each independently a hydrogen atom, a carboxy group, a hydroxy group, or a C₁₋₁₀ alkyl group optionally substituted by one or more substituents selected from
(1) a halogen atom;
(2) a hydroxy group;
(3) a sulfanyl group;
(4) a carboxy group;
(5) a C₁₋₆ alkoxy group;
(6) an amino group optionally mono- or di-substituted by a C₁₋₆ alkyl group; and
(7) a C₁₋₆ alkylthio group optionally substituted by a halogen atom,
more preferably, a hydrogen atom or a C₁₋₁₀ alkyl group optionally substituted by a carboxy group (e.g., methyl, heptyl, carboxymethyl etc.), particularly preferably, a hydrogen atom or a C₁₋₁₀ alkyl group (e.g., methyl, heptyl etc.).

R⁴ and R⁵ in the formula (I) are the same or different and are each independently a hydrogen atom, or optionally substituted C₁₋₆ alkyl group, or R⁴ and R⁵ form an optionally substituted ring (e.g., C₃₋₁₀ cycloalkyl group etc.), together with the carbon atom bonded thereto. As the optionally substituted "substituent", the substituents exemplified as the optionally substituted "substituent" in the aforementioned R⁴ and R⁵ and the like can be mentioned.

R⁴ and R⁵ are preferably the same or different and are each independently a hydrogen atom, or a C₁₋₆ alkyl group optionally substituted by one or more substituents selected from
(1) a halogen atom;
(2) a hydroxy group;
(3) a carboxy group;
(4) a guanidino group;
(5) a cyano group;
(6) a sulfanyl (SH) group;
(7) an amino group optionally mono- or di-substituted by a substituent selected from
   (a) a C₁₋₆ alkyl group optionally substituted by a halogen atom,
   (b) a C₁₋₆ alkanoyl group optionally substituted by a halogen atom,
   (c) a C₇₋₁₃ aroyl group optionally substituted by a halogen atom,
   (d) a C₁₋₆ alkylsulfonyl group optionally substituted by a halogen atom,
   (e) a C₆₋₁₀ arylsulfonyl group optionally substituted by a substituent selected from
      (i) a halogen atom, and
      (ii) a C₁₋₆ alkyl group, and
   (f) a C₁₋₆ alkoxy-carbonyl group optionally substituted by a halogen atom;
(8) a carbamoyl group optionally mono- or di-substituted by a C₁₋₆ alkyl group optionally substituted by a halogen atom;
(9) a C₁₋₆ alkoxy-carbonyl group optionally substituted by a halogen atom;
(10) a C₁₋₆ alkoxy group optionally substituted by one or more substituents selected from
   (a) a halogen atom,
   (b) a carboxy group,
   (c) a hydroxy group,
   (d) a C₁₋₆ alkoxy group,
   (e) a C₁₋₆ alkoxy-carbonyl group, and
   (f) an amino group optionally mono- or di-substituted by a C₁₋₆ alkyl group;
(11) a C₁₋₆ alkylthio group optionally substituted by a halogen atom;
(12) a C₃₋₁₀ cycloalkyl group optionally substituted by a substituent selected from
   (a) a halogen atom,
   (b) a hydroxy group,
   (c) a C₁₋₆ alkoxy group, and
   (d) a C₁₋₆ alkyl group; and
(13) an aromatic ring group (e.g., C₆₋₁₄ aryl group such as phenyl group, naphthyl group and the like; 5- or 6-membered monocyclic aromatic nitrogen-containing heterocyclic group such as imidazolyl group and the like; 8- to 14-membered fused aromatic nitrogen-containing heterocyclic group such as indolyl group and the like, etc.) optionally substituted by one or more substituents selected from
   (a) a C₁₋₆ alkyl group optionally substituted by one or more substituents selected from
      (i) a halogen atom,
      (ii) a carboxy group,
      (iii) a hydroxy group,
      (iv) a C₁₋₆ alkoxy group,
      (v) an amino group optionally mono- or di-substituted by a C₁₋₆ alkyl group, and
      (vi) a cyano group,
   (b) an amino group optionally mono- or di-substituted by a substituent selected from
      (i) a C₁₋₆ alkyl group optionally substituted by a halogen atom,
      (ii) a C₁₋₆ alkanoyl group optionally substituted by a halogen atom,
      (iii) a C₇₋₁₃ aroyl group optionally substituted by a halogen atom,
      (iv) a C₁₋₆ alkylsulfonyl group optionally substituted by a halogen atom,
      (v) a C₆₋₁₀ arylsulfonyl group optionally substituted by a substituent selected from
         (I) a halogen atom, and
         (II) a C₁₋₆ alkyl group, and
      (vi) a C₁₋₆ alkoxy-carbonyl group optionally substituted by a halogen atom,
   (c) a C₁₋₆ alkoxy group optionally substituted by one or more substituents selected from
      (i) a halogen atom,
      (ii) a C₁₋₆ alkoxy group,
      (iii) a carboxy group, and
      (iv) a hydroxy group,
   (d) a carbamoyl group optionally mono- or di-substituted by a C₁₋₆ alkyl group optionally substituted by a halogen atom,
   (e) a carboxy group,
   (f) a hydroxy group,
   (g) a halogen atom,
   (h) a sulfanyl (SH) group, and
      (i) a C₁₋₆ alkylthio group optionally substituted by a halogen atom.

In a more preferable embodiment of R⁴ and R⁵, R⁴ is a hydrogen atom and R⁵ is a C₁₋₆ alkyl group optionally substituted by one or more substituents selected from
(1) a halogen atom;
(2) a hydroxy group;
(3) a carboxy group;
(4) a guanidino group;
(5) a cyano group;
(6) a sulfanyl (SH) group;
(7) an amino group optionally mono- or di-substituted by a substituent selected from
   (a) a C₁₋₆ alkyl group optionally substituted by a halogen atom,
   (b) a C₁₋₆ alkanoyl group optionally substituted by a halogen atom,
   (c) a C₇₋₁₃ aroyl group optionally substituted by a halogen atom,
   (d) a C₁₋₆ alkylsulfonyl group optionally substituted by a halogen atom,
   (e) a C₆₋₁₀ arylsulfonyl group optionally substituted by a substituent selected from
      (i) a halogen atom, and
      (ii) a C₁₋₆ alkyl group, and
   (f) a C₁₋₆ alkoxy-carbonyl group optionally substituted by a halogen atom;
(8) a carbamoyl group optionally mono- or di-substituted by a C₁₋₆ alkyl group optionally substituted by a halogen atom;
(9) a C₁₋₆ alkoxy-carbonyl group;
(10) a C₁₋₆ alkoxy group optionally substituted by one or more substituents selected from
   (a) a halogen atom,
   (b) a carboxy group,
   (c) a hydroxy group,
   (d) a C₁₋₆ alkoxy group,
   (e) a C₁₋₆ alkoxy-carbonyl group, and
   (f) an amino group optionally mono- or di-substituted by a C₁₋₆ alkyl group;
(11) a C₁₋₆ alkylthio group optionally substituted by a halogen atom;
(12) a C₃₋₁₀ cycloalkyl group optionally substituted by a substituent selected from
   (a) a halogen atom,
   (b) a hydroxy group,
   (c) a C₁₋₆ alkoxy group, and
   (d) a C₁₋₆ alkyl group; and
(13) an aromatic ring group (e.g., C₆₋₁₄ aryl group such as phenyl group, naphthyl group and the like; 5- or 6-membered monocyclic aromatic nitrogen-containing heterocyclic group such as imidazolyl group and the like; 8- to 14-membered fused aromatic nitrogen-containing heterocyclic group such as indolyl group and the like, etc.) optionally substituted by one or more substituents selected from
   (a) a C₁₋₆ alkyl group optionally substituted by one or more substituents selected from
      (i) a halogen atom,
      (ii) a carboxy group,
      (iii) a hydroxy group,
      (iv) a C₁₋₆ alkoxy group,
      (v) an amino group optionally mono- or di-substituted by a C₁₋₆ alkyl group, and
      (vi) a cyano group,
   (b) an amino group optionally mono- or di-substituted by a substituent selected from
      (i) a C₁₋₆ alkyl group optionally substituted by a halogen atom,
      (ii) a C₁₋₆ alkanoyl group optionally substituted by a halogen atom,
      (iii) a C₇₋₁₃ aroyl group optionally substituted by a halogen atom,
      (iv) a C₁₋₆ alkylsulfonyl group optionally substituted by a halogen atom,
      (v) a C₆₋₁₀ arylsulfonyl group optionally substituted by a substituent selected from
         (I) a halogen atom, and
         (II) a C₁₋₆ alkyl group, and
      (vi) a C₁₋₆ alkoxy-carbonyl group optionally substituted by a halogen atom,
   (c) a C₁₋₆ alkoxy group optionally substituted by one or more substituents selected from
      (i) a halogen atom,
      (ii) a C₁₋₆ alkoxy group,
      (iii) a carboxy group, and
      (iv) a hydroxy group,
   (d) a carbamoyl group optionally mono- or di-substituted by a C₁₋₆ alkyl group optionally substituted by a halogen atom,
   (e) a carboxy group,
   (f) a hydroxy group,
   (g) a halogen atom,
   (h) a sulfanyl (SH) group, and
   (i) a C₁₋₆ alkylthio group optionally substituted by a halogen atom.

In a more preferable embodiment of R⁴ and R⁵,
R⁴ is a hydrogen atom, and R⁵ is a C₁₋₄ alkyl group (e.g., methyl, ethyl, butyl, isobutyl etc.) optionally substituted by a substituent selected from
(1) a C₃₋₁₀ cycloalkyl group (e.g., cyclohexyl group etc.) optionally substituted by a substituent selected from
   (a) a halogen atom,
   (b) a hydroxy group,
   (c) a C₁₋₆ alkoxy group, and
   (d) a C₁₋₆ alkyl group;
(2) a phenyl group (e.g., 4-fluorophenyl, 4-chlorophenyl, 4-tert-butylphenyl etc.) optionally substituted by one or more substituents selected from
   (a) a halogen atom, and
   (b) a C₁₋₆ alkoxy group;
(3) a C₁₋₆ alkylthio group (e.g., methylthio etc.); and
(4) a 8- to 14-membered fused aromatic nitrogen-containing heterocyclic group (e.g., indolyl group etc.).

In another preferable embodiment of R⁴ and R⁵, R⁴ and R⁵ are preferably the same or different and are each independently a hydrogen atom, or a C₁₋₆ alkyl group optionally substituted by one or more substituents selected from
(1) a halogen atom;
(2) a hydroxy group;
(3) a carboxy group;
(4) a guanidino group;
(5) a cyano group;
(6) a sulfanyl (SH) group;
(7) an amino group optionally mono- or di-substituted by a substituent selected from
   (a) a C₁₋₆ alkyl group optionally substituted by a halogen atom,
   (b) a C₁₋₆ alkanoyl group optionally substituted by a halogen atom,
   (c) a C₇₋₁₃ aroyl group optionally substituted by a halogen atom,
   (d) a C₁₋₆ alkylsulfonyl group optionally substituted by a halogen atom,
   (e) a C₆₋₁₀ arylsulfonyl group optionally substituted by a substituent selected from
      (i) a halogen atom, and
      (ii) a C₁₋₆ alkyl group, and
   (f) a C₁₋₆ alkoxy-carbonyl group optionally substituted by a halogen atom;
(8) a carbamoyl group optionally mono- or di-substituted by a C₁₋₆ alkyl group optionally substituted by a halogen atom;
(9) a C₁₋₆ alkoxy-carbonyl group optionally substituted by a halogen atom;
(10) a C₁₋₆ alkoxy group optionally substituted by one or more substituents selected from
   (a) a halogen atom,
   (b) a carboxy group,
   (c) a hydroxy group,
   (d) a C₁₋₆ alkoxy group,
   (e) a C₁₋₆ alkoxy-carbonyl group, and
   (f) an amino group optionally mono- or di-substituted by a C₁₋₆ alkyl group;
(11) a C₁₋₆ alkylthio group optionally substituted by a halogen atom;
(12) a C₃₋₁₀ cycloalkyl group optionally substituted by a substituent selected from
   (a) a halogen atom,
   (b) a hydroxy group,
   (c) a C₁₋₆ alkoxy group, and
   (d) a C₁₋₆ alkyl group; and
(13) an aromatic ring group (e.g., C₆₋₁₄ aryl group such as phenyl group, naphthyl group and the like; 5- or 6-membered monocyclic aromatic nitrogen-containing heterocyclic group such as imidazolyl group, pyridyl group and the like; 8- to 14-membered fused aromatic nitrogen-containing heterocyclic group such as indolyl group and the like, etc.) optionally substituted by one or more substituents selected from
   (a) a C₁₋₆ alkyl group optionally substituted by one or more substituents selected from
      (i) a halogen atom,
      (ii) a carboxy group,
      (iii) a hydroxy group,
      (iv) a C₁₋₆ alkoxy group,
      (v) an amino group optionally mono- or di-substituted by a C₁₋₆ alkyl group, and
      (vi) a cyano group,
   (b) an amino group optionally mono- or di-substituted by a substituent selected from
      (i) a C₁₋₆ alkyl group optionally substituted by a halogen atom,
      (ii) a C₁₋₆ alkanoyl group optionally substituted by a halogen atom,
      (iii) a C₇₋₁₃ aroyl group optionally substituted by a halogen atom,
      (iv) a C₁₋₆ alkylsulfonyl group optionally substituted by a halogen atom,
      (v) a C₆₋₁₀ arylsulfonyl group optionally substituted by a substituent selected from
         (I) a halogen atom, and
         (II) a C₁₋₆ alkyl group, and
      (vi) a C₁₋₆ alkoxy-carbonyl group optionally substituted by a halogen atom,
   (c) a C₁₋₆ alkoxy group optionally substituted by one or more substituents selected from
      (i) a halogen atom,
      (ii) a C₁₋₆ alkoxy group,
      (iii) a carboxy group, and
      (iv) a hydroxy group,
   (d) a carbamoyl group optionally mono- or di-substituted by a C₁₋₆ alkyl group optionally substituted by a halogen atom,
   (e) a carboxy group,
   (f) a hydroxy group,
   (g) a halogen atom,
   (h) a sulfanyl (SH) group, and
   (i) a C₁₋₆ alkylthio group optionally substituted by a halogen atom.

In another more preferable embodiment of R⁴ and R⁵, R⁴ is a hydrogen atom and R⁵ is a C₁₋₆ alkyl group optionally substituted by one or more substituents selected from
(1) a halogen atom;
(2) a hydroxy group;
(3) a carboxy group;
(4) a guanidino group;
(5) a cyano group;
(6) a sulfanyl (SH) group;
(7) an amino group optionally mono- or di-substituted by a substituent selected from
   (a) a C₁₋₆ alkyl group optionally substituted by a halogen atom,
   (b) a C₁₋₆ alkanoyl group optionally substituted by a halogen atom,
   (c) a C₇₋₁₃ aroyl group optionally substituted by a halogen atom,
   (d) a C₁₋₆ alkylsulfonyl group optionally substituted by a halogen atom,
   (e) a C₆₋₁₀ arylsulfonyl group optionally substituted by a substituent selected from
      (i) a halogen atom, and
      (ii) a C₁₋₆ alkyl group, and
   (f) a C₁₋₆ alkoxy-carbonyl group optionally substituted by a halogen atom;
(8) a carbamoyl group optionally mono- or di-substituted by a C₁₋₆ alkyl group optionally substituted by a halogen atom;
(9) a C₁₋₆ alkoxy-carbonyl group;
(10) a C₁₋₆ alkoxy group optionally substituted by one or more substituents selected from
   (a) a halogen atom,
   (b) a carboxy group,
   (c) a hydroxy group,
   (d) a C₁₋₆ alkoxy group,
   (e) a C₁₋₆ alkoxy-carbonyl group, and
   (f) an amino group optionally mono- or di-substituted by a C₁₋₆ alkyl group;
(11) a C₁₋₆ alkylthio group optionally substituted by a halogen atom;
(12) a C₃₋₁₀ cycloalkyl group optionally substituted by a substituent selected from
   (a) a halogen atom,
   (b) a hydroxy group,
   (c) a C₁₋₆ alkoxy group, and
   (d) a C₁₋₆ alkyl group; and
(13) an aromatic ring group (e.g., C₆₋₁₄ aryl group such as phenyl group, naphthyl group and the like; 5- or 6-membered monocyclic aromatic nitrogen-containing heterocyclic group such as imidazolyl group, pyridyl group and the like; 8- to 14-membered fused aromatic nitrogen-containing heterocyclic group such as indolyl group and the like, etc.) optionally substituted by one or more substituents selected from
   (a) a C₁₋₆ alkyl group optionally substituted by one or more substituents selected from
      (i) a halogen atom,
      (ii) a carboxy group,
      (iii) a hydroxy group,
      (iv) a C₁₋₆ alkoxy group,
      (v) an amino group optionally mono- or di-substituted by a C₁₋₆ alkyl group, and
      (vi) a cyano group,
   (b) an amino group optionally mono- or di-substituted by a substituent selected from
      (i) a C₁₋₆ alkyl group optionally substituted by a halogen atom,
      (ii) a C₁₋₆ alkanoyl group optionally substituted by a halogen atom,
      (iii) a C₇₋₁₃ aroyl group optionally substituted by a halogen atom,
      (iv) a C₁₋₆ alkylsulfonyl group optionally substituted by a halogen atom,
      (v) a C₆₋₁₀ arylsulfonyl group optionally substituted by a substituent selected from
         (I) a halogen atom, and
         (II) a C₁₋₆ alkyl group, and
      (vi) a C₁₋₆ alkoxy-carbonyl group optionally substituted by a halogen atom,
   (c) a C₁₋₆ alkoxy group optionally substituted by one or more substituents selected from
      (i) a halogen atom,
      (ii) a C₁₋₆ alkoxy group,
      (iii) a carboxy group, and
      (iv) a hydroxy group,
   (d) a carbamoyl group optionally mono- or di-substituted by a C₁₋₆ alkyl group optionally substituted by a halogen atom,
   (e) a carboxy group,
   (f) a hydroxy group,
   (g) a halogen atom,
   (h) a sulfanyl (SH) group, and
   (i) a C₁₋₆ alkylthio group optionally substituted by a halogen atom.

In another further preferable embodiment of R⁴ and R⁵, R⁴ is a hydrogen atom, and R⁵ is a C₁₋₄ alkyl group (e.g., methyl, ethyl, butyl, isobutyl etc.) optionally substituted by a substituent selected from
(1) a C₃₋₁₀ cycloalkyl group (e.g., cyclohexyl group etc.) optionally substituted by a substituent selected from
   (a) a halogen atom,
   (b) a hydroxy group,
   (c) a C₁₋₆ alkoxy group, and
   (d) a C₁₋₆ alkyl group;
(2) a phenyl group (e.g., 4-fluorophenyl, 4-chlorophenyl, 4-tert-butylphenyl etc.) optionally substituted by one or more substituents selected from
   (a) a halogen atom, and
   (b) a C₁₋₆ alkoxy group;
(3) a C₁₋₆ alkylthio group (e.g., methylthio etc.); and
(4) a 5- or 6-membered monocyclic aromatic nitrogen-containing heterocyclic group (e.g., pyridyl group etc.); and
(5) a 8- to 14-membered fused aromatic nitrogen-containing heterocyclic group (e.g., indolyl group etc.) optionally substituted by one or more substituents selected from
   (a) a halogen atom, and
   (b) a C₁₋₆ alkyl group.

In the formula (I), n is an integer of 1 - 6.

n is preferably 1.

In the formula (I), M is an optionally substituted C₁₋₁₂ alkoxy-carbonyl group, an optionally substituted carbamoyl group or an optionally substituted heterocyclic group. As the optionally substituted "substituent", the substituents exemplified as the optionally substituted "substituent" in the aforementioned M and the like can be mentioned.

M is preferably
(1) a C₁₋₁₂ alkoxy-carbonyl group (e.g., tert-butoxycarbonyl group etc.) optionally substituted by one or more substituents selected from
   (a) a halogen atom,
   (b) a C₁₋₆ alkoxy group,
   (c) a carboxy group, and
   (d) a hydroxy group;
(2) a carbamoyl group (e.g., 2-ethylhexylcarbamoyl, 1,5-dimethylhexylcarbamoyl, 1-methylhexylcarbamoyl, octylcarbamoyl, hexyl(methyl)carbamoyl, pentylcarbamoyl, hexylcarbamoyl, heptylcarbamoyl, 2,2-dimethylpropylcarbamoyl, 2-ethylbutylcarbamoyl, 1-methylhexylcarbamoyl, cyanomethylcarbamoyl, 2,2,2-trifluoroethylcarbamoyl, 2-methyl-2-methoxypropylcarbamoyl, 1-methoxycarbonylpentylcarbamoyl, cyclohexylmethylcarbamoyl, cyclopentylmethylcarbamoyl, benzylcarbamoyl, 1-phenylethylcarbamoyl, 4-methoxybenzylcarbamoyl, 4-fluorobenzylcarbamoyl, 2-thienylmethylcarbamoyl, 5-methylfuran-2-ylmethylcarbamoyl, cyclohexylethylcarbamoyl, 2-phenylethylcarbamoyl, 2-thienylethylcarbamoyl, 1-adamantyl-1-methylethylcarbamoyl, 6,6-dimethylbicyclo[3.1.1]heptan-2-ylcarbamoyl, cyclohexylcarbamoyl, cycloheptylcarbamoyl, cyclooctylcarbamoyl etc.) optionally mono- or di-substituted by substituent(s) selected from
   (a) a C₁₋₁₂ alkyl group optionally substituted by one or more substituents selected from
      (i) a C₃₋₁₀ cycloalkyl group,
      (ii) a C₁₋₆ alkoxy group,
      (iii) a halogen atom,
      (iv) a cyano group,
      (v) a C₁₋₆ alkoxy-carbonyl group,
      (vi) a phenyl group optionally substituted by one or more substituents selected from
         (I) a halogen atom, and
         (II) a C₁₋₆ alkoxy group, and
      (vii) an aromatic heterocyclic group optionally substituted by one or more substituents selected from
         (I) a halogen atom, and
         (II) a C₁₋₆ alkyl group, and
   (b) a C₃₋₁₀ cycloalkyl group; or
(3) a monocyclic aromatic heterocyclic group optionally substituted by 1 to 3 substituents selected from
   (a) a C₁₋₆ alkyl group,
   (b) a sulfanyl (SH) group, and
   (c) a phenyl group optionally substituted by one or more substituents selected from
      (i) a halogen atom,
      (ii) a C₁₋₆ alkyl group optionally substituted by a halogen atom, and
      (iii) a C₁₋₆ alkoxy group optionally substituted by a halogen atom.

M is more preferably
(1) a C₁₋₁₂ alkoxy-carbonyl group optionally substituted by one or more substituents selected from
   (a) a halogen atom,
   (b) a C₁₋₆ alkoxy group,
   (c) a carboxy group, and
   (d) a hydroxy group;
(2) a carbamoyl group (e.g., 2-ethylhexylcarbamoyl, 1,5-dimethylhexylcarbamoyl, 1-methylhexylcarbamoyl, octylcarbamoyl, hexyl(methyl)carbamoyl, pentylcarbamoyl, hexylcarbamoyl, heptylcarbamoyl, 2,2-dimethylpropylcarbamoyl, 2-ethylbutylcarbamoyl, 1-methylhexylcarbamoyl, cyanomethylcarbamoyl, 2,2,2-trifluoroethylcarbamoyl, 2-methyl-2-methoxypropylcarbamoyl, 1-methoxycarbonylpentylcarbamoyl, cyclohexylmethylcarbamoyl, cyclopentylmethylcarbamoyl, benzylcarbamoyl, 1-phenylethylcarbamoyl, 4-methoxybenzylcarbamoyl, 4-fluorobenzylcarbamoyl, 2-thienylmethylcarbamoyl, 5-methylfuran-2-ylmethylcarbamoyl, cyclohexylethylcarbamoyl, 2-phenylethylcarbamoyl, 2-thienylethylcarbamoyl, 1-adamantyl-1-methylethylcarbamoyl, 6,6-dimethylbicyclo[3.1.1]heptan-2-ylcarbamoyl, cyclohexylcarbamoyl, cycloheptylcarbamoyl, cyclooctylcarbamoyl etc.) optionally mono- or di-substituted by substituent(s) selected from
   (a) a C₁₋₈ alkyl group optionally substituted by one or more substituents selected from
      (i) a C₃₋₁₀ cycloalkyl group,
      (ii) a C₁₋₆ alkoxy group,
      (iii) a phenyl group optionally substituted by one or more substituents selected from
         (I) a halogen atom, and
         (II) a C₁₋₆ alkoxy group, and
      (iv) a 5- or 6-membered monocyclic aromatic heterocyclic group, and
   (b) a C₃₋₁₀ cycloalkyl group; or
(3) a 5- or 6-membered monocyclic aromatic heterocyclic group (e.g., triazolyl group, isoxazolyl group, oxazolyl group, oxadiazolyl group, tetrazolyl group etc.) optionally substituted by 1 to 3 substituents selected from
   (a) a C₁₋₄ alkyl group;
   (b) a sulfanyl (SH) group; and
   (c) a phenyl group optionally substituted by one or more substituents selected from
      (i) a halogen atom,
      (ii) a C₁₋₆ alkyl group optionally substituted by a halogen atom, and
      (iii) a C₁₋₆ alkoxy group optionally substituted by a halogen atom.

M is further preferably
a carbamoyl group mono-substituted by a C₁₋₁₂ alkyl group (e.g., 2-ethylhexylcarbamoyl, 1,5-dimethylhexylcarbamoyl, 1-methylhexylcarbamoyl, octylcarbamoyl, hexyl(methyl)carbamoyl etc.), or a 5-membered monocyclic aromatic heterocyclic group (preferably, oxadiazolyl group) optionally substituted by a phenyl group optionally substituted by a C₁₋₆ alkoxy group optionally substituted by a halogen atom (e.g., 3-(4-propyloxyphenyl)-1,2,4-oxadiazolin-5-yl, 3-[4-(trifluoromethoxy)phenyl]-1,2,4-oxadiazolin-5-yl etc.).

In another preferable embodiment, M is a carbamoyl group (e.g., 2-ethylhexylcarbamoyl, 1,5-dimethylhexylcarbamoyl, 1-methylhexylcarbamoyl, octylcarbamoyl, hexyl(methyl)carbamoyl, pentylcarbamoyl, hexylcarbamoyl, heptylcarbamoyl, 2,2-dimethylpropylcarbamoyl, 2-ethylbutylcarbamoyl, 1-methylhexylcarbamoyl, cyanomethylcarbamoyl, 2,2,2-trifluoroethylcarbamoyl, 2-methyl-2-methoxypropylcarbamoyl, 1-methoxycarbonylpentylcarbamoyl, cyclohexylmethylcarbamoyl, cyclopentylmethylcarbamoyl, benzylcarbamoyl, 1-phenylethylcarbamoyl, 4-methoxybenzylcarbamoyl, 4-fluorobenzylcarbamoyl, 2-thienylmethylcarbamoyl, 5-methylfuran-2-ylmethylcarbamoyl, cyclohexylethylcarbamoyl, 2-phenylethylcarbamoyl, 2-thienylethylcarbamoyl, 1-adamantyl-1-methylethylcarbamoyl, 6,6-dimethylbicyclo[3.1.1]heptan-2-ylcarbamoyl, cyclohexylcarbamoyl, cycloheptylcarbamoyl, cyclooctylcarbamoyl, 3-methylthiopropylcarbamoyl, 5-t-butylthiopentyl, 3-methoxypropylcarbamoyl etc.) optionally mono- or di-substituted by substituent(s) selected from
(1) a C₁₋₁₂ alkyl group optionally substituted by one or more substituents selected from
   (a) a C₃₋₁₀ cycloalkyl group,
   (b) a C₁₋₆ alkoxy group,
   (c) a halogen atom,
   (d) a cyano group,
   (e) a C₁₋₆ alkoxy-carbonyl group,
   (f) a C₁₋₆ alkylthio group optionally substituted by a halogen atom,
   (g) a phenyl group optionally substituted by one or more substituents selected from
      (i) a halogen atom, and
      (ii) a C₁₋₆ alkoxy group, and
   (h) an aromatic heterocyclic group optionally substituted by one or more substituents selected from
      (i) a halogen atom, and
      (ii) a C₁₋₆ alkyl group; and
(2) a C₃₋₁₀ cycloalkyl group.

In another more preferable embodiment, M is a carbamoyl group (e.g., 2-ethylhexylcarbamoyl, 1,5-dimethylhexylcarbamoyl, 1-methylhexylcarbamoyl, octylcarbamoyl, hexyl(methyl)carbamoyl, pentylcarbamoyl, hexylcarbamoyl, heptylcarbamoyl, 2,2-dimethylpropylcarbamoyl, 2-ethylbutylcarbamoyl, 1-methylhexylcarbamoyl, cyanomethylcarbamoyl, 2,2,2-trifluoroethylcarbamoyl, 2-methyl-2-methoxypropylcarbamoyl, 1-methoxycarbonylpentylcarbamoyl, cyclohexylmethylcarbamoyl, cyclopentylmethylcarbamoyl, benzylcarbamoyl, 1-phenylethylcarbamoyl, 4-methoxybenzylcarbamoyl, 4-fluorobenzylcarbamoyl, 2-thienylmethylcarbamoyl, 5-methylfuran-2-ylmethylcarbamoyl, cyclohexylethylcarbamoyl, 2-phenylethylcarbamoyl, 2-thienylethylcarbamoyl, 1-adamantyl-1-methylethylcarbamoyl, 6,6-dimethylbicyclo[3.1.1]heptan-2-ylcarbamoyl, cyclohexylcarbamoyl, cycloheptylcarbamoyl, cyclooctylcarbamoyl, 3-methylthiopropylcarbamoyl, 5-t-butylthiopentyl, 3-methoxypropylcarbamoyl etc.) optionally mono- or di-substituted by substituent(s) selected from
(1) a C₁₋₈ alkyl group optionally substituted by one or more substituents selected from
   (a) a C₃₋₁₀ cycloalkyl group,
   (b) a C₁₋₆ alkoxy group,
   (c) a C₁₋₆ alkylthio group
   (d) a phenyl group optionally substituted by one or more substituents selected from
      (i) a halogen atom, and
      (ii) a C₁₋₆ alkoxy group, and
   (e) a 5- or 6-membered monocyclic aromatic heterocyclic group, and
(2) a C₃₋₁₀ cycloalkyl group.

In the following, preferable compounds (I) are shown.

### [Compound (I)-A]

Compound (I) of the formula (I), wherein
A is a C₆₋₁₄ aryl group (preferably, phenyl group) optionally further substituted by the same or different 1 to 5 substituents selected from
   (1) a halogen atom;
   (2) a hydroxy group;
   (3) a carboxy group;
   (4) a sulfanyl (SH) group;
   (5) a C₁₋₆ alkyl group optionally substituted by 1 to 3 substituents selected from
      (a) a halogen atom,
      (b) a carboxy group,
      (c) a hydroxy group,
      (d) a C₁₋₆ alkoxy-carbonyl group,
      (e) a C₁₋₆ alkoxy group,
      (f) an amino group optionally mono- or di-substituted by a C₁₋₆ alkyl group, and
      (g) a cyano group;
   (6) a C₁₋₆ alkoxy group optionally substituted by 1 to 3 substituents selected from
      (a) a halogen atom,
      (b) a carboxy group,
      (c) a hydroxy group,
      (d) a C₁₋₆ alkoxy group,
      (e) a C₁₋₆ alkoxy-carbonyl group, and
      (f) an amino group optionally mono- or di-substituted by a C₁₋₆ alkyl group;
   (7) an amino group optionally mono- or di-substituted by a substituent selected from
      (a) a C₁₋₆ alkyl group optionally substituted by a halogen atom,
      (b) a C₁₋₆ alkanoyl group optionally substituted by a halogen atom,
      (c) a C₇₋₁₃ aroyl group optionally substituted by a halogen atom,
      (d) a C₁₋₆ alkylsulfonyl group optionally substituted by a halogen atom,
      (e) a C₆₋₁₀ arylsulfonyl group optionally substituted by a substituent selected from
         (i) a halogen atom, and
         (ii) a C₁₋₆ alkyl group, and
      (f) a C₁₋₆ alkoxy-carbonyl group optionally substituted by a halogen atom;
   (8) a C₁₋₆ alkylthio group optionally substituted by a halogen atom; and
   (9) a C₁₋₃ alkylenedioxy group;
B is a benzene ring optionally further substituted by 1 to 4 substituents selected from
   (1) a halogen atom;
   (2) a hydroxy group;
   (3) a sulfanyl (SH) group;
   (4) an amino group optionally mono- or di-substituted by a substituent selected from
      (a) a C₁₋₆ alkyl group optionally substituted by a halogen atom,
      (b) a C₁₋₆ alkanoyl group optionally substituted by a halogen atom,
      (c) a C₇₋₁₃ aroyl group optionally substituted by a halogen atom,
      (d) a C₁₋₆ alkylsulfonyl group optionally substituted by a halogen atom,
      (e) a C₆₋₁₀ arylsulfonyl group optionally substituted by a substituent selected from
         (i) a halogen atom, and
         (ii) a C₁₋₆ alkyl group optionally substituted by a halogen atom, and
      (f) a C₁₋₆ alkoxy-carbonyl group optionally substituted by a halogen atom;
   (5) a C₁₋₆ alkyl group optionally substituted by 1 to 3 substituents selected from
      (a) a halogen atom,
      (b) a carboxy group,
      (c) a hydroxy group,
      (d) a C₁₋₆ alkoxy-carbonyl group,
      (e) a C₁₋₆ alkoxy group,
      (f) an amino group optionally mono- or di-substituted by a C₁₋₆ alkyl group, and
      (g) a cyano group;
   (6) a C₁₋₆ alkoxy group optionally substituted by 1 to 3 substituents selected from
      (a) a halogen atom,
      (b) a carboxy group,
      (c) a hydroxy group,
      (d) a C₁₋₆ alkoxy group,
      (e) a C₁₋₆ alkoxy-carbonyl group, and
      (f) an amino group optionally mono- or di-substituted by a C₁₋₆ alkyl group;
   (7) a C₁₋₆ alkylthio group optionally substituted by a halogen atom; and
   (8) a C₁₋₃ alkylenedioxy group;
L is a bond or a methylene group;
R¹ is a hydrogen atom, or a C₁₋₆ alkyl group optionally substituted by one or more substituents selected from
   (1) a halogen atom;
   (2) a hydroxy group;
   (3) a sulfanyl group;
   (4) a carboxy group;
   (5) a C₁₋₆ alkoxy group;
   (6) an amino group optionally mono- or di-substituted by a C₁₋₆ alkyl group; and
   (7) a C₁₋₆ alkylthio group optionally substituted by a halogen atom;
Q is an oxygen atom;
R² and R³ are the same or different and are each independently a hydrogen atom, a carboxy group, a hydroxy group, or a C₁₋₁₀ alkyl group optionally substituted by one or more substituents selected from
   (1) a halogen atom;
   (2) a hydroxy group;
   (3) a sulfanyl group;
   (4) a carboxy group;
   (5) a C₁₋₆ alkoxy group;
   (6) an amino group optionally mono- or di-substituted by a C₁₋₆ alkyl group; and
   (7) a C₁₋₆ alkylthio group optionally substituted by a halogen atom;
R⁴ and R⁵ are the same or different and are each independently a hydrogen atom, or a C₁₋₆ alkyl group optionally substituted by one or more substituents selected from
   (1) a halogen atom;
   (2) a hydroxy group;
   (3) a carboxy group;
   (4) a guanidino group;
   (5) a cyano group;
   (6) a sulfanyl (SH) group;
   (7) an amino group optionally mono- or di-substituted by a substituent selected from
      (a) a C₁₋₆ alkyl group optionally substituted by a halogen atom,
      (b) a C₁₋₆ alkanoyl group optionally substituted by a halogen atom,
      (c) a C₇₋₁₃ aroyl group optionally substituted by a halogen atom,
      (d) a C₁₋₆ alkylsulfonyl group optionally substituted by a halogen atom,
      (e) a C₆₋₁₀ arylsulfonyl group optionally substituted by a substituent selected from
         (i) a halogen atom, and
         (ii) a C₁₋₆ alkyl group, and
      (f) a C₁₋₆ alkoxy-carbonyl group optionally substituted by a halogen atom;
   (8) a carbamoyl group optionally mono- or di-substituted by a C₁₋₆ alkyl group optionally substituted by a halogen atom;
   (9) a C₁₋₆ alkoxy-carbonyl group optionally substituted by a halogen atom;
   (10) a C₁₋₆ alkoxy group optionally substituted by one or more substituents selected from
      (a) a halogen atom,
      (b) a carboxy group,
      (c) a hydroxy group,
      (d) a C₁₋₆ alkoxy group,
      (e) a C₁₋₆ alkoxy-carbonyl group, and
      (f) an amino group optionally mono- or di-substituted by a C₁₋₆ alkyl group;
   (11) a C₁₋₆ alkylthio group optionally substituted by a halogen atom;
   (12) a C₃₋₁₀ cycloalkyl group optionally substituted by a substituent selected from
      (a) a halogen atom,
      (b) a hydroxy group,
      (c) a C₁₋₆ alkoxy group, and
      (d) a C₁₋₆ alkyl group; and
   (13) an aromatic ring group (e.g., C₆₋₁₄ aryl group such as phenyl group, naphthyl group and the like; 5- or 6-membered monocyclic aromatic nitrogen-containing heterocyclic group such as imidazolyl group and the like; 8- to 14-membered fused aromatic nitrogen-containing heterocyclic group such as indolyl group and the like etc.) optionally substituted by one or more substituents selected from
      (a) a C₁₋₆ alkyl group optionally substituted by one or more substituents selected from
         (i) a halogen atom,
         (ii) a carboxy group,
         (iii) a hydroxy group,
         (iv) a C₁₋₆ alkoxy group,
         (v) an amino group optionally mono- or di-substituted by a C₁₋₆ alkyl group, and
         (vi) a cyano group,
      (b) an amino group optionally mono- or di-substituted by a substituent selected from
         (i) a C₁₋₆ alkyl group optionally substituted by a halogen atom,
         (ii) a C₁₋₆ alkanoyl group optionally substituted by a halogen atom,
         (iii) a C₇₋₁₃ aroyl group optionally substituted by a halogen atom,
         (iv) a C₁₋₆ alkylsulfonyl group optionally substituted by a halogen atom,
         (v) a C₆₋₁₀ arylsulfonyl group optionally substituted by a substituent selected from
            (I) a halogen atom, and
            (II) a C₁₋₆ alkyl group, and
         (vi) a C₁₋₆ alkoxy-carbonyl group optionally substituted by a halogen atom,
      (c) a C₁₋₆ alkoxy group optionally substituted by one or more substituents selected from
         (i) a halogen atom,
         (ii) a C₁₋₆ alkoxy group,
         (iii) a carboxy group, and
         (iv) a hydroxy group,
      (d) a carbamoyl group optionally mono- or di-substituted by a C₁₋₆ alkyl group optionally substituted by a halogen atom,
      (e) a carboxy group,
      (f) a hydroxy group,
      (g) a halogen atom,
      (h) a sulfanyl (SH) group, and
      (i) a C₁₋₆ alkylthio group optionally substituted by a halogen atom;
n is 1; and
M is
   (1) a C₁₋₁₂ alkoxy-carbonyl group (e.g., tert-butoxycarbonyl group etc.) optionally substituted by one or more substituents selected from
      (a) a halogen atom,
      (b) a C₁₋₆ alkoxy group,
      (c) a carboxy group, and
      (d) a hydroxy group;
   (2) a carbamoyl group optionally mono- or di-substituted by substituent(s) selected from
      (a) a C₁₋₁₂ alkyl group optionally substituted by one or more substituents selected from
         (i) a C₃₋₁₀ cycloalkyl group,
         (ii) a C₁₋₆ alkoxy group,
         (iii) a halogen atom,
         (iv) a cyano group,
         (v) a C₁₋₆ alkoxy-carbonyl group,
         (vi) a phenyl group optionally substituted by one or more substituents selected from
            (I) a halogen atom, and
            (II) a C₁₋₆ alkoxy group, and
         (vii) an aromatic heterocyclic group optionally substituted by one or more substituents selected from
            (I) a halogen atom, and
            (II) a C₁₋₆ alkyl group, and
      (b) a C₃₋₁₀ cycloalkyl group; or
   (3) a monocyclic aromatic heterocyclic group optionally substituted by 1 to 3 substituents selected from
      (a) a C₁₋₆ alkyl group,
      (b) a sulfanyl (SH) group, and
      (c) a phenyl group optionally substituted by one or more substituents selected from
         (i) a halogen atom,
         (ii) a C₁₋₆ alkyl group optionally substituted by a halogen atom, and
         (iii) a C₁₋₆ alkoxy group optionally substituted by a halogen atom.

### [Compound (I)-B]

Compound (I) of the formula (I), wherein
A is a phenyl group optionally further substituted by a C₁₋₆ alkyl group optionally substituted by 1 to 3 substituents (preferably, a halogen atom) selected from
   (1) a halogen atom;
   (2) a carboxy group;
   (3) a hydroxy group;
   (4) a C₁₋₆ alkoxy-carbonyl group;
   (5) a C₁₋₆ alkoxy group;
   (6) an amino group optionally mono- or di-substituted by a C₁₋₆ alkyl group; and
   (7) a cyano group;
B is a benzene ring optionally substituted by 1 to 4 substituents selected from
   (1) a halogen atom (e.g., fluorine atom etc.); and
   (2) a C₁₋₆ alkoxy group (e.g., methoxy etc.);
L is a bond;
R¹ is a hydrogen atom;
Q is an oxygen atom;
R² and R³ are the same or different and are each independently a hydrogen atom or a C₁₋₁₀ alkyl group (e.g., methyl, heptyl etc.);
R⁴ is a hydrogen atom;
R⁵ is a C₁₋₆ alkyl group optionally substituted by one or more substituents selected from
   (1) a halogen atom;
   (2) a hydroxy group;
   (3) a carboxy group;
   (4) a guanidino group;
   (5) a cyano group;
   (6) a sulfanyl (SH) group;
   (7) an amino group optionally mono- or di-substituted by a substituent selected from
      (a) a C₁₋₆ alkyl group optionally substituted by a halogen atom,
      (b) a C₁₋₆ alkanoyl group optionally substituted by a halogen atom,
      (c) a C₇₋₁₃ aroyl group optionally substituted by a halogen atom,
      (d) a C₁₋₆ alkylsulfonyl group optionally substituted by a halogen atom,
      (e) a C₆₋₁₀ arylsulfonyl group optionally substituted by a substituent selected from
         (i) a halogen atom, and
         (ii) a C₁₋₆ alkyl group, and
      (f) a C₁₋₆ alkoxy-carbonyl group optionally substituted by a halogen atom;
   (8) a carbamoyl group optionally mono- or di-substituted by a C₁₋₆ alkyl group optionally substituted by a halogen atom;
   (9) a C₁₋₆ alkoxy-carbonyl group;
   (10) a C₁₋₆ alkoxy group optionally substituted by one or more substituents selected from
      (a) a halogen atom,
      (b) a carboxy group,
      (c) a hydroxy group,
      (d) a C₁₋₆ alkoxy group,
      (e) a C₁₋₆ alkoxy-carbonyl group, and
      (f) an amino group optionally mono- or di-substituted by a C₁₋₆ alkyl group;
   (11) a C₁₋₆ alkylthio group optionally substituted by a halogen atom;
   (12) a C₃₋₁₀ cycloalkyl group optionally substituted by a substituent selected from
      (a) a halogen atom,
      (b) a hydroxy group,
      (c) a C₁₋₆ alkoxy group, and
      (d) a C₁₋₆ alkyl group; and
   (13) an aromatic ring group (e.g., C₆₋₁₄ aryl group such as phenyl group, naphthyl group and the like; 5- or 6-membered monocyclic aromatic nitrogen-containing heterocyclic group such as imidazolyl group and the like; 8- to 14-membered fused aromatic nitrogen-containing heterocyclic group such as indolyl group and the like etc.) optionally substituted by one or more substituents selected from
      (a) a C₁₋₆ alkyl group optionally substituted by one or more substituents selected from
         (i) a halogen atom,
         (ii) a carboxy group,
         (iii) a hydroxy group,
         (iv) a C₁₋₆ alkoxy group,
         (v) an amino group optionally mono- or di-substituted by a C₁₋₆ alkyl group, and
         (vi) a cyano group,
      (b) an amino group optionally mono- or di-substituted by a substituent selected from
         (i) a C₁₋₆ alkyl group optionally substituted by a halogen atom,
         (ii) a C₁₋₆ alkanoyl group optionally substituted by a halogen atom,
         (iii) a C₇₋₁₃ aroyl group optionally substituted by a halogen atom,
         (iv) a C₁₋₆ alkylsulfonyl group optionally substituted by a halogen atom,
         (v) a C₆₋₁₀ arylsulfonyl group optionally substituted by a substituent selected from
            (I) a halogen atom, and
            (II) a C₁₋₆ alkyl group, and
         (vi) a C₁₋₆ alkoxy-carbonyl group optionally substituted by a halogen atom,
      (c) a C₁₋₆ alkoxy group optionally substituted by one or more substituents selected from
         (i) a halogen atom,
         (ii) a C₁₋₆ alkoxy group,
         (iii) a carboxy group, and
         (iv) a hydroxy group,
      (d) a carbamoyl group optionally mono- or di-substituted by a C₁₋₆ alkyl group optionally substituted by a halogen atom,
      (e) a carboxy group,
      (f) a hydroxy group,
      (g) a halogen atom,
      (h) a sulfanyl (SH) group, and
      (i) a C₁₋₆ alkylthio group optionally substituted by a halogen atom;
n is 1; and
M is
   (1) a C₁₋₁₂ alkoxy-carbonyl group optionally substituted by one or more substituents selected from
      (a) a halogen atom,
      (b) a C₁₋₆ alkoxy group,
      (c) a carboxy group, and
      (d) a hydroxy group;
   (2) a carbamoyl group (e.g., 2-ethylhexylcarbamoyl, 1,5-dimethylhexylcarbamoyl, 1-methylhexylcarbamoyl, octylcarbamoyl, hexyl(methyl)carbamoyl, pentylcarbamoyl, hexylcarbamoyl, heptylcarbamoyl, 2,2-dimethylpropylcarbamoyl, 2-ethylbutylcarbamoyl, 1-methylhexylcarbamoyl, cyanomethylcarbamoyl, 2,2,2-trifluoroethylcarbamoyl, 2-methyl-2-methoxypropylcarbamoyl, 1-methoxycarbonylpentylcarbamoyl, cyclohexylmethylcarbamoyl, cyclopentylmethylcarbamoyl, benzylcarbamoyl, 1-phenylethylcarbamoyl, 4-methoxybenzylcarbamoyl, 4-fluorobenzylcarbamoyl, 2-thienylmethylcarbamoyl, 5-methylfuran-2-ylmethylcarbamoyl, cyclohexylethylcarbamoyl, 2-phenylethylcarbamoyl, 2-thienylethylcarbamoyl, 1-adamantyl-1-methylethylcarbamoyl, 6,6-dimethylbicyclo[3.1.1]heptan-2-ylcarbamoyl, cyclohexylcarbamoyl, cycloheptylcarbamoyl, cyclooctylcarbamoyl etc.) optionally mono- or di-substituted by substituent(s) selected from
      (a) a C₁₋₈ alkyl group optionally substituted by one or more substituents selected from
         (i) a C₃₋₁₀ cycloalkyl group,
         (ii) a C₁₋₆ alkoxy group,
         (iii) a phenyl group optionally substituted by one or more substituents selected from
            (I) a halogen atom, and
            (II) a C₁₋₆ alkoxy group, and
         (iv) a 5- or 6-membered monocyclic aromatic heterocyclic group, and
      (b) a C₃₋₁₀ cycloalkyl group; or
   (3) a 5- or 6-membered monocyclic aromatic heterocyclic group (e.g., triazolyl group, isoxazolyl group, oxazolyl group, oxadiazolyl group, tetrazolyl group etc.) optionally substituted by 1 to 3 substituents selected from
      (a) a C₁₋₄ alkyl group,
      (b) a sulfanyl (SH) group, and
      (c) a phenyl group optionally substituted by one or more substituents selected from
         (i) a halogen atom,
         (ii) a C₁₋₆ alkyl group optionally substituted by a halogen atom, and
         (iii) a C₁₋₆ alkoxy group optionally substituted by a halogen atom.

### [Compound (I)-C]

Compound (I) of the formula (I), wherein
A is a phenyl group substituted by 1 to 3 C₁₋₆ alkyl groups;
B is a benzene ring without a further substituent;
L is a bond;
R¹ is a hydrogen atom;
Q is an oxygen atom;
R² and R³ are the same or different and are each independently a hydrogen atom or a C₁₋₁₀ alkyl group (e.g., methyl, heptyl etc.);
R⁴ is a hydrogen atom;
R⁵ is a C₁₋₄ alkyl group (e.g., methyl, ethyl, butyl, isobutyl etc.) optionally substituted by a substituent selected from
   (1) a C₃₋₁₀ cycloalkyl group (e.g., cyclohexyl group etc.) optionally substituted by a substituent selected from
      (a) a halogen atom,
      (b) a hydroxy group,
      (c) a C₁₋₆ alkoxy group, and
      (d) a C₁₋₆ alkyl group;
   (2) a phenyl group (e.g., 4-fluorophenyl, 4-chlorophenyl, 4-tert-butylphenyl etc.) optionally substituted by one or more substituents selected from
      (a) a halogen atom, and
      (b) a C₁₋₆ alkoxy group;
   (3) a C₁₋₆ alkylthio group (e.g., methylthio etc.); and
   (4) a 8- to 14-membered fused aromatic nitrogen-containing heterocyclic group (e.g., indolyl group etc.) optionally substituted by one or more substituents selected from
      (a) a halogen atom, and
      (b) a C₁₋₆ alkyl group;
         n is 1; and
         M is a C₁₋₁₂ alkoxy-carbonyl group (e.g., tert-butoxycarbonyl etc.).

### [Compound (I) -D]

Compound (I) of the formula (I), wherein
A is a phenyl group substituted by 1 to 3 C₁₋₆ alkyl groups;
B is a benzene ring without a further substituent;
L is a bond;
R¹ is a hydrogen atom;
Q is an oxygen atom;
R² and R³ are the same or different and are each independently a hydrogen atom or a C₁₋₁₀ alkyl group (e.g., methyl, heptyl etc.);
R⁴ is a hydrogen atom;
R⁵ is a C₁₋₄ alkyl group (e.g., methyl, ethyl, butyl, isobutyl etc.) optionally substituted by a substituent selected from
   (1) a C₃₋₁₀ cycloalkyl group (e.g., cyclohexyl group) optionally substituted by a substituent selected from
      (a) a halogen atom,
      (b) a hydroxy group,
      (c) a C₁₋₆ alkoxy group, and
      (d) a C₁₋₆ alkyl group;
   (2) a phenyl group (e.g., 4-fluorophenyl, 4-chlorophenyl, 4-tert-butylphenyl etc.) optionally substituted by one or more substituents selected from
      (a) a halogen atom, and
      (b) a C₁₋₆ alkoxy group;
   (3) a C₁₋₆ alkylthio group (e.g., methylthio etc.); and
   (4) a 8- to 14-membered fused aromatic nitrogen-containing heterocyclic group (e.g., indolyl group etc.) optionally substituted by one or more substituents selected from
      (a) a halogen atom, and
      (b) a C₁₋₆ alkyl group;
n is 1; and
M is a carbamoyl group (e.g., 2-ethylhexylcarbamoyl, 1,5-dimethylhexylcarbamoyl, 1-methylhexylcarbamoyl, octylcarbamoyl, hexyl (methyl) carbamoyl etc.) mono-substituted by a C₁₋₁₂ alkyl group.

### [Compound (I)-E]

Compound (I) of the formula (I), wherein
A is a phenyl group substituted by 1 to 3 C₁₋₆ alkyl groups;
B is a benzene ring without a further substituent;
L is a bond;
R¹ is a hydrogen atom;
Q is an oxygen atom;
R² and R³ are the same or different and are each independently a hydrogen atom or a C₁₋₁₀ alkyl group (e.g., methyl, heptyl etc.);
R⁴ is a hydrogen atom;
R⁵ is a C₁₋₄ alkyl group (e.g., methyl, ethyl, butyl, isobutyl etc.) optionally substituted by a substituent selected from
   (1) a C₃₋₁₀ cycloalkyl group (e.g., cyclohexyl group etc.) optionally substituted by a substituent selected from
      (a) a halogen atom,
      (b) a hydroxy group,
      (c) a C₁₋₆ alkoxy group, and
      (d) a C₁₋₆ alkyl group;
   (2) a phenyl group (e.g., 4-fluorophenyl, 4-chlorophenyl, 4-tert-butylphenyl etc.) optionally substituted by one or more substituents selected from
      (a) a halogen atom, and
      (b) a C₁₋₆ alkoxy group;
   (3) a C₁₋₆ alkylthio group (e.g., methylthio etc.); and
   (4) a 8- to 14-membered fused aromatic nitrogen-containing heterocyclic group (e.g., indolyl group etc.) optionally substituted by one or more substituents selected from
      (a) a halogen atom, and
      (b) a C₁₋₆ alkyl group;
n is 1; and
M is a 5-membered monocyclic aromatic heterocyclic group (preferably, triazolyl group, isoxazolyl group, oxazolyl group, oxadiazolyl group, tetrazolyl group and the like, more preferably, oxadiazolyl group) (e.g., 3-(4-propyloxyphenyl)-1,2,4-oxadiazol-5-yl, 3-[4-(trifluoromethoxy)phenyl]-1,2,4-oxadiazol-5-yl etc.) optionally substituted by a phenyl group optionally substituted by a C₁₋₆ alkoxy group optionally substituted by a halogen atom.

As other preferable compound (I), the following compounds can be mentioned.

### [compound (I)-A']

Compound (I) of the formula (I), wherein
A is a C₆₋₁₄ aryl group (preferably, phenyl group) optionally further substituted by the same or different 1 to 5 substituents selected from
   (1) a halogen atom;
   (2) a hydroxy group;
   (3) a carboxy group;
   (4) a sulfanyl (SH) group;
   (5) a C₁₋₆ alkyl group optionally substituted by 1 to 3 substituents selected from
      (a) a halogen atom,
      (b) a carboxy group,
      (c) a hydroxy group,
      (d) a C₁₋₆ alkoxy-carbonyl group,
      (e) a C₁₋₆ alkoxy group,
      (f) an amino group optionally mono- or di-substituted by a C₁₋₆ alkyl group, and
      (g) a cyano group;
   (6) a C₁₋₆ alkoxy group optionally substituted by 1 to 3 substituents selected from
      (a) a halogen atom,
      (b) a carboxy group,
      (c) a hydroxy group,
      (d) a C₁₋₆ alkoxy group,
      (e) a C₁₋₆ alkoxy-carbonyl group, and
      (f) an amino group optionally mono- or di-substituted by a C₁₋₆ alkyl group;
   (7) an amino group optionally mono- or di-substituted by a substituent selected from
      (a) a C₁₋₆ alkyl group optionally substituted by a halogen atom,
      (b) a C₁₋₆ alkanoyl group optionally substituted by a halogen atom,
      (c) a C₇₋₁₃ aroyl group optionally substituted by a halogen atom,
      (d) a C₁₋₆ alkylsulfonyl group optionally substituted by a halogen atom,
      (e) a C₆₋₁₀ arylsulfonyl group optionally substituted by a substituent selected from
         (i) a halogen atom, and
         (ii) a C₁₋₆ alkyl group, and
      (f) a C₁₋₆ alkoxy-carbonyl group optionally substituted by a halogen atom;
   (8) a C₁₋₆ alkylthio group optionally substituted by a halogen atom; and
   (9) a C₁₋₃ alkylenedioxy group; or a 5- or 6-membered monocyclic aromatic heterocyclic group (e.g., pyridyl group) optionally further substituted by a substituent selected from
      (1) a halogen atom;
      (2) a hydroxy group;
      (3) a carboxy group;
      (4) a sulfanyl (SH) group;
      (5) a C₁₋₆ alkyl group optionally substituted by 1 to 3 substituents selected from
         (a) a halogen atom,
         (b) a carboxy group,
         (c) a hydroxy group,
         (d) a C₁₋₆ alkoxy-carbonyl group,
         (e) a C₁₋₆ alkoxy group,
         (f) an amino group optionally mono- or di-substituted by a C₁₋₆ alkyl group, and
         (g) a cyano group;
      (6) a C₁₋₆ alkoxy group optionally substituted by 1 to 3 substituents selected from
         (a) a halogen atom,
         (b) a carboxy group,
         (c) a hydroxy group,
         (d) a C₁₋₆ alkoxy group,
         (e) a C₁₋₆ alkoxy-carbonyl group, and
         (f) an amino group optionally mono- or di-substituted by a C₁₋₆ alkyl group;
      (7) an amino group optionally mono- or di-substituted by a substituent selected from
         (a) a C₁₋₆ alkyl group optionally substituted by a halogen atom,
         (b) a C₁₋₆ alkanoyl group optionally substituted by a halogen atom,
         (c) a C₇₋₁₃ aroyl group optionally substituted by a halogen atom,
         (d) a C₁₋₆ alkylsulfonyl group optionally substituted by a halogen atom,
         (e) a C₆₋₁₀ arylsulfonyl group optionally substituted by a substituent selected from
            (i) a halogen atom, and
            (ii) a C₁₋₆ alkyl group, and
         (f) a C₁₋₆ alkoxy-carbonyl group optionally substituted by a halogen atom;
      (8) a 4- to 7-membered monocyclic non-aromatic heterocyclic group;
      (9) a C₁₋₆ alkylthio group optionally substituted by a halogen atom; and
      (10) a C₁₋₃ alkylenedioxy group;
B is a benzene ring optionally further substituted by 1 to 4 substituents selected from
   (1) a halogen atom;
   (2) a hydroxy group;
   (3) a sulfanyl (SH) group;
   (4) an amino group optionally mono- or di-substituted by a substituent selected from
      (a) a C₁₋₆ alkyl group optionally substituted by a halogen atom,
      (b) a C₁₋₆ alkanoyl group optionally substituted by a halogen atom,
      (c) a C₇₋₁₃ aroyl group optionally substituted by a halogen atom,
      (d) a C₁₋₆ alkylsulfonyl group optionally substituted by a halogen atom,
      (e) a C₆₋₁₀ arylsulfonyl group optionally substituted by a substituent selected from
         (i) a halogen atom, and
         (ii) a C₁₋₆ alkyl group optionally substituted by a halogen atom, and
      (f) a C₁₋₆ alkoxy-carbonyl group optionally substituted by a halogen atom;
   (5) a C₁₋₆ alkyl group optionally substituted by 1 to 3 substituents selected from
      (a) a halogen atom,
      (b) a carboxy group,
      (c) a hydroxy group,
      (d) a C₁₋₆ alkoxy-carbonyl group,
      (e) a C₁₋₆ alkoxy group,
      (f) an amino group optionally mono- or di-substituted by a C₁₋₆ alkyl group, and
      (g) a cyano group;
   (6) a C₁₋₆ alkoxy group optionally substituted by 1 to 3 substituents selected from
      (a) a halogen atom,
      (b) a carboxy group,
      (c) a hydroxy group,
      (d) a C₁₋₆ alkoxy group,
      (e) a C₁₋₆ alkoxy-carbonyl group, and
      (f) an amino group optionally mono- or di-substituted by a C₁₋₆ alkyl group;
   (7) a C₁₋₆ alkylthio group optionally substituted by a halogen atom;
   (8) a C₁₋₃ alkylenedioxy group; and
   (9) a 4- to 7-membered monocyclic non-aromatic heterocyclic group (e.g., 4-morpholinyl);
L is a bond or a methylene group;
R¹ is a hydrogen atom, or a C₁₋₆ alkyl group optionally substituted by one or more substituents selected from
   (1) a halogen atom;
   (2) a hydroxy group;
   (3) a sulfanyl group;
   (4) a carboxy group;
   (5) a C₁₋₆ alkoxy group;
   (6) an amino group optionally mono- or di-substituted by a C₁₋₆ alkyl group; and
   (7) a C₁₋₆ alkylthio group optionally substituted by a halogen atom;
Q is an oxygen atom;
R² and R³ are the same or different and are each independently a hydrogen atom, a carboxy group, a hydroxy group, or a C₁₋₁₀ alkyl group optionally substituted by one or more substituents selected from
   (1) a halogen atom;
   (2) a hydroxy group;
   (3) a sulfanyl group;
   (4) a carboxy group;
   (5) a C₁₋₆ alkoxy group;
   (6) an amino group optionally mono- or di-substituted by a C₁₋₆ alkyl group; and
   (7) a C₁₋₆ alkylthio group optionally substituted by a halogen atom;
R⁴ and R⁵ are the same or different and are each independently a hydrogen atom, or a C₁₋₆ alkyl group optionally substituted by one or more substituents selected from
   (1) a halogen atom;
   (2) a hydroxy group;
   (3) a carboxy group;
   (4) a guanidino group;
   (5) a cyano group;
   (6) a sulfanyl (SH) group;
   (7) an amino group optionally mono- or di-substituted by a substituent selected from
      (a) a C₁₋₆ alkyl group optionally substituted by a halogen atom,
      (b) a C₁₋₆ alkanoyl group optionally substituted by a halogen atom,
      (c) a C₇₋₁₃ aroyl group optionally substituted by a halogen atom,
      (d) a C₁₋₆ alkylsulfonyl group optionally substituted by a halogen atom,
      (e) a C₆₋₁₀ arylsulfonyl group optionally substituted by a substituent selected from
         (i) a halogen atom, and
         (ii) a C₁₋₆ alkyl group, and
      (f) a C₁₋₆ alkoxy-carbonyl group optionally substituted by a halogen atom;
   (8) a carbamoyl group optionally mono- or di-substituted by a C₁₋₆ alkyl group optionally substituted by a halogen atom;
   (9) a C₁₋₆ alkoxy-carbonyl group optionally substituted by a halogen atom;
   (10) a C₁₋₆ alkoxy group optionally substituted by one or more substituents selected from
      (a) a halogen atom,
      (b) a carboxy group,
      (c) a hydroxy group,
      (d) a C₁₋₆ alkoxy group,
      (e) a C₁₋₆ alkoxy-carbonyl group, and
      (f) an amino group optionally mono- or di-substituted by a C₁₋₆ alkyl group;
   (11) a C₁₋₆ alkylthio group optionally substituted by a halogen atom;
   (12) a C₃₋₁₀ cycloalkyl group optionally substituted by a substituent selected from
      (a) a halogen atom,
      (b) a hydroxy group,
      (c) a C₁₋₆ alkoxy group, and
      (d) a C₁₋₆ alkyl group; and
   (13) an aromatic ring group (e.g., C₆₋₁₄ aryl group such as phenyl group, naphthyl group and the like; 5- or 6-membered monocyclic aromatic nitrogen-containing heterocyclic group such as imidazolyl group, pyridyl group and the like; 8- to 14-membered fused aromatic nitrogen-containing heterocyclic group such as indolyl group and the like etc.) optionally substituted by one or more substituents selected from
      (a) a C₁₋₆ alkyl group optionally substituted by one or more substituents selected from
         (i) a halogen atom,
         (ii) a carboxy group,
         (iii) a hydroxy group,
         (iv) a C₁₋₆ alkoxy group,
         (v) an amino group optionally mono- or di-substituted by a C₁₋₆ alkyl group, and
         (vi) a cyano group,
      (b) an amino group optionally mono- or di-substituted by a substituent selected from
         (i) a C₁₋₆ alkyl group optionally substituted by a halogen atom,
         (ii) a C₁₋₆ alkanoyl group optionally substituted by a halogen atom,
         (iii) a C₇₋₁₃ aroyl group optionally substituted by a halogen atom,
         (iv) a C₁₋₆ alkylsulfonyl group optionally substituted by a halogen atom,
         (v) a C₆₋₁₀ arylsulfonyl group optionally substituted by a substituent selected from
            (I) a halogen atom, and
            (II) a C₁₋₆ alkyl group, and
         (vi) a C₁₋₆ alkoxy-carbonyl group optionally substituted by a halogen atom,
      (c) a C₁₋₆ alkoxy group optionally substituted by one or more substituents selected from
         (i) a halogen atom,
         (ii) a C₁₋₆ alkoxy group,
         (iii) a carboxy group, and
         (iv) a hydroxy group,
      (d) a carbamoyl group optionally mono- or di-substituted by a C₁₋₆ alkyl group optionally substituted by a halogen atom,
      (e) a carboxy group,
      (f) a hydroxy group,
      (g) a halogen atom,
      (h) a sulfanyl (SH) group, and
      (i) a C₁₋₆ alkylthio group optionally substituted by a halogen atom;
n is 1; and
M is
   (1) a C₁₋₁₂ alkoxy-carbonyl group (e.g., tert-butoxycarbonyl group etc.) optionally substituted by one or more substituents selected from
      (a) a halogen atom,
      (b) a C₁₋₆ alkoxy group,
      (c) a carboxy group, and
      (d) a hydroxy group;
   (2) a carbamoyl group optionally mono- or di-substituted by substituent(s) selected from
      (a) a C₁₋₁₂ alkyl group optionally substituted by one or more substituents selected from
         (i) a C₃₋₁₀ cycloalkyl group,
         (ii) a C₁₋₆ alkoxy group,
         (iii) a halogen atom,
         (iv) a cyano group,
         (v) a C₁₋₆ alkoxy-carbonyl group,
         (vi) a C₁₋₆ alkylthio group optionally substituted by a halogen atom,
         (vii) a phenyl group optionally substituted by one or more substituents selected from
            (I) a halogen atom, and
            (II) a C₁₋₆ alkoxy group, and
         (viii) an aromatic heterocyclic group optionally substituted by one or more substituents selected from
            (I) a halogen atom, and
            (II) a C₁₋₆ alkyl group, and
      (b) a C₃₋₁₀ cycloalkyl group; or
   (3) a monocyclic aromatic heterocyclic group optionally substituted by 1 to 3 substituents selected from
      (a) a C₁₋₆ alkyl group,
      (b) a sulfanyl (SH) group, and
      (c) a phenyl group optionally substituted by one or more substituents selected from
         (i) a halogen atom,
         (ii) a C₁₋₆ alkyl group optionally substituted by a halogen atom, and
         (iii) a C₁₋₆ alkoxy group optionally substituted by a halogen atom.

### [Compound (I)-B']

Compound (I) of the formula (I), wherein
A is
   (1) a phenyl group optionally further substituted by a C₁₋₆ alkyl group optionally substituted by 1 to 3 substituents selected from
      (a) a halogen atom,
      (b) a carboxy group,
      (c) a hydroxy group,
      (d) a C₁₋₆ alkoxy-carbonyl group,
      (e) a C₁₋₆ alkoxy group,
      (f) an amino group optionally mono- or di-substituted by a C₁₋₆ alkyl group, and
      (g) a cyano group; or
   (2) a pyridyl group (e.g., 2-pyridyl, 3-pyridyl, 4-pyridyl) optionally further substituted by 1 to 3 of
      (a) an amino group optionally mono- or di-substituted by a C₁₋₆ alkyl group (e.g., n-hexylamino), or
      (b) a 4- to 7-membered monocyclic non-aromatic nitrogen-containing heterocyclic group (e.g., 4-morpholinyl);
B is a benzene ring optionally further substituted by 1 to 4 substituents selected from
   (1) a halogen atom (e.g., fluorine atom etc.);
   (2) a C₁₋₆ alkoxy group (e.g., methoxy etc.); and
   (3) a 4- to 7-membered monocyclic non-aromatic heterocyclic group (e.g., 4-morpholinyl);
L is a bond;
R¹ is a hydrogen atom, or a C₁₋₆ alkyl group substituted by a C₁₋₆ alkylthio group optionally substituted by a halogen atom;
Q is an oxygen atom;
R² and R³ are the same or different and are each independently a hydrogen atom, or a C₁₋₁₀ alkyl group (e.g., methyl, heptyl, carboxymethyl etc.) optionally substituted by a carboxy group;
R⁴ is a hydrogen atom;
R⁵ is a C₁₋₆ alkyl group optionally substituted by one or more substituents selected from
   (1) a halogen atom;
   (2) a hydroxy group;
   (3) a carboxy group;
   (4) a guanidino group;
   (5) a cyano group;
   (6) a sulfanyl (SH) group;
   (7) an amino group optionally mono- or di-substituted by a substituent selected from
      (a) a C₁₋₆ alkyl group optionally substituted by a halogen atom,
      (b) a C₁₋₆ alkanoyl group optionally substituted by a halogen atom,
      (c) a C₇₋₁₃ aroyl group optionally substituted by a halogen atom,
      (d) a C₁₋₆ alkylsulfonyl group optionally substituted by a halogen atom,
      (e) a C₆₋₁₀ arylsulfonyl group optionally substituted by a substituent selected from
         (i) a halogen atom, and
         (ii) a C₁₋₆ alkyl group, and
      (f) a C₁₋₆ alkoxy-carbonyl group optionally substituted by a halogen atom;
(8) a carbamoyl group optionally mono- or di-substituted by a C₁₋₆ alkyl group optionally substituted by a halogen atom;
(9) a C₁₋₆ alkoxy-carbonyl group;
(10) a C₁₋₆ alkoxy group optionally substituted by one or more substituents selected from
   (a) a halogen atom,
   (b) a carboxy group,
   (c) a hydroxy group,
   (d) a C₁₋₆ alkoxy group,
   (e) a C₁₋₆ alkoxy-carbonyl group, and
   (f) an amino group optionally mono- or di-substituted by a C₁₋₆ alkyl group;
(11) a C₁₋₆ alkylthio group optionally substituted by a halogen atom;
(12) a C₃₋₁₀ cycloalkyl group optionally substituted by a substituent selected from
   (a) a halogen atom,
   (b) a hydroxy group,
   (c) a C₁₋₆ alkoxy group, and
   (d) a C₁₋₆ alkyl group; and
(13) an aromatic ring group (e.g., C₆₋₁₄ aryl group such as phenyl group, naphthyl group and the like; 5- or 6-membered monocyclic aromatic nitrogen-containing heterocyclic group such as imidazolyl group, pyridyl group and the like; 8- to 14-membered fused aromatic nitrogen-containing heterocyclic group such as indolyl group and the like etc.) optionally substituted by one or more substituents selected from
   (a) a C₁₋₆ alkyl group optionally substituted by one or more substituents selected from
      (i) a halogen atom,
      (ii) a carboxy group,
      (iii) a hydroxy group,
      (iv) a C₁₋₆ alkoxy group,
      (v) an amino group optionally mono- or di-substituted by a C₁₋₆ alkyl group, and
      (vi) a cyano group,
   (b) an amino group optionally mono- or di-substituted by a substituent selected from
      (i) a C₁₋₆ alkyl group optionally substituted by a halogen atom,
      (ii) a C₁₋₆ alkanoyl group optionally substituted by a halogen atom,
      (iii) a C₇₋₁₃ aroyl group optionally substituted by a halogen atom,
      (iv) a C₁₋₆ alkylsulfonyl group optionally substituted by a halogen atom,
      (v) a C₆₋₁₀ arylsulfonyl group optionally substituted by a substituent selected from
         (I) a halogen atom, and
         (II) a C₁₋₆ alkyl group, and
      (vi) a C₁₋₆ alkoxy-carbonyl group optionally substituted by a halogen atom,
   (c) a C₁₋₆ alkoxy group optionally substituted by one or more substituents selected from
      (i) a halogen atom,
      (ii) a C₁₋₆ alkoxy group,
      (iii) a carboxy group, and
      (iv) a hydroxy group,
   (d) a carbamoyl group optionally mono- or di-substituted by a C₁₋₆ alkyl group optionally substituted by a halogen atom,
   (e) a carboxy group,
   (f) a hydroxy group,
   (g) a halogen atom,
   (h) a sulfanyl (SH) group, and
   (i) a C₁₋₆ alkylthio group optionally substituted by a halogen atom;
n is 1; and
M is
   (1) a C₁₋₁₂ alkoxy-carbonyl group optionally substituted by one or more substituents selected from
      (a) a halogen atom,
      (b) a C₁₋₆ alkoxy group,
      (c) a carboxy group, and
      (d) a hydroxy group;
   (2) a carbamoyl group (e.g., 2-ethylhexylcarbamoyl, 1,5-dimethylhexylcarbamoyl, 1-methylhexylcarbamoyl, octylcarbamoyl, hexyl(methyl)carbamoyl, pentylcarbamoyl, hexylcarbamoyl, heptylcarbamoyl, 2,2-dimethylpropylcarbamoyl, 2-ethylbutylcarbamoyl, 1-methylhexylcarbamoyl, cyanomethylcarbamoyl, 2,2,2-trifluoroethylcarbamoyl, 2-methyl-2-methoxypropylcarbamoyl, 1-methoxycarbonylpentylcarbamoyl, cyclohexylmethylcarbamoyl, cyclopentylmethylcarbamoyl, benzylcarbamoyl, 1-phenylethylcarbamoyl, 4-methoxybenzylcarbamoyl, 4-fluorobenzylcarbamoyl, 2-thienylmethylcarbamoyl, 5-methylfuran-2-ylmethylcarbamoyl, cyclohexylethylcarbamoyl, 2-phenylethylcarbamoyl, 2-thienylethylcarbamoyl, 1-adamantyl-1-methylethylcarbamoyl, 6,6-dimethylbicyclo[3.1.1]heptan-2-ylcarbamoyl, cyclohexylcarbamoyl, cycloheptylcarbamoyl, cyclooctylcarbamoyl, 3-methylthiopropylcarbamoyl, 5-t-butylthiopentyl, 3-methoxypropylcarbamoyl etc.) optionally mono- or di-substituted by substituent(s) selected from
      (a) a C₁₋₈ alkyl group optionally substituted by one or more substituents selected from
         (i) a C₃₋₁₀ cycloalkyl group,
         (ii) a C₁₋₆ alkoxy group,
         (iii) a C₁₋₆ alkylthio group,
         (iv) a phenyl group optionally substituted by one or more substituents selected from
            (I) a halogen atom, and
            (II) a C₁₋₆ alkoxy group, and
         (v) a 5- or 6-membered monocyclic aromatic heterocyclic group, and
      (b) a C₃₋₁₀ cycloalkyl group; or
   (3) a 5- or 6-membered monocyclic aromatic heterocyclic group (e.g., triazolyl group, isoxazolyl group, oxazolyl group, oxadiazolyl group, tetrazolyl group etc.) optionally substituted by 1 to 3 substituents selected from
      (a) a C₁₋₄ alkyl group,
      (b) a sulfanyl (SH) group, and
      (c) a phenyl group optionally substituted by one or more substituents selected from
         (i) a halogen atom,
         (ii) a C₁₋₆ alkyl group optionally substituted by a halogen atom, and
         (iii) a C₁₋₆ alkoxy group optionally substituted by a halogen atom.

### [Compound (I)-C']

Compound (I) of the formula (I), wherein
A is a phenyl group substituted by 1 to 3 C₁₋₆ alkyl groups;
B is a benzene ring without a further substituent;
L is a bond;
R¹ is a hydrogen atom;
Q is an oxygen atom;
R² and R³ are the same or different and are each independently a hydrogen atom or a C₁₋₁₀ alkyl group (e.g., methyl, heptyl etc.);
R⁴ is a hydrogen atom;
R⁵ is a C₁₋₄ alkyl group (e.g., methyl, ethyl, butyl, isobutyl etc.) optionally substituted by a substituent selected from
   (1) a C₃₋₁₀ cycloalkyl group (e.g., cyclohexyl group etc.) optionally substituted by a substituent selected from
      (a) a halogen atom,
      (b) a hydroxy group,
      (c) a C₁₋₆ alkoxy group, and
      (d) a C₁₋₆ alkyl group;
   (2) a phenyl group (e.g., 4-fluorophenyl, 4-chlorophenyl, 4-tert-butylphenyl etc.) optionally substituted by one or more substituents selected from
      (a) a halogen atom, and
      (b) a C₁₋₆ alkoxy group;
   (3) a C₁₋₆ alkylthio group (e.g., methylthio etc.);
   (4) a 5- or 6-membered monocyclic aromatic nitrogen-containing heterocyclic group (e.g., pyridyl group etc.); and
   (5) a 8- to 14-membered fused aromatic nitrogen-containing heterocyclic group (e.g., indolyl group etc.);
n is 1; and
M is a C₁₋₁₂ alkoxy-carbonyl group (e.g., tert-butoxycarbonyl etc.).

### [Compound (I)-D']

Compound (I) of the formula (I), wherein
A is a phenyl group substituted by 1 to 3 C₁₋₆ alkyl groups;
B is a benzene ring without a further substituent;
L is a bond;
R¹ is a hydrogen atom;
Q is an oxygen atom;
R² and R³ are the same or different and are each independently a hydrogen atom or a C₁₋₁₀ alkyl group (e.g., methyl, heptyl etc.);
R⁴ is a hydrogen atom;
R⁵ is a C₁₋₄ alkyl group (e.g., methyl, ethyl, butyl, isobutyl etc.) optionally substituted by a substituent selected from
   (1) a C₃₋₁₀ cycloalkyl group (e.g., cyclohexyl group etc.) optionally substituted by a substituent selected from
      (a) a halogen atom,
      (b) a hydroxy group,
      (c) a C₁₋₆ alkoxy group, and
      (d) a C₁₋₆ alkyl group;
   (2) a phenyl group (e.g., 4-fluorophenyl, 4-chlorophenyl, 4-tert-butylphenyl etc.) optionally substituted by one or more substituents selected from
      (a) a halogen atom, and
      (b) a C₁₋₆ alkoxy group;
   (3) a C₁₋₆ alkylthio group (e.g., methylthio etc.);
   (4) a 5- or 6-membered monocyclic aromatic nitrogen-containing heterocyclic group (e.g., pyridyl group etc.); and
   (5) a 8- to 14-membered fused aromatic nitrogen-containing heterocyclic group (e.g., indolyl group etc.);
n is 1; and
M is a carbamoyl group (e.g., 2-ethylhexylcarbamoyl, 1,5-dimethylhexylcarbamoyl, 1-methylhexylcarbamoyl, octylcarbamoyl, hexyl (methyl) carbamoyl etc.) mono-substituted by a C₁₋₁₂ alkyl group.

### [Compound (I)-E']

Compound (I) of the formula (I), wherein
A is a phenyl group substituted by 1 to 3 C₁₋₆ alkyl groups;
B is a benzene ring without a further substituent;
L is a bond;
R¹ is a hydrogen atom;
Q is an oxygen atom;
R² and R³ are the same or different and are each independently a hydrogen atom or a C₁₋₁₀ alkyl group (e.g., methyl, heptyl etc.);
R⁴ is a hydrogen atom;
R⁵ is a C₁₋₄ alkyl group (e.g., methyl, ethyl, butyl, isobutyl etc.) optionally substituted by a substituent selected from
   (1) a C₃₋₁₀ cycloalkyl group (e.g., cyclohexyl group etc.) optionally substituted by a substituent selected from
      (a) a halogen atom,
      (b) a hydroxy group,
      (c) a C₁₋₆ alkoxy group, and
      (d) a C₁₋₆ alkyl group;
   (2) a phenyl group (e.g., 4-fluorophenyl, 4-chlorophenyl, 4-tert-butylphenyl etc.) optionally substituted by one or more substituents selected from
      (a) a halogen atom, and
      (b) a C₁₋₆ alkoxy group;
   (3) a C₁₋₆ alkylthio group (e.g., methylthio etc.);
   (4) a 5- or 6-membered monocyclic aromatic nitrogen-containing heterocyclic group (e.g., pyridyl group etc.); and
   (5) a 8- to 14-membered fused aromatic nitrogen-containing heterocyclic group (e.g., indolyl group etc.);
n is 1; and
M is a 5-membered monocyclic aromatic heterocyclic group (preferably, triazolyl group, isoxazolyl group, oxazolyl group, oxadiazolyl group, tetrazolyl group and the like, more preferably, oxadiazolyl group) (e.g., 3-(4-propyloxyphenyl)-1,2,4-oxadiazol-5-yl, 3-[4-(trifluoromethoxy)phenyl]-1,2,4-oxadiazol-5-yl etc.) optionally substituted by a phenyl group optionally substituted by a C₁₋₆ alkoxy group optionally substituted by a halogen atom.

Specific examples of compound (I) include, for example, the compounds of Examples 1 - 114.

When compound (I) is a salt, examples of such salt include salts with inorganic bases, ammonium salt, salts with organic bases, salts with inorganic acids, salts with organic acids, salts with basic or acidic amino acids and the like.

Preferable examples of the salts with inorganic bases include alkali metal salts such as sodium salt, potassium salt and the like; alkaline earth metal salts such as calcium salt, magnesium salt, barium salt and the like; aluminum salt and the like.

Preferable examples of the salts with organic bases include salts with trimethylamine, triethylamine, pyridine, picoline, ethanolamine, diethanolamine, triethanolamine, dicyclohexylamine, N,N'-dibenzylethylenediamine and the like.

Preferable examples of the salts with inorganic acids include salts with hydrochloric acid, hydrobromic acid, nitric acid, sulfuric acid, phosphoric acid and the like.

Preferable examples of the salts with organic acids include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like.

Preferable examples of the salts with basic amino acids include salts with arginine, lysine, ornithine and the like.

Preferable examples of the salts with acidic amino acids include salts with aspartic acid, glutamic acid and the like.

Among these salts, pharmaceutically acceptable salts are preferable. Pharmaceutically acceptable preferable salts include, when a basic functional group is present in the compound, for example, salts with inorganic acids such as hydrochloric acid, hydrobromic acid, nitric acid, sulfuric acid, phosphoric acid and the like, and salts with organic acids such as acetic acid, phthalic acid, fumaric acid, oxalic acid, tartaric acid, maleic acid, citric acid, succinic acid, methanesulfonic acid, p-toluenesulfonic acid and the like. When an acidic functional group is present in the compound, inorganic salts such as alkali metal salt (e.g., sodium salt, potassium salt etc.), alkaline earth metal salt (e.g., calcium salt, magnesium salt, barium salt etc.) and the like, ammonium salt and the like can be mentioned.

Compound (I) may be a crystal, and compound (I) encompasses a single crystalline form and a crystalline form mixture.

Compound (I) encompasses solvate (e.g., hydrate etc.) and non-solvate (e.g., non-hydrate etc.).

Compound (I) may be labeled or substituted with an isotope (e.g., 2H, ³H, ¹¹C, ¹⁴C, ¹⁸F, ³⁵S, ¹²⁵I etc.) and the like. A compound labeled or substituted with an isotope can be used as, for example, a tracer (PET tracer) used in positron emission tomography (PET), and is useful in the fields of medical diagnosis and the like.

When compound (I) of the present invention has an asymmetric center, isomers such as enantiomer, diastereomer and the like may be present. Such isomers and a mixture thereof are all encompassed within the scope of the present invention. When an isomer is formed due to the conformation or tautomerism, such isomers and a mixture thereof are also encompassed in compound (I) of the present invention.

The production method of the compound of the present invention is explained in the following.

The starting materials and reagents used in each step in the following production method, and the obtained compounds each may form a salt. Examples of such salt include those similar to the aforementioned salts of the compound of the present invention and the like.

When the compound obtained in each step is a free compound, it can be converted to a desired salt by a method known per se. Conversely, when the compound obtained in each step is a salt, it can be converted to a free form or a desired other kind of salt by a method known per se.

The compound obtained in each step can also be used for the next reaction as a reaction mixture thereof or after obtaining a crude product thereof. Alternatively, the compound obtained in each step can be isolated and/or purified from the reaction mixture by a separation means such as concentration, crystallization, recrystallization, distillation, solvent extraction, fractionation, chromatography and the like according to a conventional method.

When the starting materials and reagent compounds of each step are commercially available, the commercially available products can be used as they are.

In each step, protection or deprotection reaction of a functional group is performed by the method known per se, for example, the methods described in "Protective Groups in Organic Synthesis, 4th Ed." (Theodora W. Greene, Peter G.M. Wuts) Wiley-Interscience, 2006; "Protecting Groups 3rd Ed." (P.J. Kocienski) Thieme, 2004 and the like, or the methods described in the Examples.

When compound (I) contains optical isomer, stereoisomer, regio isomer and rotamer, each can be obtained as a single product by a synthetic method or a separation method (concentration, solvent extraction, column chromatography, recrystallization and the like) known per se.

Optical isomer can be produced by a method known per se. Specifically, optical isomer is obtained by using an optically active synthesis intermediate or optically resolving the final product racemate according to a conventional method.

When compound (I) is a crystal, the crystal can be produced by crystallization according to a crystallization method known per se.

Representative production methods are described below as examples of the production method of the compound of the present invention (I); however, the production method is not limited thereto.

Compound (I) can be produced by the method shown in the following reaction scheme 1, a method analogous thereto or the like. 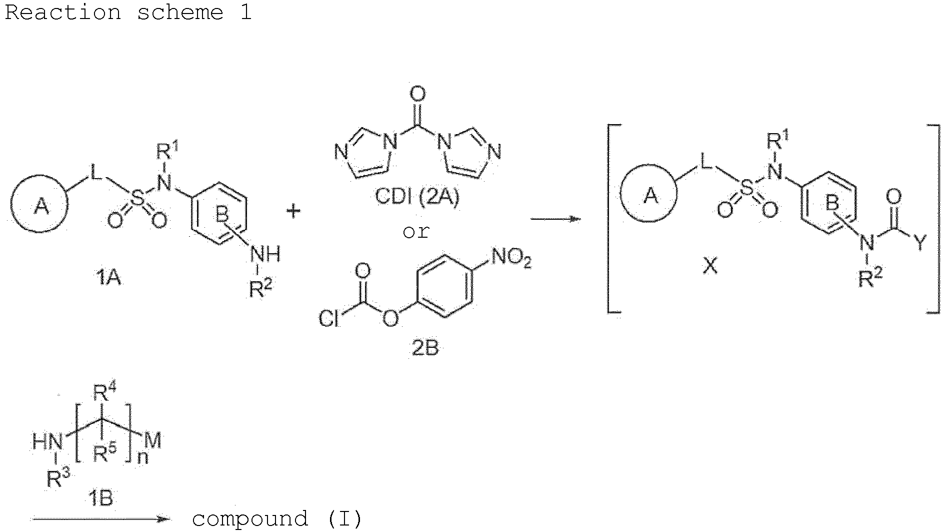 wherein Y is an imidazolin-1-yl group or a 4-nitrophenoxy group, and other symbols are each as defined above.

Compound (I) can be produced by reacting compound (1B) with intermediate (X) produced by reacting compound (1A) with compound (2A) or compound (2B) in a solvent that does not adversely influence the reaction in the presence of a base.

The amount of compound (2A) or compound (2B) to be used is generally 1 - 1.5 mol, preferably 1 - 1.2 mol, per 1 mol of compound (1A).

As the base, organic bases such as triethylamine, pyridine, N,N-diisopropylethylamine and the like can be mentioned. When compound (2B) is used, a catalytic amount of N,N-dimethylaminopyridine is preferably used in combination.

The amount of the base to be used is generally 1 - 10 mol, preferably 2 - 5 mol, per 1 mol of compound (1A).

When N,N-dimethylaminopyridine is combined, the amount thereof to be used is generally 0.01 - 0.2 mol, preferably 0.1 mol, per 1 mol of compound (1A).

As the solvent, nitrile solvents such as acetonitrile and the like; amide solvents such as N,N-dimethylformamide, N,N-dimethylacetamide and the like; halogenated hydrocarbons such as chloroform, dichloromethane and the like, and the like can be mentioned.

The amount of compound (1B) to be used is generally 1 - 4 mol, preferably 1 - 2.5 mol, per 1 mol of compound (1A).

The reaction temperature is generally 0°C - 60°C, preferably 15°C - 30°C.

The reaction time is generally 2 - 48 hr.

The starting compounds, compound (1A) and compound (1B), can be produced by the methods described in the below-mentioned reaction schemes 2 - 6 or according thereto, or by a method known per se.

### (Starting material synthesis 1: Production method 1 of compound (1A))

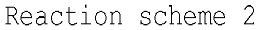

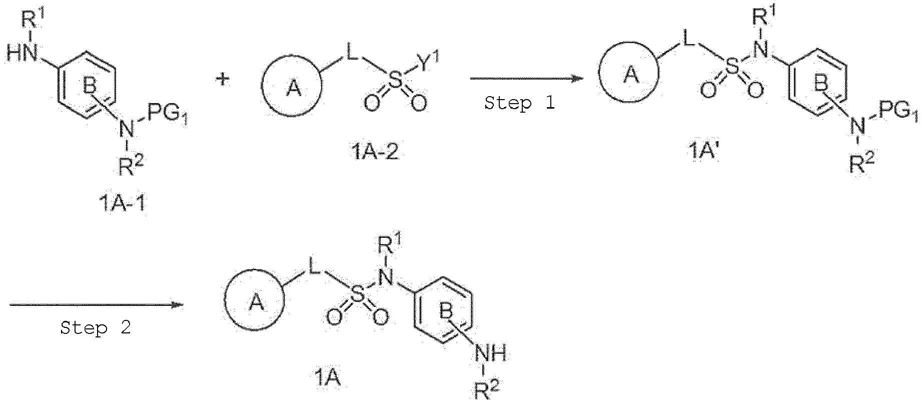

wherein PG₁ is a protecting group (e.g., tert-butoxycarbonyl group, benzyloxycarbonyl group etc.), Y¹ is a leaving group (e.g., a halogen atom etc.), and other symbols are each as defined above.

### (step 1)

In this step, compound (1A-1) is reacted with compound (1A-2) in a solvent that does not adversely influence the reaction in the presence of a base to produce compound (1A').

The amount of compound (1A-2) to be used is generally 1 - 1.2 mol, preferably 1 mol, per 1 mol of compound (1A-1).

As the base, organic bases such as triethylamine, pyridine, N,N-diisopropylethylamine and the like can be mentioned. Where necessary, a catalytic amount of N,N-dimethylaminopyridine may be used in combination.

The amount of the base to be used is generally 1 - 5 mol, preferably 1.2 - 2 mol, per 1 mol of compound (1A-1).

When N,N-dimethylaminopyridine is combined, the amount thereof to be used is generally 0.01 - 0.2 mol, preferably 0.1 mol, per 1 mol of compound (1A-1).

As the solvent, nitrile solvents such as acetonitrile and the like; amide solvents such as N,N-dimethylformamide, N,N-dimethylacetamide and the like; halogenated hydrocarbons such as chloroform, dichloromethane and the like, and the like can be mentioned.

The reaction temperature is generally 0°C - 60°C, preferably 15°C - 30°C.

The reaction time is generally 2 - 24 hr.

### (step 2)

In this step, an amino-protecting group (PG₁) is removed from compound (1A') to produce compound (1A).

Deprotection reaction can be performed by reference to the aforementioned "Protective Groups in Organic Synthesis, 4th Ed." (Theodora W. Greene, Peter G.M. Wuts) Wiley-Interscience, 2006. To be specific, for example, when PG₁ is a tert-butoxycarbonyl group, it can be removed by a method using proton acids such as hydrochloric acid and trifluoroacetic acid, a method using Lewis acids such as boron trifluoride and tin tetrachloride, or the like.

### (Starting material synthesis 2: Production method 2 of compound (1A))

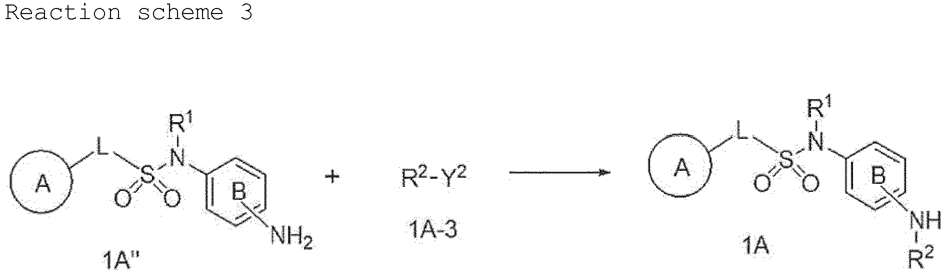

wherein Y² is a leaving group (e.g., a halogen atom (e.g., chlorine atom, bromine atom, iodine atom etc.), an alkylsulfonyloxy group (e.g., methanesulfonyloxy, trifluoromethanesulfonyloxy etc.), an arylsulfonyloxy group (e.g., p-toluenesulfonyloxy etc.) etc.), and other symbols are each as defined above.

In this step, compound (1A") is reacted with compound (1A-3) in a solvent that does not adversely influence the reaction in the presence of a base to produce compound (1A).

The amount of compound (1A-3) to be used is generally 1 - 1.2 mol, preferably 1 mol, per 1 mol of compound (1A").

As the base, organic bases such as triethylamine, pyridine, N,N-diisopropylethylamine and the like, inorganic bases such as potassium carbonate, sodium carbonate, potassium hydroxide and the like, and the like can be mentioned.

The amount of the base to be used is generally 1 - 5 mol, preferably 1 - 1.5 mol, per 1 mol of compound (1A").

As the solvent, nitrile solvents such as acetonitrile and the like; amide solvents such as N,N-dimethylformamide, N,N-dimethylacetamide and the like; halogenated hydrocarbons such as chloroform, dichloromethane and the like; acetone, and the like can be mentioned.

The reaction temperature is generally 0°C - 60°C, preferably 15°C - 30°C.

The reaction time is generally 2 - 48 hr.

### (Starting material synthesis 3: Production method of compound (1A"))

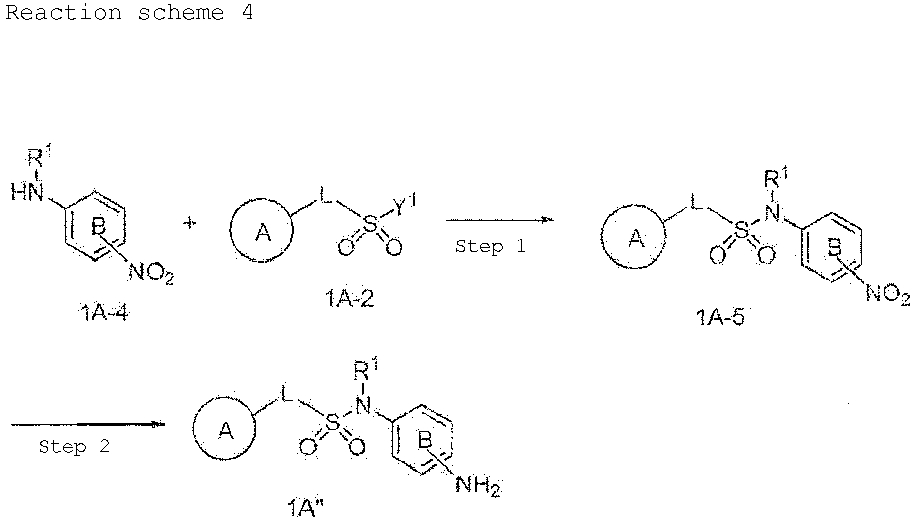

wherein each symbol is as defined above.

### (step 1)

In this step, compound (1A-4) is reacted with compound (1A-2) in a solvent that does not adversely influence the reaction in the presence of a base to produce compound (1A-5).

The amount of compound (1A-2) to be used is generally 1 - 1.2 mol, preferably 1 mol, per 1 mol of compound (1A-4).

As the base, organic bases such as triethylamine, pyridine, N,N-diisopropylethylamine and the like, inorganic bases such as sodium hydride and the like can be mentioned. Where necessary, a catalytic amount of N,N-dimethylaminopyridine may be used in combination.

The amount of the base to be used is generally 1 - 5 mol, preferably 1.2 - 2 mol, per 1 mol of compound (1A-4).

When N,N-dimethylaminopyridine is combined, the amount thereof to be used is generally 0.01 - 0.2 mol, preferably 0.1 mol, per 1 mol of compound (1A-4).

As the solvent, nitrile solvents such as acetonitrile and the like; amide solvents such as N,N-dimethylformamide, N,N-dimethylacetamide and the like; halogenated hydrocarbons such as chloroform, dichloromethane and the like, and the like can be mentioned.

The reaction temperature is generally 0°C - 60°C, preferably 15°C - 30°C.

The reaction time is generally 2 - 24 hr.

### (step 2)

In this step, a nitro group of compound (1A-5) is reduced to produce compound (1A").

Compound (1A") can be produced by reducing compound (1A-5) in a solvent that does not adversely influence the reaction in the presence of a metal catalyst at normal pressure or under pressurization (1.0 - 10 atm) under a hydrogen atmosphere.

As the metal catalyst, palladium/carbon, palladium hydroxide/carbon and the like can be mentioned.

The amount of the metal catalyst to be used is generally 0.05 - 1 mol, preferably 0.2 mol, per 1 mol of compound (1A-5).

As the solvent, alcohol solvent such as methanol, ethanol and the like; acetic acid and the like can be mentioned.

The reaction temperature is generally 0°C - 60°C, preferably 15°C - 30°C.

The reaction time is generally 2 - 48 hr.

### (Starting material synthesis 4: Production method of compound (I-1) (compound (I) wherein M in the formula (I) is an optionally substituted C₁₋₁₂ alkoxy-carbonyl group) and compound (1-2) (compound (I) wherein M in the formula (I) is a carboxy group))

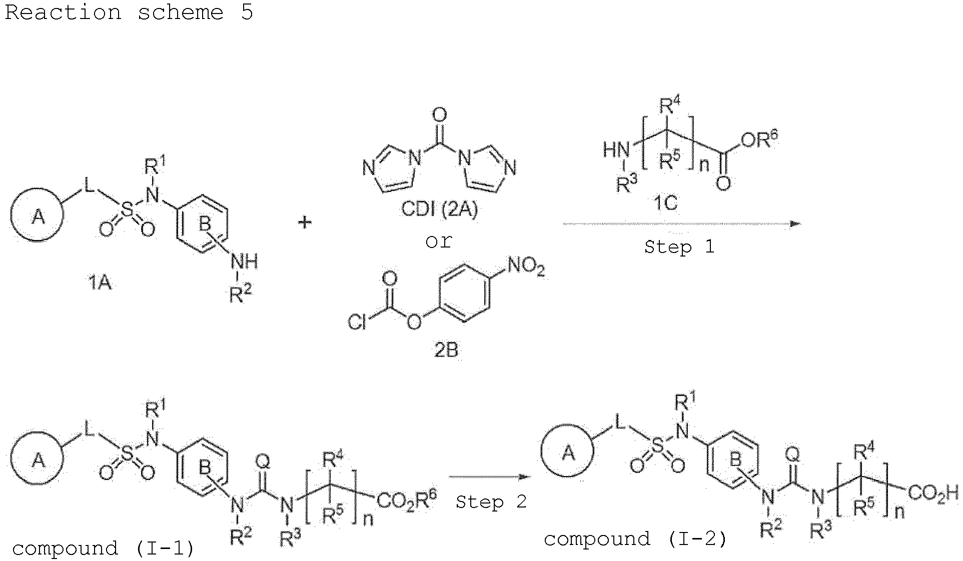

wherein R⁶ is an optionally substituted C₁₋₁₂ alkoxy group, and other symbols are each as defined above.

### (step 1)

Compound (I-1) can be produced from compound (1A) by a method similar to the production of compound (I) from compound (1A) described in reaction scheme 1 except that compound (1C) was used instead of compound (1B) in the aforementioned reaction scheme 1.

Compound (1C) may be a commercially available product or can also be produced according to a method known per se or a method analogous thereto.

### (step 2)

In this step, compound (1-2) is produced by subjecting compound (I-1) to hydrolysis reaction.

Compound (1-2) can be produced by reacting compound (I-1) in a solvent that does not adversely influence the reaction and in the presence of a base.

As the base, sodium hydroxide, potassium hydroxide, lithium hydroxide and the like can be mentioned.

The amount of the base to be used is generally 1.0 - 10 mol, preferably 1.5 mol, per 1 mol of compound (I-1).

As the solvent, alcohol solvents such as methanol, ethanol and the like; tetrahydrofuran; a mixed solvent thereof with water, and the like can be mentioned.

The reaction temperature is generally 0°C - 100°C, preferably 15°C - 80°C.

The reaction time is generally 2 - 48 hr.

### (Starting material synthesis 5: Production method of compound (I-3))

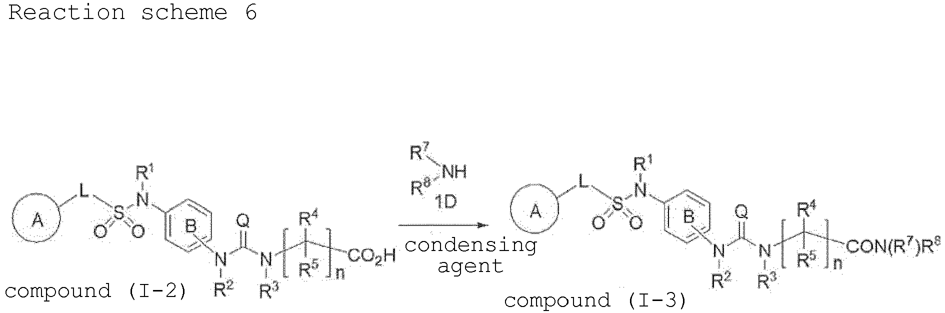

wherein R⁷ and R⁸ are the same or different and are each independently an optionally substituted C₁₋₁₂ alkyl group or an optionally substituted C₃₋₁₀ cycloalkyl group, and other symbols are each as defined above.

In this step, compound (1-2) and compound (1D) are condensed by a condensing agent in the presence of a base and a condensation additive to produce compound (1-3).

As the condensing agent, 1-ethyl-3-(3'-dimethylaminopropyl)carbodiimide (WSC), dicyclohexylcarbodiimide (DCC), diisopropylcarbodiimide (DIC), N-ethyl-N'-3-dimethylaminopropylcarbodiimide and hydrochloride thereof (EDC•HCl), hexafluorophosphoric acid (benzotriazol-1-yloxy)tripyrrolidinophosphonium (PyBop), O-(benzotriazol-1-yl)-N,N,N',N'-tetramethyluronium tetrafluoroborate (TBTU), 1-[bis(dimethylamino)methylene]-5-chloro-1H-benzotriazolium 3-oxide hexafluorophosphate (HCTU), O-benzotriazole-N,N,N',N'-tetramethyluronium hexafluoroborate (HBTU) and the like can be mentioned.

The amount of the condensing agent to be used is generally 1 - 10 mol, preferably 1 - 5 mol, per 1 mol of compound (1-2).

As the condensation additive, 1-hydroxybenzotriazole (HOBt), ethyl 1-hydroxy-1H-1,2,3-triazole-5-carboxylate (HOCt), 1-hydroxy-7-azabenzotriazole (HOAt) and the like can be mentioned, with preference given to 1-hydroxy-7-azabenzotriazole (HOAt).

The amount of the condensation additive to be used is preferably 0.05 - 1.5 mol per 1 mol of compound (1-2).

As the base, organic bases such as triethylamine, pyridine, N,N-diisopropylethylamine and the like, inorganic bases such as sodium hydride and the like can be mentioned.

The amount of the base to be used is generally 1 - 5 mol, preferably 1.5 mol, per 1 mol of compound (1-2).

Examples of the solvent include aromatic hydrocarbons such as toluene, xylene and the like; amide solvents such as N,N-dimethylformamide, N,N-dimethylacetamide and the like; ethers such as diethyl ether, tetrahydrofuran, dioxane and the like; halogenated hydrocarbons such as chloroform, dichloromethane and the like, and the like or a mixture thereof. Of these, N,N-dimethylformamide, dichloromethane and the like are preferable.

The reaction temperature is generally -10 - 30°C, preferably 0°C - 20°C, and the reaction time is generally 1 - 30 hr.

### (Starting material synthesis 6: Production method of compound wherein Q in the formula (I) is a sulfur atom)

In this step, the aforementioned starting compound, intermediate or resultant product (compound of the formula (I)) wherein Q is an oxygen atom is reacted with Lawesson's reagent (2,4-bis(4-methoxyphenyl)-1,3,2,4-dithiadiphosphetane-2,4-disulfide) in a solvent that does not adversely influence the reaction to produce a compound wherein Q is a sulfur atom.

The amount of Lawesson's reagent to be used is preferably about 1 - about 10 mol per 1 mol of the compound wherein Q is an oxygen atom.

Examples of the solvent that does not adversely influence the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethyl sulfoxide and the like; nitriles such as acetonitrile and the like, and the like. These solvents may be used in a mixture at an appropriate ratio.

The reaction temperature is generally about -80 - about 150°C, preferably about -10 - about 100°C.

The reaction time is generally about 0.5 - about 20 hr.

The compound of the present invention has low toxicity (e.g., acute toxicity, chronic toxicity, genetic toxicity, reproductive toxicity, cardiotoxicity, drug interaction, carcinogenicity etc.), and can be directly used or mixed with a pharmacologically acceptable carrier and the like to give a pharmaceutical composition and used as a prophylactic or therapeutic agent, or a diagnostic agent or the like for the below-mentioned various diseases in mammals (e.g., human, mouse, rat, rabbit, dog, cat, bovine, horse, swine, monkey etc.).

As used herein, examples of the pharmacologically acceptable carrier include various organic or inorganic carrier substances conventionally used as preparation materials, and the pharmacologically acceptable carrier can be blended as an excipient, a lubricant, a binder and a disintegrant in solid preparation; as a solvent, a solubilizing agent, a suspending agent, a isotonic agent, a buffering agent and a soothing agent in liquid preparation, and the like. Where necessary, preparation additives such as preservative, antioxidant, colorant, sweetening agent and the like can also be used.

Preferable examples of the excipient include lactose, sucrose, D-mannitol, D-sorbitol, starch, pregelatinized starch, dextrin, crystalline cellulose, low-substituted hydroxypropylcellulose, sodium carboxymethylcellulose, gum arabic, pullulan, light anhydrous silicic acid, synthetic aluminum silicate, and magnesium alumino metasilicate.

Preferable examples of the lubricant include magnesium stearate, calcium stearate, talc, colloidal silica and the like.

Preferable examples of the binder include pregelatinized starch, sucrose, gelatin, gum arabic, methylcellulose, carboxymethylcellulose, sodium carboxymethylcellulose, crystalline cellulose, sucrose, D-mannitol, trehalose, dextrin, pullulan, hydroxypropylcellulose, hydroxypropylmethylcellulose, and polyvinylpyrrolidone.

Preferable examples of the disintegrant include lactose, sucrose, starch, carboxymethylcellulose, calcium carboxymethylcellulose, croscarmellose sodium, sodium carboxymethyl starch, light anhydrous silicic acid, and low-substituted hydroxypropylcellulose.

Preferable examples of the solvent include water for injection, physiological brine, Ringer's solution, alcohol, propylene glycol, polyethylene glycol, sesame oil, corn oil, olive oil, and cottonseed oil.

Preferable examples of the solubilizing agent include polyethylene glycol, propylene glycol, D-mannitol, trehalose, benzyl benzoate, ethanol, trisaminomethane, cholesterol, triethanolamine, sodium carbonate, sodium citrate, sodium salicylate, and sodium acetate.

Preferable examples of the suspending agent include surfactants such as stearyl triethanolamine, sodium lauryl sulfate, laurylaminopropionic acid, lecithin, benzalkonium chloride, benzetonium chloride, glycerin monostearate and the like; hydrophilic polymers such as poly(vinyl alcohol), polyvinylpyrrolidone, carboxymethylcellulose sodium, methylcellulose, hydroxymethylcellulose, hydroxyethylcellulose, hydroxypropylcellulose and the like; polysorbates, and polyoxyethylene hydrogenated castor oil

Preferable examples of the isotonic agent include sodium chloride, glycerol, D-mannitol, D-sorbitol, and glucose.

Preferable examples of the buffering agent include buffer solutions of phosphate, acetate, carbonate, citrate and the like.

Preferable examples of the soothing agent include benzyl alcohol and the like.

Preferable examples of the preservative include p-oxybenzoates, chlorobutanol, benzyl alcohol, phenethyl alcohol, dehydroacetic acid, sorbic acid and the like.

Preferable examples of the antioxidant include sulfite, ascorbate and the like.

Preferable examples of the colorant include water-soluble food tar color (e.g., food colors such as Food Color Red Nos. 2 and 3, Food Color Yellow Nos. 4 and 5, Food Color Blue 1 and 2 and the like), water insoluble lake pigment (e.g., aluminum salts of the aforementioned water-soluble food tar colors etc.), and natural dye (e.g., β-carotene, chlorophyll, red iron oxide etc.) .

Preferable examples of the sweetening agent include saccharin sodium, dipotassium glycyrrhizinate, aspartame, stevia and the like.

Examples of the dosage form of the aforementioned pharmaceutical composition include oral preparations such as tablet (including sugar-coated tablet, film-coated tablet, sublingual tablet, orally disintegrating tablet), capsule (including soft capsule, microcapsule), granule, powder, troche, syrup, emulsion, suspension, films (e.g., orally disintegrable films) and the like; and parenteral agents such as injection (e.g., subcutaneous injection, intravenous injection, intramuscular injection, intraperitoneal injection, drip infusion etc.), external preparation (e.g., dermal preparation, ointment etc.), suppository (e.g., rectal suppository, vaginal suppository etc.), pellet, nasal preparation, pulmonary preparation (inhalant), eye drop and the like.

These can be each safely administered orally or parenterally (e.g., topical, rectal, intravenous administration).

These preparations may also be immediate-release preparations or controlled-release preparations such as sustained-release preparation and the like (e.g., sustained-release microcapsule).

The pharmaceutical composition can be produced according to a method conventionally used in the technical field of pharmaceutical formulation, for example, the method described in the Japanese Pharmacopoeia and the like.

The content of the compound of the present invention in the pharmaceutical composition varies depending on the dosage form, dose of the compound of the present invention and the like. It is, for example, about 0.1 - 100 wt%.

When an oral preparation is produced, coating may be applied where necessary for the purpose of masking of taste, enteric property or sustainability.

Examples of the coating base used for coating include sugar coating base, water-soluble film coating base, enteric film coating base, and sustained-release film coating base.

As the sugar coating base, sucrose is used and one or more kinds selected from talc, precipitated calcium carbonate, gelatin, gum arabic, pullulan, carnauba wax and the like may be further used in combination.

Examples of the water-soluble film coating base include cellulose polymers such as hydroxypropylcellulose, hydroxypropylmethylcellulose, hydroxyethylcellulose, methylhydroxyethylcellulose and the like; synthetic polymers such as polyvinyl acetal diethylaminoacetate, aminoalkylmethacrylate copolymer E [Eudragit E (trade name)], polyvinylpyrrolidone and the like; polysaccharides such as pullulan and the like.

Examples of the enteric film coating base include cellulose polymers such as hydroxypropylmethylcellulose phthalate, hydroxypropylmethylcellulose acetate succinate, carboxymethylethylcellulose, cellulose acetate phthalate and the like; acrylic acid polymers such as methacrylic acid copolymer L [Eudragit L (trade name)], methacrylic acid copolymer LD [Eudragit L-30D55 (trade name)], methacrylic acid copolymer S [Eudragit S (trade name)] and the like; and natural products such as shellac and the like.

Examples of the sustained-release film coating base include cellulose polymers such as ethylcellulose and the like; and acrylic acid polymers such as aminoalkylmethacrylate copolymer RS [Eudragit RS (trade name)], ethyl acrylate - methyl methacrylate copolymer suspension [Eudragit NE (trade name)] and the like.

Two or more kinds of the above-mentioned coating bases may be used by mixing them at an appropriate ratio. During coating, for example, a light shielding agent such as titanium oxide, red ferric oxide and the like may also be used.

The compound of the present invention has superior glucagon-like peptide-1 (GLP-1) receptor action enhancing activity. Thus, it is useful as, for example, an agent for the prophylaxis or treatment of diabetes (e.g., type 1 diabetes, type 2 diabetes, gestational diabetes, obese diabetes etc.); an agent for the prophylaxis or treatment of hyperlipidemia (e.g., hypertriglyceridemia, hypercholesterolemia, hypoHDLemia, postprandial hyperlipemia etc.); insulin resistance improving agent; insulin sensitizer; agent for the prophylaxis or treatment of impaired glucose tolerance (IGT); an agent for preventing progression of impaired glucose tolerance to diabetes; and an agent for the prophylaxis or treatment of obesity.

The compound of the present invention can also be used as an agent for the prophylaxis or treatment of, for example, diabetic complications [e.g., neuropathy, nephropathy, retinopathy, cataract, macroangiopathy, osteopenia, hyperosmolar diabetic coma, infections (e.g., respiratory infection, urinary tract infection, gastrointestinal infection, dermal soft tissue infections, inferior limb infection etc.), diabetic gangrene, xerostomia, hypacusis, cerebrovascular diseases, peripheral blood circulation disorder etc.], obesity, fatty liver, insulin resistant syndrome, syndrome X, metabolic syndrome, hyperinsulinemia, hyperinsulinemia-induced sensory disorder, visceral obesity syndrome and the like.

While the dose of the compound of the present invention varies depending on the subject of administration, administration route, target disease, symptom and the like, for example, it is generally about 0.005 to 300 mg/kg body weight, preferably 0.01 to 10 mg/kg body weight, further preferably 0.025 to 5 mg/kg body weight per one dose by oral administration to an adult diabetes patient. This amount is desirably administered once to 3 times per day.

The compound of the present invention can be used in combination with a medicament such as a therapeutic agent for diabetes, a therapeutic agent for diabetic complications, a therapeutic agent for hyperlipidemia, an antihypertensive agent, an antiobesity agent, a diuretic, a chemotherapeutic agent, an immunotherapeutic agent, an antithrombotic agent, a therapeutic agent for osteoporosis, an antidementia agent, an erectile dysfunction improvement agent, a therapeutic agent for incontinence•frequent urination, a therapeutic agent for dysuria and the like (hereinafter to be abbreviated as a concomitant drug). These concomitant drugs may be low-molecular-weight compounds, or high-molecular-weight protein, polypeptide, antibody, nucleic acid (including antisense nucleic acid, siRNA, shRNA), or vaccine and the like.

The administration time of the compound of the present invention and the concomitant drug is not restricted, and they may be administered to an administration subject simultaneously, or may be administered at different times.

Examples of such administration mode include (1) administration of a single preparation obtained by simultaneously processing the compound of the present invention and the concomitant drug, (2) simultaneous administration of two kinds of preparations of the compound of the present invention and the concomitant drug, which have been separately produced, by the same administration route, (3) administration of two kinds of preparations of the compound of the present invention and the concomitant drug, which have been separately produced, by the same administration route in a staggered manner, (4) simultaneous administration of two kinds of preparations of the compound of the present invention and the concomitant drug, which have been separately produced, by different administration routes, (5) administration of two kinds of preparations of the compound of the present invention and the concomitant drug, which have been separately produced, by different administration routes in a staggered manner (e.g., administration in the order of the compound of the present invention and the concomitant drug, or in the reverse order) and the like.

The dose of the concomitant drug can be appropriately determined based on the dose employed in clinical situations. The mixing ratio of the compound of the present invention and a concomitant drug can be appropriately determined depending on the administration subject, administration route, target disease, symptom, combination and the like. When the subject of administration is human, for example, a concomitant drug can be used in not less than 0.01 and less than 100 parts by weight relative to 1 part by weight of the compound of the present invention.

Examples of the therapeutic agents for diabetes include insulin preparations (e.g., animal insulin preparations extracted from the pancreas of bovine, swine; human insulin preparations synthesized by genetic engineering techniques using *Escherichia coli* or yeast; zinc insulin; protamine zinc insulin; fragments or derivatives of insulin (e.g., INS-1), oral insulin preparation), insulin resistance improving agent (e.g., pioglitazone or a salt thereof (preferably, hydrochloride), rosiglitazone or a salt thereof (preferably, maleate), tesaglitazar, ragaglitazar, muraglitazar, edaglitazone, metaglidasen, naveglitazar, AMG-131, THR-0921 etc.), α-glucosidase inhibitors (e.g., voglibose, acarbose, miglitol, emiglitate etc.), biguanide (e.g., metformin, buformin, or a salt thereof (e.g., hydrochloride, fumarate, succinate etc.) etc.), insulin secretagogues [sulfonylureas (e.g., tolbutamide, glibenclamide, gliclazide, chlorpropamide, tolazamide, acetohexamide, glyclopyramide, glimepiride, glipizide, glybuzole etc.), repaglinide, nateglinide, mitiglinide or calcium salt hydrate thereof, etc.], dipeptidylpeptidase 4 inhibitors (e.g., Alogliptin or a salt thereof (preferably, benzoate), Vildagliptin, Sitagliptin, Saxagliptin, T-6666, TS-021 etc.), β3 agonist (e.g., AJ-9677 etc.), GPR40 agonist, GLP-1 receptor agonist [e.g., GLP-1, GLP-1 MR agent, NN-2211, AC-2993 (exendin-4), BIM-51077, Aib(8,35)hGLP-1(7,37)NH₂, CJC-1131 etc.], amylin agonist (e.g., pramlintide etc.), phosphotyrosine phosphatase inhibitors (e.g., sodium vanadate etc.), gluconeogenesis inhibitor (e.g., glycogen phosphorylase inhibitor, glucose-6-phosphatase inhibitor, glucagon antagonist etc.), sodium.glucose conjugated transporter-2 (SGLT-2) inhibitor (e.g., canagliflozin, T-1095 etc.), GLP-1 analogue (e.g., liraglutide etc.), lipase inhibitor (e.g., orlistat etc.), 11β-hydroxysteroid dehydrogenase inhibitor (e.g., BVT-3498 etc.), adiponectin or an agonist thereof, IKK inhibitor (e.g., AS-2868 etc.), leptin resistance improving drugs, somatostatin receptor agonists, glucokinase activators (e.g., Ro-28-1675 etc.), GIP (Glucose-dependent insulinotropic peptide) and the like.

Examples of the therapeutic agent for diabetic complications include aldose reductase inhibitors (e.g., tolrestat, epalrestat, zenarestat, zopolrestat, minalrestat, fidarestat, CT-112, ranirestat (AS-3201)), neurotrophic factor and an increasing drug thereof (e.g., NGF, NT-3, BDNF, neurotrophin production and secretion-promoter described in WO 01/14372 (e.g., 4-(4-chlorophenyl)-2-(2-methyl-1-imidazolyl)-5-[3-(2-methylphenoxy)propyl]oxazole)), PKC inhibitor (e.g., ruboxistaurin mesylate etc.), AGE inhibitor (e.g., ALT946, pimagedine, N-phenacylthiazolium bromide (ALT766), EXO-226, pyridorin, pyridoxamine etc.), active oxygen scavenger (e.g., thioctic acid etc.), cerebral vasodilator (e.g., tiapuride, mexiletine etc.), somatostatin receptor agonists (e.g., BIM23190 etc.), apoptosis signal regulating kinase-1 (ASK-1) inhibitor and the like.

Examples of the therapeutic agent for hyperlipidemia include HMG-CoA reductase inhibitor (e.g., cerivastatin, pravastatin, simvastatin, lovastatin, atorvastatin, fluvastatin, itavastatin, rosuvastatin, pitavastatin or salts thereof (e.g., sodium salt, calcium salt) etc.), squalene synthase inhibitor (e.g., lapaquistat or a salt thereof (preferably, acetate)), fibrate compound (e.g., bezafibrate, clofibrate, simfibrate, clinofibrate etc.), ACAT inhibitor (e.g., Avasimibe, Eflucimibe etc.), anion exchange resin (e.g., colestyramine etc.), probucol, nicotinic acid drug (e.g., nicomol, niceritrol), ethyl icosapentate, phytosterol (e.g., soysterol, γ-oryzanol etc.) and the like.

Examples of the antihypertensive agent include angiotensin converting enzyme inhibitor (e.g., captopril, enalapril, delapril etc.), angiotensin II antagonist (e.g., candesartan cilexetil, losartan, eprosartan, valsartan, telmisartan, irbesartan, olmesartan medoxomil, tasosartan, 1-{[2'-(2,5-dihydro-5-oxo-4H-1,2,4-oxadiazol-3-yl)biphenyl-4-yl]methyl}-2-ethoxy-1H-benzimidazole-7-carboxylic acid etc.), calcium channel blocker (e.g., manidipine, nifedipine, nicardipine, amlodipine, efonidipine etc.), potassium channel opener (e.g., levcromakalim, L-27152, AL0671, NIP-121 etc.), clonidine and the like.

Examples of the antiobesity agent include antiobesity drugs acting on the central nervous system (e.g., dexfenfluramine, fenfluramine, phentermine, sibutramine, amfepramone, dexamphetamine, mazindol, phenylpropanolamine, clobenzorex; MCH receptor antagonists (e.g., SB-568849; SNAP-7941; compounds described in WO 01/82925 and WO 01/87834 etc.); neuropeptide Y antagonist (e.g., CP-422935 etc.); cannabinoid receptor antagonists (e.g., SR-141716, SR-147778 etc.); ghrelin antagonist; 11β-hydroxysteroid dehydrogenase inhibitor (e.g., BVT-3498 etc.) etc.), lipase inhibitor (e.g., orlistat, cetilistat (ATL-962) etc.), β3 agonist (e.g., AJ-9677 etc.), anorectic peptides (e.g., leptin, CNTF (ciliary neurotrophic factor) etc.), cholecystokinin agonist (e.g., lintitript, FPL-15849 etc.), anorexigenic agent (e.g., P-57 etc.) and the like.

Examples of the diuretic include xanthine derivatives (e.g., theobromine sodium salicylate, theobromine calcium salicylate etc.), thiazide preparations (e.g., ethiazide, cyclopenthiazide, trichloromethiazide, hydrochlorothiazide, hydroflumethiazide, benzyl hydrochlorothiazide, penflutizide, polythiazide, methyclothiazide etc.), antialdosterone preparations (e.g., spironolactone, triamterene etc.), carbonate dehydratase inhibitors (e.g., acetazolamide etc.), chlorobenzenesulfonamide preparations (e.g., chlortalidone, mefruside, indapamide etc.), azosemide, isosorbide, etacrynic acid, piretanide, bumetanide, furosemide and the like.

Examples of the chemotherapeutic agent include alkylating agent (e.g., cyclophosphamide, ifosfamide etc.), metabolic antagonist (e.g., methotrexate, 5-fluorouracil and a derivative thereof etc.), antitumor antibiotics (e.g., mitomycin, adriamycin etc.), plant-derived antitumor drug (e.g., vincristine, vindesine, taxol etc.), cisplatin, carboplatin, etoposide and the like. Of these, 5-fluorouracil derivative, furtulon or Neo-Furtulon and the like, are preferable.

Examples of the immunotherapeutic agent include components derived from microorganism or bacterium (e.g., muramyl dipeptide derivative, Picibanil etc.), polysaccharides having immunity enhancing activity (e.g., lentinan, schizophyllan, krestin etc.), cytokine obtained by genetic engineering method (e.g., interferon, interleukin (IL) etc.), colony stimulating factor (e.g., granulocyte colony stimulating factor, erythropoietin etc.) and the like, and, of these, interleukins such as IL-1, IL-2, IL-12 and the like are preferable.

Examples of the antithrombotic agent include heparin (e.g., heparin sodium, heparin calcium, dalteparin sodium etc.), warfarin (e.g., warfarin potassium), anti-thrombin drugs (e.g., argatroban etc.), thrombolytic agents (e.g., urokinase, tisokinase, alteplase, nateplase, monteplase, pamiteplase etc.), platelet aggregation inhibitors (e.g., ticlopidine hydrochloride, cilostazol, ethyl icosapentate, beraprost sodium, sarpogrelate hydrochloride etc.) and the like.

Examples of the therapeutic agent for osteoporosis include alfacalcidol, calcitriol, elcatonin, calcitonin salmon, estriol, ipriflavone, risedronate disodium, pamidronate disodium, alendronate sodium hydrate, incadronate disodium and the like.

Examples of the antidementia agent include tacrine, donepezil, rivastigmine, galanthamine and the like.

Examples of the erectile dysfunction improving agent include apomorphine, sildenafil citrate and the like.

Examples of the therapeutic agent for incontinence• frequent urination include flavoxate hydrochloride, oxybutynin hydrochloride, propiverine hydrochloride and the like.

Examples of the therapeutic agent for dysuria include acetylcholinesterase inhibitors (e.g., distigmine) and the like.

Examples of the concomitant drug include medicament confirmed to have a cachexia improving effect in animal models and clinical situations, namely, cyclooxygenase inhibitor (e.g., indomethacin etc.), progesterone derivative (e.g., megestrol acetate etc.), glucocorticoids (e.g., dexamethasone etc.), metoclopramide pharmaceuticals, tetrahydrocannabinol pharmaceuticals, fat metabolism ameliorating agent (e.g., eicosapentaenoic acid etc.), growth hormone, IGF-1, or TNF-α as a factor inducing cachexia, LIF, IL-6, antibody to oncostatin M and the like.

Further examples of the concomitant drug include nerve regeneration promoting drugs (e.g., Y-128, VX-853, prosaptide etc.), antidepressant (e.g., desipramine, amitriptyline, imipramine etc.), antiepileptic (e.g., lamotrigine etc.), antiarrhythmic drugs (e.g., mexiletine etc.), acetylcholine receptor ligand (e.g., ABT-594 etc.), endothelin receptor antagonist (e.g., ABT-627 etc.), monoamine uptake inhibitors (e.g., tramadol etc.), narcotic analgesics (e.g., morphine etc.), GABA receptor agonist (e.g., gabapentin etc.), α2 receptor agonist (e.g., clonidine etc.), topical analgesic (e.g., capsaicin etc.), antianxiety drug (e.g., benzodiazepine etc.), dopamine agonist (e.g., apomorphine etc.), midazolam, ketoconazole and the like.

Preferable concomitant drug includes dipeptidyl peptidase-4 inhibitor, insulin secretagogue, α-glucosidase inhibitor, insulin resistance improving agent, sodium.glucose conjugated transporter-2 inhibitor, GLP-1 receptor agonist, lipase inhibitor and the like.

Two or more kinds of the above-mentioned concomitant drugs may be used in combination at an appropriate ratio.

When the compound of the present invention is used in combination with a concomitant drug, the amounts thereof can be increased or decreased within the safe range in consideration of the counter effect thereof. Particularly, the doses of insulin resistance improving agent, insulin secretagogue and biguanide can be reduced from the general doses. Therefore, the counter effects that will be caused by these agents can be prevented safely. In addition, the doses of therapeutic agents for diabetic complications, therapeutic agents for hyperlipidemia, and antihypertensive agents can be reduced and, as a result, the counter effects that will be caused by these agents can be prevented effectively.

### [Examples]

The present invention is explained in more detail in the following by referring to Examples, Production Examples, Formulation Examples and Test Examples. The present invention is not limited by these Examples and the like, and may be changed within the scope of the present invention.

The apparatuses, reagents and the like used in the present Examples can be, unless otherwise specified, easily obtained or prepared according to a method generally performed in the pertinent technical field or are commercially available.

The "room temperature" in the following Examples generally shows about 10°C to about 25°C. In addition, % in the title compound shows the yield.

### Example 1: Synthesis of (2R)-2-({4-[(4-tert-butylphenyl)sulfonylamino]phenyl}carbamoylamino)-3-(4-chlorophenyl)-N-(2-ethylhexyl)propanamide(compound 1)

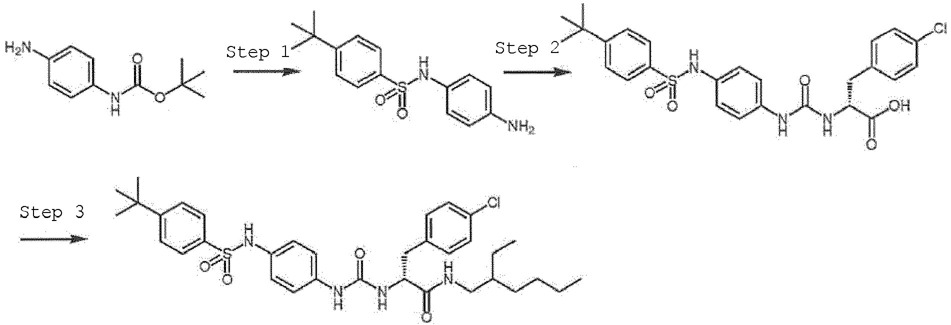

### (step 1) Synthesis of N-(4-aminophenyl)-4-tert-butyl-benzenesulfonamide

To a solution of tert-butyl N-(4-aminophenyl)carbamate (2.23 g, 10.7 mmol) in dichloromethane (100 mL) were added pyridine (1.30 mL, 16.1 mmol), dimethylaminopyridine (0.131 g, 1.07 mmol) and 4-tert-butylbenzenesulfonyl chloride (2.49 g, 10.7 mmol) and the mixture was stirred overnight. To the reaction mixture was added 0.1N hydrochloric acid (10 mL), and the mixture was extracted with dichloromethane. The solvent was evaporated and the obtained residue was purified by silica gel column chromatography (hexane/ethyl acetate) to give tert-butyl N-{4-[(4-tert-butylphenyl)sulfonylamino]phenyl}carbamate (3.38 g, 8.35 mmol, 78%). To the obtained compound were added dichloromethane (15 mL) and trifluoroacetic acid (15 mL), and the mixture was stirred overnight. The solvent in the reaction mixture was evaporated, saturated aqueous sodium hydrogen carbonate solution was added, and the mixture was extracted with dichloromethane to give the title compound (2.51 g, 8.25 mmol, 98%).

### (step 2) Synthesis of (2R)-2-({4-[(4-tert-butylphenyl)sulfonylamino]phenyl}carbamoylamino)-3-(4-chlorophenyl)propanoic acid

To a solution of N-(4-aminophenyl)-4-tert-butyl-benzenesulfonamide (0.213 g, 0.701 mmol) obtained in step 1 in dichloromethane (7.0 mL) were added triethylamine (0.489 mL, 3.51 mmol) and carbonyldiimidazole (0.136 g, 0.841 mmol) and the mixture was stirred for 1 hr. To the reaction mixture was added 4-chloro-D-phenylalanine methyl ester hydrochloride (0.386 g, 1.54 mmol) and the mixture was stirred at room temperature overnight. To the reaction mixture was added 0.1N hydrochloric acid (2.0 mL), and the mixture was extracted with dichloromethane. The solvent was evaporated, tetrahydrofuran (3.5 mL) and 1N sodium hydroxide solution (3.5 mL) were added to the obtained residue, and the mixture was stirred at room temperature for 3 hr. After extraction with ethyl acetate, the aqueous layer was adjusted to pH 2 or below with 1N hydrochloric acid, and the mixture was extracted with ethyl acetate. The organic layer was dried over sodium sulfate, the desiccant was filtered off, and the solvent was evaporated to give the title compound (0.174 g, 0.328 mmol, 47%).

### (step 3) Synthesis of (2R)-2-({4-[(4-tert-butylphenyl)sulfonylamino]phenyl}carbamoylamino)-3-(4-chlorophenyl)-N-(2-ethylhexyl)propanamide

To a solution of (2R)-2-({4-[(4-tert-butylphenyl)sulfonylamino]phenyl}carbamoylamino)-3-(4-chlorophenyl)propanoic acid (33.4 mg, 0.0630 mmol) obtained in step 2 in DMF (1.2 mL) were added 1-ethyl-3-(3'-dimethylaminopropyl)carbodiimide (WSC) hydrochloride (24.2 g, 0.126 mmol), 1-hydroxy-7-azabenzotriazol (1.72 mg, 0.0126 mmol), triethylamine (26.2 mL, 0.189 mmol) and 2-ethylhexane-1-amine (20.6 mL, 0.126 mmol) and the mixture was stirred at room temperature for 3 hr. To the reaction mixture was added 0.1N hydrochloric acid (1.2 mL), and the mixture was extracted with dichloromethane. The solvent was evaporated and the obtained residue was purified by high performance liquid chromatography (water-acetonitrile (each containing 0.1% trifluoroacetic acid)) to give the title compound (22.4 mg, 0.0349 mmol, 55%). MS (ESI) m/z 642 (M+H)⁺
¹H NMR (400 MHz, DMSO-d₆) δ 9.90 (s, 1H), 8.55 (s, 1H), 7.93 (t, J= 5.8 Hz, 1H), 7.64-7.59 (m, 2H), 7.57-7.52 (m, 2H), 7.34-7.28 (m, 2H), 7.21-7.14 (m, 4H), 6.96-6.90 (m, 2H), 6.29 (dd, J= 8.4, 2.1 Hz, 1H), 4.49-4.38 (m, 1H), 3.07-2.96 (m, 1H), 2.95-2.84 (m, 2H), 2.78 (dd, J= 13.7, 7.5 Hz, 1H), 1.34-1.07 (m, 18H), 0.87-0.74 (m, 6H).

According to the method of Example 1 and using the starting materials corresponding thereto, Examples 2 - 61 and 89 were produced. The corresponding starting materials were commercially available products, or produced according to a method known per se or the method of the following Production Example 1.

### Example 2: Synthesis of tert-butyl (2R)-3-phenyl-2-{[4-(p-tolylsulfonylamino)phenyl]carbamoylamino}propanoate (compound 62)

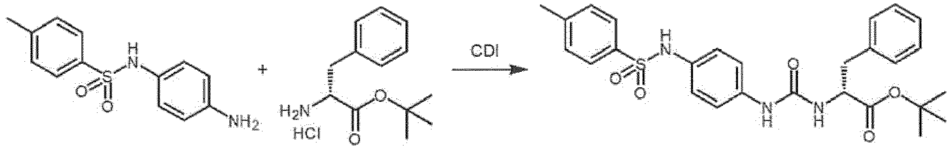

To a solution of N-(4-aminophenyl)-4-methyl-benzenesulfonamide (0.119 g, 0.454 mmol) obtained by a method similar to that in Example 1, step 1, in dichloromethane (4.5 mL) were added triethylamine (0.316 mL, 2.27 mmol) and carbonyldiimidazole (CDI) (0.0882 g, 0.544 mmol), and the mixture was stirred for 1 hr. To the reaction mixture was added D-phenylalanine t-butylester hydrochloride (0.234 g, 0.907 mmol) and the mixture was stirred at room temperature overnight. To the reaction mixture was added 0.1N hydrochloric acid (1.5 mL), and the mixture was extracted with dichloromethane. The solvent was evaporated and the obtained residue was purified by high performance liquid chromatography (water-acetonitrile (each containing 0.1% trifluoroacetic acid)) to give the title compound (0.120 g, 0.236 mmol, 52%). MS (ESI) m/z 510 (M+H)⁺
¹H NMR (400 MHz, DMSO-d6) δ 9.84 (s, 1H), 8.60 (s, 1H), 7.60-7.52 (m, 2H), 7.34-7.26 (m, 4H), 7.26-7.17 (m, 5H), 6.96-6.88 (m, 2H), 6.32 (d, J= 8.0 Hz, 1H), 4.36 (m, 1H), 3.01-2.89 (m, 2H), 2.32 (s, 3H), 1.34 (s, 9H).

According to the method of Example 2 and using the starting materials corresponding thereto, Examples 63 - 84 and 90 were produced.

### Production Example 1: Synthesis of (1R)-2-cyclohexyl-1-{3-[4-(trifluoromethoxy)phenyl]-1,2,4-oxadiazol-5-yl}ethanamine

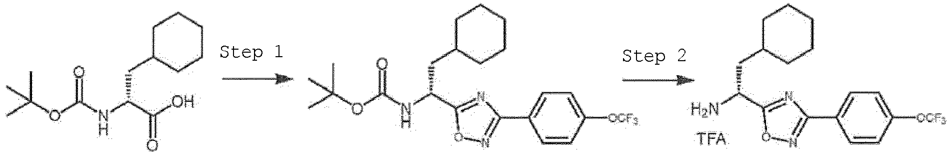

### (step 1) Synthesis of tert-butyl N-((1R)-2-cyclohexyl-1-{3-[4-(trifluoromethoxy)phenyl]-1,2,4-oxadiazol-5-yl}ethyl)carbamate

To a solution of (2R)-2-(tert-butoxycarbonylamino)-3-cyclohexyl-propanoic acid (0.134 g, 0.464 mmol) in DMF (4.6 mL) were added WSC hydrochloride (0.134 g, 0.696 mmol), 1-hydroxy-7-azabenzotriazol (12.6 mg, 0.0928 mmol), triethylamine (0.129 mL, 0.928 mmol) and N'-hydroxy-4-(trifluoromethoxy)benzamidine (0.112 g, 0.511 mmol) and the mixture was stirred at room temperature for 2 hr. Thereafter, the reaction mixture was stirred at 80°C for 4 hr. To the reaction mixture was added 0.1N hydrochloric acid (1.5 mL), and the mixture was extracted with dichloromethane. The solvent was evaporated and the obtained residue was purified by high performance liquid chromatography (water-acetonitrile (each containing 0.1% trifluoroacetic acid)) to give the title compound (0.151 g, 0.332 mmol, 72%).

### (step 2) Synthesis of (1R)-2-cyclohexyl-1-{3-[4-(trifluoromethoxy)phenyl]-1,2,4-oxadiazol-5-yl}ethanamine

To tert-butyl N-((1R)-2-cyclohexyl-1-{3-[4-(trifluoromethoxy)phenyl]-1,2,4-oxadiazol-5-yl}ethyl)carbamate (50.4 mg, 0.333 mmol) obtained in step 1 were added dichloromethane (1.6 mL) and trifluoroacetic acid (1.6 mL), and the mixture was stirred overnight. The solvent in the reaction mixture was evaporated to give trifluoroacetate (0.142 g, 0.303 mmol, 91%) of the title compound.
MS (ESI) m/z 356 (M+H)⁺

The compounds described in the following Table 1 were produced according to the method of Production Example 1 and using the starting materials and reagents (commercially available products) corresponding thereto.

**[Table 1]**

| structural formula | salt | compound name | MS (ESt) m/z (M+H)⁺ |
|---|---|---|---|
| 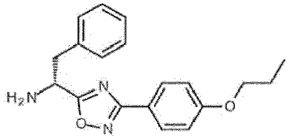 | TFA | (1R)-2-phenyl-1-[3-(4-propoxyphenyl)-1,2,4-oxadiazol-5-yl]ethanamine | 324 |
| 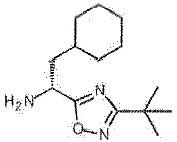 | TFA | (1R)-1-(3-tert-butyl-1,2,4-oxadiazol-5-yl)-2-cyclohexyl-ethanamine | 252 |
| 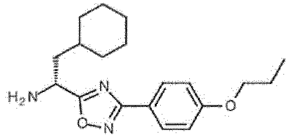 | TFA | (1R)-2-cyclohexyl-1-[3-(4-propoxyphenyl)-1,2,4-oxadiazol-5-yl]ethanamine | 330 |

### Example 3: Synthesis of tert-butyl (2R)-2-({heptyl-[4-(p-tolylsulfonylamino)phenyl]carbamoyl}amino)-3-phenyl-propanoate

### (compound 85)

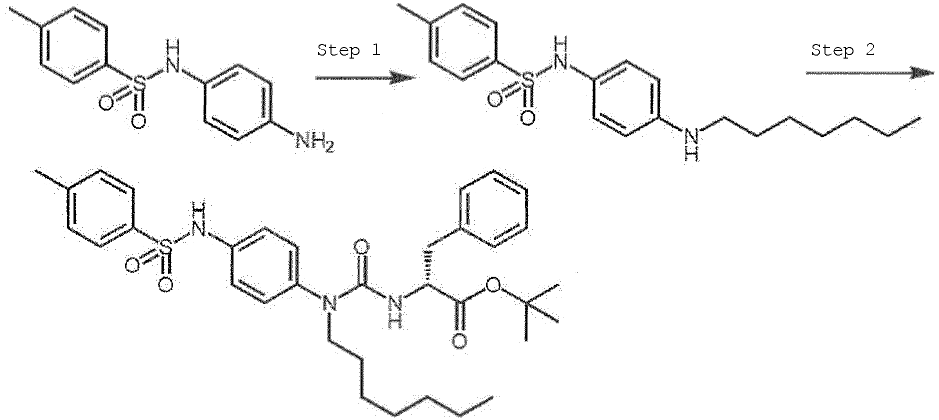

(step 1) Synthesis of N-[4-(heptylamino)phenyl]-4-methyl-benzenesulfonamide

To a solution (50 mL) of N-(4-aminophenyl)-4-methyl-benzenesulfonamide (2.05 g, 7.83 mmol) obtained in the same manner as in Example 1, step 1, in acetonitrile were added potassium carbonate (5.41 g, 39.1 mmol) and 1-bromoheptane (1.60 mL, 10.2 mmol), and the mixture was stirred at 80°C overnight. Water (50 mL) was added to the reaction solution and the mixture was extracted with ethyl acetate. The solvent was evaporated and the obtained residue was purified by silica gel column chromatography (hexane/ethyl acetate) to give the title compound (2.29 g, 6.35 mmol, 81%).

### (step 2) Synthesis of tert-butyl (2R)-2-({heptyl-[4-(p-tolylsulfonylamino)phenyl]carbamoyl}amino)-3-phenyl-propanoate

To a solution of N-[4-(heptylamino)phenyl]-4-methyl-benzenesulfonamide (44.0 mg, 0.122 mmol) obtained in step 1 in dichloromethane (2.4 mL) were added pyridine (29.7 mL, 0.366 mmol), dimethylaminopyridine (1.50 mg, 0.0122 mmol) and 4-nitrophenyl chloroformate (34.2 g, 0.159 mmol) and the mixture was stirred for 2 hr. To the reaction mixture was added D-phenylalanine t-butylester hydrochloride (62.9 mg, 0.244 mmol) and the mixture was stirred at room temperature overnight. To the reaction mixture was added 0.1N hydrochloric acid (0.8 mL), and the mixture was extracted with dichloromethane. The solvent was evaporated and the obtained residue was purified by high performance liquid chromatography (water-acetonitrile (each containing 0.1% trifluoroacetic acid)) to give the title compound (41.5 g, 0.0683 mmol, 56%).
MS (ESI) m/z 608 (M+H)⁺
¹H NMR (400 MHz, DMSO-d₆) δ 8.74 (s, 1H), 7.38-7.33 (m, 2H), 7.33-7.28 (m, 2H), 7.28-7.20 (m, 4H), 7.20-7.13 (m, 3H), 6.83-6.75 (m, 2H), 6.35 (d, J= 8.0 Hz, 1H), 4.38-4.28 (m, 1H), 2.97-2.85 (m, 2H), 2.32 (s, 3H), 1.28 (s, 9H), 1.25-1.04 (m, 12H), 0.76 (t, J= 6.9 Hz, 3H).

According to the method of Example 3 and using the starting materials corresponding thereto, compound 86 was produced.

### Example 4: Synthesis of tert-butyl (2R)-2-{ [3-fluoro-4-(p-tolylsulfonylamino)phenyl]carbamoylamino}-3-phenyl-propanoate

### (compound 87)

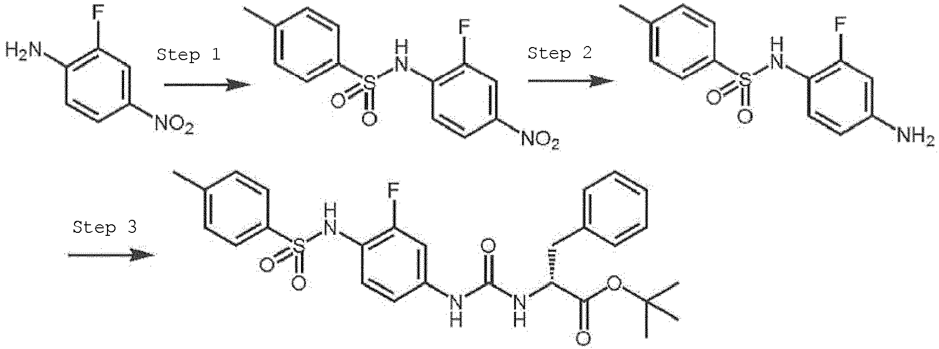

### (step 1) Synthesis of N-(2-fluoro-4-nitro-phenyl)-4-methyl-benzenesulfonamide

To a solution of 2-fluoro-4-nitro-aniline (0.167 g, 1.07 mmol) in dichloromethane (10.0 mL) were added sodium hydride (70.0 mg, 1.61 mmol) and 4-methylbenzenesulfonyl chloride (0.306 g, 1.61 mmol) and the mixture was stirred overnight. To the reaction mixture was added 0.1N hydrochloric acid (2.0 mL), and the mixture was extracted with dichloromethane. The solvent was evaporated and the obtained residue was purified by silica gel column chromatography (hexane/ethyl acetate) to give the title compound (0.206 g, 0.663 mmol, 62%).

### (step 2) Synthesis of N-(4-amino-2-fluoro-phenyl)-4-methyl-benzenesulfonamide

To a solution of N-(2-fluoro-4-nitro-phenyl)-4-methyl-benzenesulfonamide (0.206 g, 0.663 mmol) obtained in step 1 in methanol (13.2 mL) was added Pd/C (70.1 mg) and the mixture was stirred under a hydrogen atmosphere overnight. The catalyst was filtered off and the filtrate was concentrated under reduced pressure. The obtained residue was purified by silica gel column chromatography (hexane/ethyl acetate) to give the title compound (0.112 g, 0.400 mmol, 60%).

### (step 3) Synthesis of tert-butyl (2R)-2-{[3-fluoro-4-(p-tolylsulfonylamino)phenyl]carbamoylamino}-3-phenyl-propanoate

To a solution of N-(4-amino-2-fluoro-phenyl)-4-methyl-benzenesulfonamide (42.7 mg, 0.152 mmol) obtained in step 2 in dichloromethane (1.5 mL) were added triethylamine (0.106 mL, 0.762 mmol) and carbonyldiimidazole (29.6 mg, 0.183 mmol) and the mixture was stirred for 1 hr. To the reaction mixture was added D-phenylalanine t-butylester hydrochloride (86.4 mg, 0.335 mmol) and the mixture was stirred at room temperature overnight. To the reaction mixture was added 0.1N hydrochloric acid (0.5 mL), and the mixture was extracted with dichloromethane. The solvent was evaporated and the obtained residue was purified by high performance liquid chromatography (water-acetonitrile (each containing 0.1% trifluoroacetic acid)) to give the title compound (2.2 mg, 0.00417 mmol, 3%). MS (ESI) m/z 528 (M+H)⁺
¹H NMR (400 MHz, DMSO-d₆) δ 9.75 (s, 1H), 8.90 (s, 1H), 7.61-7.50 (m, 2H), 7.39-7.28 (m, 5H), 7.28-7.15 (m, 3H), 7.02 (t, J= 8.8 Hz, 1H), 6.94-6.84 (m, 1H), 6.45 (d, J= 7.9 Hz, 1H), 4.45-4.31 (m, 1H), 3.01-2.93 (m, 2H), 2.36 (s, 3H), 1.35 (s, 9H).

According to the method of Example 4 and using the starting materials corresponding thereto, compound 88 was produced.

### Example 5: Synthesis of (2R)-2-({4-[(4-tert-butylphenyl)sulfonyl-(3-methylsulfanylpropyl)amino]phenyl}carbamoylamino)-3-cyclohexyl-N-(1,5-dimethylhexyl)propanamide (compound 97)

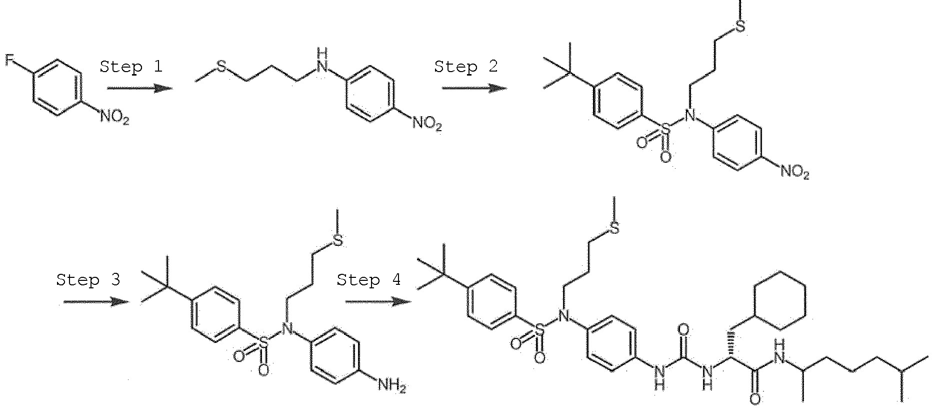

### (step 1) Synthesis of N-(3-methylsulfanylpropyl)-4-nitroaniline

To a solution of 1-fluoro-4-nitro-benzene (0.210 g, 1.49 mmol) in dimethylsulfoxide (7.5 mL) were added potassium carbonate (1.03 g, 7.46 mmol) and 3-methylsulfanylpropane-1-amine (0.327 mL, 2.98 mmol) and the mixture was stirred at 90°C for 4 hr. To the reaction mixture was added 0.1N hydrochloric acid (2.0 mL), and the mixture was extracted with ethyl acetate. The solvent was evaporated and the obtained residue was purified by silica gel column chromatography (hexane/ethyl acetate) to give the title compound (0.280 g, 1.24 mmol, 83%).

### (step 2) Synthesis of 4-tert-butyl-N-(3-methylsulfanylpropyl)-N-(4-nitrophenyl)benzenesulfonamide

To a solution of sodium hydride (20.3 mg, 0.508 mmol) in tetrahydrofuran (2.5 mL) were added dropwise a solution of N-(3-methylsulfanylpropyl)-4-nitro-aniline (57.5 mg, 0.254 mmol) obtained in step 1 in tetrahydrofuran (2.5 mL) at 0°C and the mixture was stirred for 10 min. After stirring, 4-tert-butylbenzenesulfonyl chloride (70.9 mg, 0.305 mmol) was added and the mixture was stirred for 3 hr. To the reaction mixture was added 0.1N hydrochloric acid (2.0 mL), and the mixture was extracted with ethyl acetate. The obtained residue was purified by silica gel column chromatography (hexane/ethyl acetate) to give the title compound (64.6 mg, 0.153 mmol, 30%).

### (step 3) Synthesis of N-(4-aminophenyl)-4-tert-butyl-N-(3-methylsulfanylpropyl)benzenesulfonamide

To a solution of 4-tert-butyl-N-(3-methylsulfanylpropyl)-N-(4-nitrophenyl)benzenesulfonamide (64.6 mg, 0.153 mmol) obtained in step 2 in methanol/ethyl acetate (1/1, 3.0 mL) was added Pd/C (18.1 mg) and the mixture was stirred under a hydrogen atmosphere overnight. The catalyst was filtered off and the filtrate was concentrated under reduced pressure to give the title compound (46.9 mg, 0.119 mmol, 78%).

### (step 4) Synthesis of (2R)-2-({4-[(4-tert-butylphenyl)sulfonyl-(3-methylsulfanylpropyl)amino]phenyl}carbamoylamino)-3-cyclohexyl-N-(1,5-dimethylhexyl)propanamide

According to the method of Example 1, step 3 and using N-(4-aminophenyl)-4-tert-butyl-N-(3-methylsulfanylpropyl)benzenesulfonamide (38.0 mg, 0.0968 mmol) obtained in step 3, the title compound (33.0 mg, 0.0474 mmol, 49%) was obtained.
MS (ESI) m/z 702 (M+H)⁺
¹H NMR (400 MHz, DMSO-d₆) δ 8.73 (d, J= 10.1 Hz, 1H), 7.87 (dd, J= 15.4, 8.4 Hz, 1H), 7.65-7.55 (m, 2H), 7.51-7.44 (m, 2H), 7.37-7.29 (m, 2H), 6.87 (dd, J= 8.8, 4.0 Hz, 2H), 6.31 (dd, J= 8.6, 4.4 Hz, 1H), 4.26-4.14 (m, 1H), 3.81-3.68 (m, 1H), 3.60-3.41 (m, 2H), 2.43 (t, J= 7.2 Hz, 2H), 1.95 (d, J= 0.6 Hz, 3H), 1.82-1.08 (m, 29H), 1.07-0.96 (m, 3H), 0.94-0.76 (m, 8H).

According to the method of Example 5 and using the starting materials corresponding thereto, compound 111 and compound 112 were produced.

### Example 6: Synthesis of (2R)-2-[[4-[(4-tert-butylphenyl)sulfonylamino]-2-fluoro-phenyl]carbamoylamino]-N-(2-ethylhexyl)-3-(1H-indol-3-yl)propanamide (compound 98)

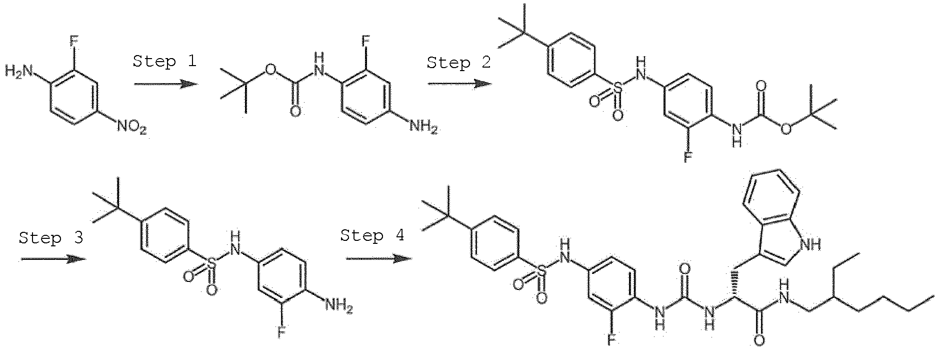

### (step 1) Synthesis of tert-butyl N-(4-amino-2-fluoro-phenyl)carbamate

To a solution of sodium hydride (0.114 g, 2.60 mmol) in tetrahydrofuran (8.0 mL) was added dropwise a solution of 2-fluoro-4-nitro-aniline (0.203 g, 1.30 mmol) in tetrahydrofuran (5.0 mL) and the mixture was stirred at 0°C for 10 min. To the reaction mixture was added di-tert-butyl dicarbonate (0.369 g, 1.69 mmol), and the mixture was stirred overnight. To the reaction mixture was added 0.1% hydrochloric acid (10 mL) and the mixture was extracted with ethyl acetate. The solvent was evaporated and to a solution of the obtained residue in methanol (13 mL) was added Pd/C (0.154 g) and the mixture was stirred under a hydrogen atmosphere overnight. The catalyst was filtered off and the filtrate was concentrated under reduced pressure to give the title compound was obtained.

### (step 2) Synthesis of tert-butyl N-{4-[(4-tert-butylphenyl)sulfonylamino]-2-fluoro-phenyl}carbamate

To a solution of tert-butyl N-(4-amino-2-fluorophenyl)carbamate obtained in step 1 in dichloromethane (13 mL) were added pyridine (0.158 mL, 1.95 mmol), dimethylaminopyridine (15.9 mg, 0.130 mmol), and 4-tert-butylbenzenesulfonyl chloride (0.303 g, 1.30 mmol) and the mixture was stirred overnight. To the reaction mixture was added 0.1% hydrochloric acid (10 mL) and the mixture was extracted with ethyl acetate. The solvent was evaporated and the obtained residue was purified by silica gel column chromatography (hexane/ethyl acetate) to remove impurities, whereby a mixture containing the title compound was obtained.

### (step 3) Synthesis of N-(4-amino-3-fluoro-phenyl)-4-tert-butyl-benzenesulfonamide

To a solution of a mixture containing tert-butyl N-{4-[(4-tert-butylphenyl)sulfonylamino]-2-fluoro-phenyl}carbamate obtained in step 2 in dichloromethane (10 mL) was added trifluoroacetic acid (3.0 mL), and the mixture was stirred for 1 hr. The reaction mixture was concentrated under reduced pressure, a saturated aqueous potassium carbonate solution (5.0 mL) was added to the residue, and the mixture was extracted with ethyl acetate. The solvent was evaporated and the obtained residue was purified by silica gel column chromatography (hexane/ethyl acetate) to give the title compound (0.115 g, 0.357 mmol, 14% (3 steps)).

### (step 4) Synthesis of (2R)-2-({4-[(4-tert-butylphenyl)sulfonylamino]-2-fluoro-phenyl}carbamoylamino)-N-(2-ethylhexyl)-3-(1H-indol-3-yl)propanamide

According to the method of Example 8, step 3 and using N-(4-amino-3-fluoro-phenyl)-4-tert-butyl-benzenesulfonamide (0.115 g, 0.357 mmol) obtained in step 3, the title compound (14.6 mg, 0.0220 mmol, 6%) was obtained.
MS (ESI) m/z 664 (M+H)⁺
¹H NMR (400 MHz, DMSO-d₆) δ 10.80 (d, J= 2.3 Hz, 1H), 10.17 (s, 1H), 8.43-8.35 (m, 1H), 7.97-7.80 (m, 2H), 7.71-7.60 (m, 2H), 7.60-7.50 (m, 3H), 7.30 (d, J= 8.0 Hz, 1H), 7.08 (d, J= 2.3 Hz, 1H), 7.07-7.01 (m, 1H), 6.94 (t, J= 7.6 Hz, 1H), 6.90 (dd, J= 12.8, 2.4 Hz, 1H), 6.82 (d, J= 8.0 Hz, 1H), 6.76 (dd, J= 9.3, 2.3 Hz, 1H), 4.54-4.41 (m, 1H), 3.15-2.85 (m, 4H), 1.41-1.05 (m, 18H), 0.88-0.70 (m, 6H).

### Example 7: Synthesis of (2R)-2-({3-[(4-tert-butylphenyl)sulfonylamino]-4-morpholino-phenyl}carbamoylamino)-3-cyclohexyl-N-(2-ethylhexyl)propanamide (compound 100)

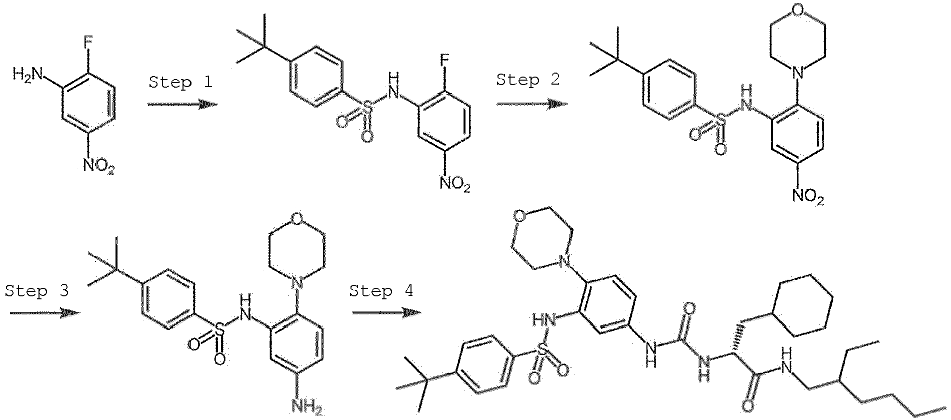

### (step 1) Synthesis of 4-tert-butyl-N-(2-fluoro-5-nitro-phenyl)benzenesulfonamide

To a solution of sodium hydride (0.116 g, 2.65 mmol) in tetrahydrofuran (15 mL) was added dropwise a solution of 2-fluoro-4-nitro-aniline (0.207 g, 1.32 mmol) in tetrahydrofuran (5.0 mL) and the mixture was stirred at 0°C for 10 min. To the reaction mixture was added 4-tert-butylbenzenesulfonyl chloride (0.399 g, 1.72 mmol), and the mixture was stirred overnight. To the reaction mixture was added 0.1% hydrochloric acid (10 mL) and the mixture was extracted with ethyl acetate. The solvent was evaporated and impurities were removed from the obtained residue by silica gel column chromatography (hexane/ethyl acetate) to give a mixture containing the title compound.

### (step 2) Synthesis of 4-tert-butyl-N-(2-morpholino-5-nitrophenyl)benzenesulfonamide

To a solution of a mixture containing 4-tert-butyl-N-(2-fluoro-5-nitro-phenyl)benzenesulfonamide obtained in step 1 in dimethyl sulfoxide (5.0 mL) were added potassium carbonate (0.523 g, 3.96 mmol) and morpholine (0.228 mL, 2.64 mmol), and the mixture was stirred at 90°C for 4 hr. Water (5.0 mL) was added to the reaction mixture, and the mixture was extracted with ethyl acetate. The solvent was evaporated and impurities were removed from the obtained residue by silica gel column chromatography (hexane/ethyl acetate) to give a mixture containing the title compound.

### (step 3) Synthesis of N-(5-amino-2-morpholino-phenyl)-4-tert-butyl-benzenesulfonamide

To a solution of a mixture containing 4-tert-butyl-N-(2-morpholino-5-nitro-phenyl)benzenesulfonamide obtained in step 2 in methanol (15 mL) was added Pd/C (31.1 mg), and the mixture was stirred under a hydrogen atmosphere for 5 hr. The catalyst was filtered off and the filtrate was concentrated under reduced pressure to give the title compound (74.2 mg, 0.190 mmol, 14% (3 steps)).

### (step 4) Synthesis of (2R)-2-({3-[(4-tert-butylphenyl)sulfonylamino]-4-morpholino-phenyl}carbamoylamino)-3-cyclohexyl-N-(2-ethylhexyl)propanamide

According to the method of Example 8, step 3 and using N-(5-amino-2-morpholino-phenyl)-4-tert-butyl-benzenesulfonamide (74.2 mg, 0.190 mmol) obtained in step 3 and (2R)-2-amino-3-cyclohexyl-N-(2-ethylhexyl)propanamide hydrochloride (0.0911 g, 0.286 mmol), the title compound (84.1 mg, 0.121 mmol, 63%) was obtained.
MS (ESI) m/z 698 (M+H)⁺

### Example 8: Synthesis of (2R)-3-cyclohexyl-N-(1,5-dimethylhexyl)-2-({4-[(6-morpholino-3-pyridyl)sulfonylamino]phenyl}carbamoylamino)propanamide (compound 104)

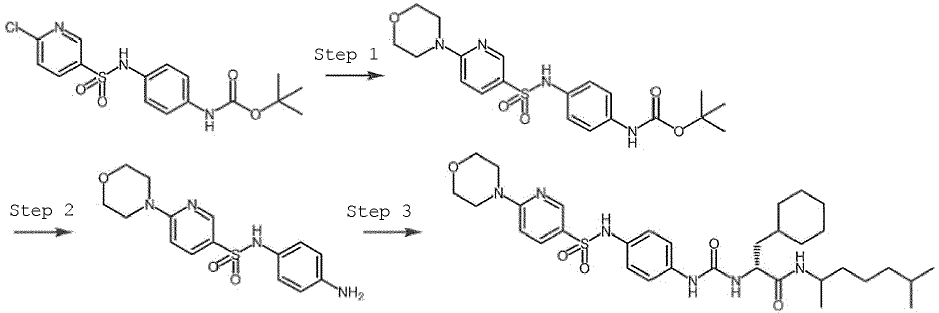

### (step 1) Synthesis of tert-butyl N-{4-[(6-morpholino-3-pyridyl)sulfonylamino]phenyl}carbamate

To a solution of tert-butyl N-[4-[(6-chloro-3-pyridyl)sulfonylamino]phenyl]carbamate (0.121 g, 0.315 mmol) obtained by a method similar to that in Example 1, step 1, in 1,4-dioxane (3.2 mL) were added morpholine (0.0543 mL, 0.630 mmol) and diisopropylethylamine (0.165 mL, 0.945 mmol), and the mixture was stirred at 90°C for 4 hr. To the reaction mixture was added 0.1N hydrochloric acid (0.5 mL), and the mixture was extracted with ethyl acetate. The solvent was evaporated and the obtained residue was purified by silica gel column chromatography (hexane/ethyl acetate) to give the title compound (0.112 g, 0.258 mmol, 82%).

### (step 2) Synthesis of N-(4-aminophenyl)-6-morpholino-pyridine-3-sulfonamide

To a solution of tert-butyl N-{4-[(6-morpholino-3-pyridyl)sulfonylamino]phenyl}carbamate (0.112 g, 0.258 mmol) obtained in step 1 in dichloromethane (2.5 mL) was added trifluoroacetic acid (2.5 mL) and the mixture was stirred for 1 hr. The reaction mixture was concentrated under reduced pressure, a saturated aqueous potassium carbonate solution (5.0 mL) was added to the residue, and the mixture was extracted with ethyl acetate. The solvent was evaporated to give the title compound (77.6 mg, 0.232 mmol).

### (step 3) Synthesis of (2R)-3-cyclohexyl-N-(1,5-dimethylhexyl)-2-({4-[(6-morpholino-3-pyridyl)sulfonylamino]phenyl}carbamoylamino)propanamide

To a solution of N-(4-aminophenyl)-6-morpholino-pyridine-3-sulfonamide (30.9 mg, 0.0887 mmol) obtained in step 2 in dichloromethane (1.8 mL) were added triethylamine (0.0742 mL, 0.532 mmol) and carbonyldiimidazole (21.6 mg, 0.133 mmol), and the mixture was stirred for 2 hr. To the reaction mixture was added (2R)-2-amino-3-cyclohexyl-N-(1,5-dimethylhexyl)propanamide hydrochloride (42.4 mg, 0.133 mmol) and the mixture was stirred at room temperature for 3 hr. To the reaction mixture was added 0.1N hydrochloric acid (0.5 mL), and the mixture was extracted with dichloromethane. The solvent was evaporated and the obtained residue was purified by high performance liquid chromatography (water-acetonitrile (each containing 0.1% trifluoroacetic acid)) to give the title compound (29.7 mg, 0461 mmol, 52%).
MS (ESI) m/z 643 (M+H)⁺
¹H NMR (400 MHz, DMSO-d₆) δ 9.75 (d, J= 1.6 Hz, 1H), 8.59 (d, J= 8.0 Hz, 1H), 8.28 (d, J= 2.5 Hz, 1H), 7.83 (dd, J= 16.7, 8.3 Hz, 1H), 7.66 (dd, J= 9.1, 2.6 Hz, 1H), 7.26-7.16 (m, 2H), 6.97-6.91 (m, 2H), 6.87 (d, J= 9.2 Hz, 1H), 6.25 (d, J= 8.5 Hz, 1H), 4.23-4.08 (m, 1H), 3.80-3.67 (m, 1H), 3.61-3.50 (m, 4H), 1.80-1.54 (m, 6H), 1.52-1.03 (m, 16H), 1.03-0.95 (m, 3H), 0.94-0.73 (m, 8H).

According to the method of Example 8 and using the starting materials corresponding thereto, compound 105, compound 109, compound 110, compound 113 and compound 114 were produced.

### Example 9: Synthesis of 2-(4-[(4-tert-butylphenyl)sulfonylamino]-N-{[(1R)-1-(cyclohexylmethyl)-2-(1,5-dimethylhexylamino)-2-oxo-ethyl]carbamoyl}anilino)acetic acid (compound 108)

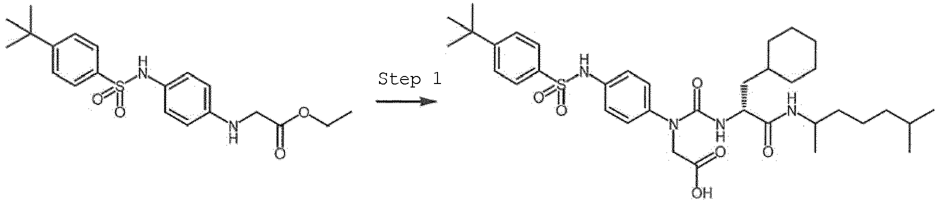

### (step 1) Synthesis of 2-[4-[(4-tert-butylphenyl)sulfonylamino]-N-[[(1R)-1-(cyclohexylmethyl)-2-(1,5-dimethylhexylamino)-2-oxo-ethyl]carbamoyl]anilino]acetic acid

To a solution of ethyl 2-{4-[(4-tert-butylphenyl)sulfonylamino]anilino} acetate (0.192 g, 0.352 mmol) obtained by the method of Example 3, step 1, in dichloromethane (3.5 mL) were added pyridine (57.2 mL, 0.705 mmol), dimethylaminopyridine (8.61 mg, 0.0705 mmol) and 4-nitrophenyl chloroformate (0.107 g, 0.529 mmol) and the mixture was stirred for 2 hr. To the reaction mixture was added (2R)-2-amino-3-cyclohexyl-N-(1,5-dimethylhexyl)propanamide hydrochloride (0.149 g, 0.529 mmol), and the mixture was stirred at room temperature for 2 hr. The solvent in the reaction mixture was evaporated, tetrahydrofuran (1.8 mL) and 1N sodium hydroxide solution (1.8 mL) were added, and the mixture was stirred at room temperature for 3 hr. The reaction solution was adjusted to pH 2 or below with 1N hydrochloric acid and extracted with ethyl acetate. The solvent was evaporated and the obtained residue was purified by high performance liquid chromatography (water-acetonitrile (each containing 0.1% trifluoroacetic acid)) to give the title compound (0.162 g, 0.241 mmol, 69%).
MS (ESI) m/z 671 (M+H)⁺

According to the method of Example 9 and using the starting materials corresponding thereto, Examples 101 was produced.

### Production Example 2: Synthesis of (2R)-2-amino-3-cyclohexyl-N-(1,5-dimethylhexyl)propanamide

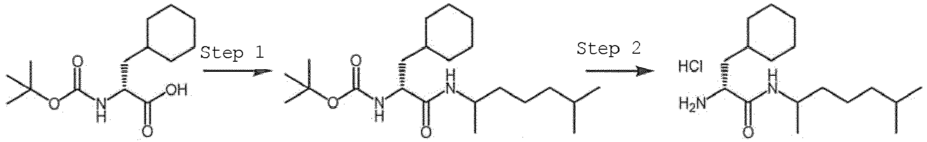

### (step 1) Synthesis of tert-butyl N-[(1R)-1-(cyclohexylmethyl)-2-(1,5-dimethylhexylamino)-2-oxo-ethyl]carbamate

To a solution of (2R)-2-(tert-butoxycarbonylamino)-3-cyclohexyl-propanoic acid (1.01 g, 3.49 mmol) in dichloromethane (35 mL) were added WSC hydrochloride (1.00 g, 5.24 mmol), 1-hydroxy-7-azabenzotriazol (9.50 mg, 0.698 mmol), triethylamine (0.973 mL, 6.98 mmol) and 6-methylheptan-2-amine (1.17 mL, 6.98 mmol), and the mixture was stirred at room temperature for 4 hr. To the reaction mixture was added 0.1N hydrochloric acid (10 mL), and the mixture was extracted with dichloromethane. The solvent was evaporated and the obtained residue was purified by silica gel column chromatography (hexane/ethyl acetate) to give the title compound (1.15 g, 3.00 mmol, 86%).

### (step 2) Synthesis of (2R)-2-amino-3-cyclohexyl-N-(1,5-dimethylhexyl)propanamide

To tert-butyl N-[(1R)-1-(cyclohexylmethyl)-2-(1,5-dimethylhexylamino)-2-oxo-ethyl]carbamate (1.15 g, 3.00 mmol) obtained in step 1 were added dichloromethane (25 mL) and trifluoroacetic acid (5.0 mL), and the mixture was stirred for 1 hr. The solvent in the reaction mixture was evaporated, 1N hydrochloric acid (5.0 mL) was added to the obtained residue and the mixture was freeze-dried to give hydrochloride (0.869 g, 2.73 mmol, 91%) of the title compound.
MS (ESI) m/z 283 (M+H)⁺

The compounds described in the following Table 2 were produced according to the method of Production Example 2 and using the starting materials and reagents (commercially available products) corresponding thereto.

**[Table 2]**

| structural formula | salt | compound name | MS (ESI) m/z (M+H)⁺ |
|---|---|---|---|
| 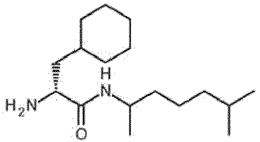 | HCl | (2R)-2-amino-3-cyclohexyl-N-(1,5-dimethylhexyl)propanamide | 283 |
| 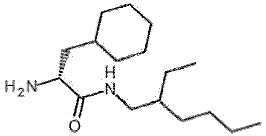 | HCl | (2R)-2-amino-3-cyclohexyl-N-(2-ethylhexyl)propanamide | 283 |
| 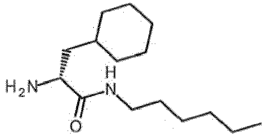 | HCl | (2R)-2-amino-3-cyclohexyl-N-hexyl-propanamide | 255 |
| 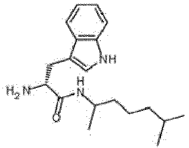 | HCl | (2R)-2-amino-N-(1,5-dimethylhexyl)-3-(1H-indol-3-yl)propanamide | 316 |
| 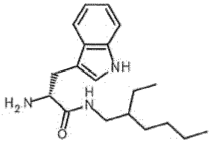 | HCl | (2R)-2-amino-N-(2-ethylhexyl)-3-(1H-indol-3-yl)propanamide | 316 |
| 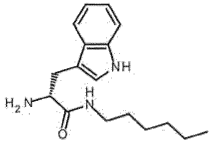 | HCl | (2R)-2-amino-N-hexyl-3-(1H-indol-3-yl)propanamide | 288 |
| 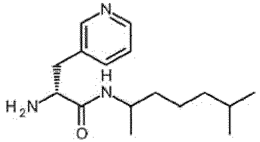 | HCl | (2R)-2-amino-N-(1,5-dimethylhexyl)-3-(3-pyridyl)propanamide | 278 |
| 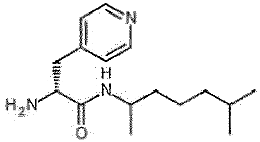 | HCl | (2R)-2-amino-N-(1,5-dimethylhexyl)-3-(4-pyridyl)propanamide | 278 |

The structural formulas and physicochemical data of the compounds of Examples 1 - 114 are shown in Table 3-1 to Table 3-23.

**[Table 3-1]**

| comp. No. | structure | MS (ESI) m/z (M+H)⁺ | NMR |
|---|---|---|---|
| 1 | 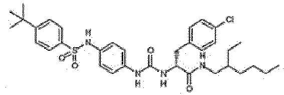 | 642 | 1H NMR (400 MHz, DMSO-d6) δ 9.90 (s, 1H), 8.55 (s, 1H), 7.93 (t, J = 5.8 Hz, 1H), 7.64 - 7.59 (m, 2H), 7.57 - 7.52 (m, 2H), 7.34 - 7.28 (m, 2H), 7.21 - 7.14 (m, 4H), 6.96 - 6.90 (m, 2H), 6.29 (dd, J = 8.4, 2.1 Hz, 1H), 4.49 - 4.38 (m, 1H), 3.07 - 2.96 (m, 1H), 2.95 - 2.84 (m, 2H), 2.78 (dd, J = 13.7, 7.5 Hz, 1H), 1.34 - 1.07 (m, 18H), 0.87 - 0.74 (m, 6H). |
| 2 | 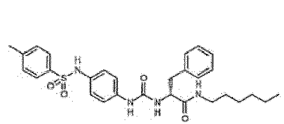 | 537 | 1H NMR (400 MHz, DMSO-d6) δ 9.81 (s, 1H), 8.56 (s, 1H), 7.99 (t, J = 5.6 Hz, 1H), 7.60 - 7.50 (m, 2H), 7.33 - 7.29 (m, 2H), 7.28 - 7.22 (m, 2H), 7.20 - 7.14 (m, 5H), 6.97 - 6.85 (m, 2H), 6.26 (d, J = 8.3 Hz, 1H), 4.48 - 4.31 (m, 1H), 3.11 - 2.85 (m, 3H), 2.79 (dd, J = 13.6, 7.6 Hz, 1H), 2.32 (s, 3H), 1.37 - 1.13 (m, 8H), 0.83 (t, J = 6.9 Hz, 2H). |
| 3 | 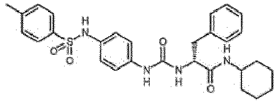 | 535 | 1H NMR (400 MHz, DMSO-d6) δ 9.81 (s, 1H), 8.59 (s, 1H), 7.88 (d, J = 7.9 Hz, 1H), 7.62 - 7.51 (m, 2H), 7.34 - 7.29 (m, 2H), 7.28 - 7.22 (m, 2H), 7.21 - 7.13 (m, 5H), 6.97 - 6.84 (m, 2H), 6.25 (d, J = 8.4 Hz, 1H), 4.49 - 4.32 (m, 1H), 3.55 - 3.42 (m, 1H), 2.88 (dd, J = 13.5, 6.0 Hz, 1H), 2.79 (dd, J = 13.5, 7.2 Hz, 1H), 2.32 (s, 3H), 1.75 - 1.47 (m, 5H), 1.32 - 0.95 (m, 5H). |
| 4 | 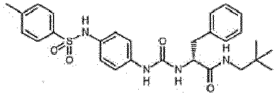 | 523 | 1H NMR (400 MHz, DMSO-d6) δ 9.60 (s, 1H), 8.54 (s, 1H), 8.03 - 7.87 (m, 1H), 7.62 - 7.50 (m, 2H), 7.33 - 7.29 (m, 2H), 7.28 - 7.23 (m, 2H), 7.22 - 7.13 (m, 5H), 6.96 - 6.84 (m, 2H), 6.28 (d, J = 8.4 Hz, 1H), 4.52 (m, 1H), 2.98 - 2.87 (m, 2H), 2.86 - 2.72 (m, 2H), 2.32 (s, 3H), 0.78 (s, 9H). |
| 5 | 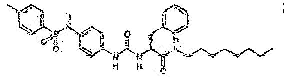 | 565 | 1H NMR (400 MHz, DMSO-d6) δ 9.81 (s, 1H), 8.56 (s, 1H), 7.99 (t, J = 5.6 Hz, 1H), 7.61 - 7.50 (m, 2H), 7.34 - 7.29 (m, 2H), 7.28 - 7.22 (m, 2H), 7.21 - 7.14 (m, 5H), 6.96 - 6.83 (m, 2H), 6.26 (d, J = 8.3 Hz, 1 H), 4.45 - 4.33 (m, 1H), 2.98 (m, 6.2 Hz, 3H), 2.79 (dd, J = 13.6, 7.6 Hz, 1H), 2.32 (s, 3H), 1.40 - 1.12 (m, 12H), 0.84 (t, J = 6.8 Hz, 3H). |

**[Table 3-2]**

| comp. No. | structure | MS (ESI) m/z (M+H)⁺ | NMR |
|---|---|---|---|
| 6 | 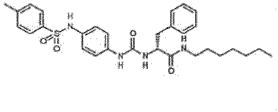 | 551 | 1H NMR (400 MHz, DMSO-d6) δ 9.81 (s, 1H), 8.56 (s, 1H), 7.99 (t, J = 5.6 Hz, 1H), 7.58 - 7.50 (m, 2H), 7.33 - 7.29 (m, 2H), 7.28 - 7.22 (m, 2H), 7.21 - 7.14 (m, 5H), 6.96 - 6.82 (m, 2H), 6.26 (d, J = 8.3 Hz, 1H), 4.38 m, 1H), 3.13 - 2.87 (m, 3H), 2.79 (dd, J = 13.6, 7.6 Hz, 1H), 2.32 (s, 3H), 1.40 - 1.11 (m, 10H), 0.84 (t, J = 6.9 Hz, 3H). |
| 7 | 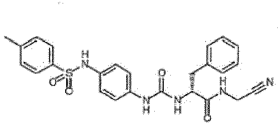 | 492 | 1H NMR (400 MHz, DMSO-d6) δ 9.82 (s, 1H), 8.80 (t, J = 5.6 Hz, 1H), 8.56 (s, 1H), 7.61 - 7.50 (m, 2H), 7.33 - 7.29 (m, 2H), 7.29 - 7.24 (m, 2H), 7.23 - 7.14 (m, 5H), 6.94 - 6.86 (m, 2H), 6.33 (d, J = 8.2 Hz, 1H), 4.48 - 4.39 (m, 1H), 4.13 (d, J = 5.6 Hz, 2H), 2.97 (dd, J = 13.7, 5.3 Hz, 1H), 2.82 (dd, J = 13.8, 8.1 Hz, 1H), 2.32 (s, 3H). |
| 8 | 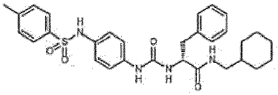 | 549 | 1H NMR (400 MHz, DMSO-d6) δ 9.81 (s, 1H), 8.56 (s, 1H), 7.99 (t, J = 5.8 Hz, 1H), 7.58 - 7.52 (m, 2H), 7.33 - 7.29 (m, 2H), 7.28 - 7.22 (m, 2H), 7.20 - 7.14 (m, 5H), 6.94 - 6.85 (m, 2H), 6.27 (d, J = 8.4 Hz, 1H), 4.48 - 4.35 (m, 1H), 3.01 - 2.86 (m, 2H), 2.84 - 2.71 (m, 2H), 2.32 (s, 3H), 1.70 - 1.48 (m, 5H), 1.35 - 1.23 (m, 1 H), 1.21 - 1.03 (m, 3H), 0.86 - 0.71 (m, 2H). |
| 9 | 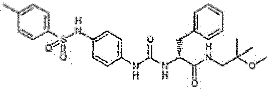 | 539 | not measured |
| 10 | 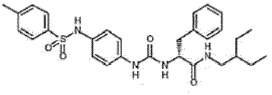 | 537 | 1H NMR (400 MHz, DMSO-d6) δ 9.81 (s, 1H), 8.56 (s, 1H), 7.92 (t, J = 5.8 Hz, 1H), 7.62 - 7.49 (m, 2H), 7.34 - 7.29 (m, 2H), 7.28 - 7.21 (m, 2H), 7.21 - 7.13 (m, 5H), 6.97 - 6.82 (m, 2H), 6.27 (d, J = 8.4 Hz, 1H), 4.44 (td, J = 7.8, 5.9 Hz, 1H), 3.07 - 2.97 (m, 1H), 2.97 - 2.86 (m, 2H), 2.79 (dd, J = 13.7, 7.6 Hz, 1H), 2.32 (s, 3H), 1.30 - 1.10 (m, 5H), 0.79 (t, J = 7.3 Hz, 6H). |

**[Table 3-3]**

| comp. No. | structure | MS (ESI) m/z (M+H)⁺ | NMR |
|---|---|---|---|
| 11 | 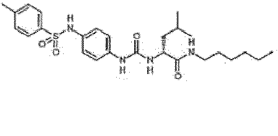 | 503 | 1H NMR (400 MHz, DMSO-d6) δ 9.81 (s, 1H), 8.48 (s, 1H), 8.02 (t, J = 5.7 Hz, 1H), 7.59 - 7.51 (m, 2H), 7.31 (dd, J = 8.1, 0.9 Hz, 2H), 7.24 - 7.16 (m, 2H), 6.95 - 6.86 (m, 2H), 6.22 (d, J = 8.5 Hz, 1H), 4.22 - 4.10 (m, 1H), 3.13 - 2.92 (m, 2H), 2.32 (s, 3H), 1.65 - 1.50 (m, 1H), 1.48 - 1.31 (m, 4H), 1.24 (q, J = 4.6 Hz, 6H), 0.91 - 0.81 (m, 9H). |
| 12 | 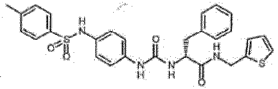 | 549 | not measured |
| 13 | 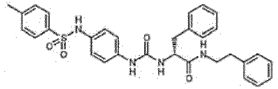 | 557 | 1H NMR (400 MHz, DMSO-d6) δ 9.81 (s, 1H), 8.56 (s, 1H), 8.14 (t, J = 5.6 Hz, 1H), 7.60 - 7.51 (m, 2H), 7.33 - 7.29 (m, 2H), 7.28 - 7.21 (m, 4H), 7.21 - 7.14 (m, 6H), 7.14 - 7.10 (m, 2H), 6.94 - 6.84 (m, 2H), 6.24 (d, J = 8.3 Hz, 1H), 4.44 - 4.34 (m, 1H), 3.28 - 3.16 (m, 2H), 2.89 (dd, J = 13.7, 5.4 Hz, 1H), 2.73 (dd, J = 13.7, 7.9 Hz, 1H), 2.66 (t, J = 7.5 Hz, 2H), 2.32 (s, 3H). |
| 14 | 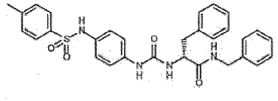 | 543 | not measured |
| 15 | 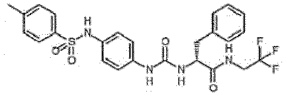 | 535 | not measured |

**[Table 3-4]**

| comp. No. | structure | MS (ESI) m/z (M+H)⁺ | NMR |
|---|---|---|---|
| 16 | 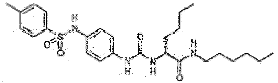 | 503 | 1H NMR (400 MHz, DMSO-d6) δ 9.81 (s, 1H), 8.55 (s, 1H), 8.00 (t, J = 5.7 Hz, 1H), 7.59 - 7.51 (m, 2H), 7.35 - 7.29 (m, 2H), 7.23 - 7.16 (m, 2H), 6.95 - 6.86 (m, 2H), 6.27 (d, J = 8.2 Hz, 1H), 4.17 - 4.07 (m, 1 H), 3.15 - 2.94 (m, 2H), 2.32 (s, 3H), 1.63 - 1.42 (m, 2H), 1.41 - 1.32 (m, 2H), 1.32 - 1.16 (m, 10H), 0.84 (m, 6H). |
| 17 | 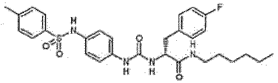 | 555 | 1H NMR (400 MHz, DMSO-d6) δ 9.81 (s, 1H), 8.55 (s, 1H), 8.00 (t, J = 5.6 Hz, 1 H), 7.61 - 7.51 (m, 2H), 7.36 - 7.29 (m, 2H), 7.23 - 7.14 (m, 4H), 7.12 - 7.02 (m, 2H), 6.94 - 6.85 (m, 2H), 6.28 (d, J = 8.3 Hz, 1H), 4.37 (q, J = 7.4 Hz, 1H), 3.11 - 2.85 (m, 3H), 2.79 (dd, J = 13.7, 7.4 Hz, 1H), 2.32 (s, 3H), 1.39 - 1.11 (m, 8H), 0.84 (t, J = 6.9 Hz, 3H). |
| 18 | 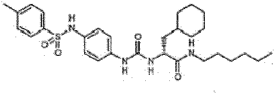 | 543 | 1H NMR (400 MHz, DMSO-d6) δ 9.81 (s, 1H), 8.49 (s, 1H), 8.07 - 7.96 (m, 1H), 7.61 - 7.51 (m, 2H), 7.37 - 7.28 (m, 2H), 7.24 - 7.16 (m, 2H), 6.98 - 6.86 (m, 2H), 6.21 (d, J = 8.4 Hz, 1H), 4.16 (td, J = 8.4, 5.8 Hz, 1H), 3.15 - 3.02 (m, 1H), 3.03 - 2.91 (m, 1H), 2.33 (s, 3H), 1.79 - 1.53 (m, 5H), 1.49 - 1.00 (m, 15H), 0.94 - 0.77 (m, 4H). |
| 19 | 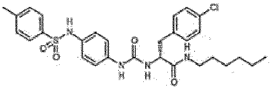 | 572 | 1H NMR (400 MHz, DMSO-d6) δ 9.75 (s, 1H), 8.49 (s, 1H), 7.94 (t, J = 5.7 Hz, 1H), 7.56 - 7.44 (m, 2H), 7.30 - 7.20 (m, 4H), 7.13 - 7.06 (m, 4H), 6.89 - 6.77 (m, 2H), 6.22 (d, J = 8.4 Hz, 1H), 4.38 - 4.25 (m, 1H), 3.04 - 2.93 (m, 1H), 2.93 - 2.78 (m, 2H), 2.73 (dd, J = 13.6, 7.4 Hz, 1H), 2.26 (s, 3H), 1.29 - 1.04 (m, 8H), 0.77 (t, J = 6.9 Hz, 3H). |
| 20 | 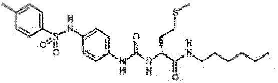 | 521 | 1H NMR (400 MHz, DMSO-d6) δ 9.82 (s, 1H), 8.56 (s, 1H), 8.04 (t, J = 5.6 Hz, 1H), 7.63 - 7.52 (m, 2H), 7.36 - 7.29 (m, 2H), 7.22 - 7.13 (m, 2H), 7.00 - 6.86 (m, 2H), 6.37 (d, J = 8.1 Hz, 1H), 4.28 - 4.13 (m, 1H), 3.15 - 2.95 (m, 2H), 2.39 (t, J = 7.9 Hz, 2H), 2.33 (s, 3H), 2.02 (s, 3H), 1.94 - 1.80 (m, 1H), 1.79 - 1.67 (m, 1H), 1.44 - 1.32 (m, 2H), 1.31 - 1.19 (m, 6H), 0.84 (t, J = 6.8 Hz, 3H), |

**[Table 3-5]**

| comp. No. | structure | MS (ESI) m/z (M+H)⁺ | NMR |
|---|---|---|---|
| 21 | 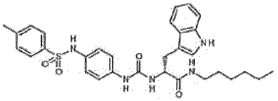 | 576 | 1H NMR (400 MHz, DMSO-d6) δ 10.80 (d, J = 2.5 Hz, 1H), 9.80 (s, 1H), 8.58 (s, 1H), 7.95 (t, J = 5.6 Hz, 1H), 7.63 - 7.49 (m, 3H), 7.38 - 7.24 (m, 3H), 7,22 - 7.12 (m, 2H), 7.10 - 6.99 (m, 2H), 6,98 - 6.86 (m, 3H), 6.25 (d, J = 8.1 Hz, 1H), 4.48 - 4.34 (m, 1H), 3.08 - 2.90 (m, 4H), 2.32 (s, 3H), 1.36 - 1.11 (m, 8H), 0.83 (t, J = 6.9 Hz, 3H). |
| 22 | 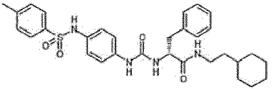 | 563 | 1H NMR (400 MHz, DMSO-d6) δ 9.74 (s, 1H), 8.49 (s, 1H), 7.89 (t, J = 5.6 Hz, 1H), 7.55 - 7.42 (m, 2H), 7.26 - 7,22 (m, 2H), 7.22 - 7.16 (m, 2H), 7.14 - 7.07 (m, 5H), 6.88 - 6.76 (m, 2H), 6.19 (d, J = 8.3 Hz, 1H), 4.37 - 4.23 (m, 1H), 3.06 - 2.88 (m, 2H), 2.84 (dd, J = 13.6, 5.9 Hz, 1H), 2.72 (dd, J = 13.6, 7,5 Hz, 1H), 2.25 (s, 3H), 1.64 - 1.46 (m, 5H), 1.21 - 0.95 (m, 6H), 0.82 - 0.68 (m, 2H). |
| 23 | 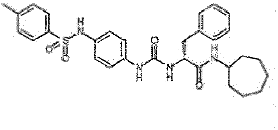 | 549 | 1H NMR (400 MHz, DMSO-d6) δ 9.74 (s, 1H), 8.51 (s, 1H), 7.85 (d, J = 7.9 Hz, 1H), 7.54 - 7.44 (m, 2H), 7.27 - 7.22 (m, 2H), 7.18 (tt, J = 7.2, 1.1 Hz, 2H), 7.14 - 7.07 (m, 5H), 6.88 - 6.77 (m, 2H), 6.18 (d, J = 8.3 Hz, 1 H), 4.40 - 4.28 (m, 1H), 3.69 - 3.56 (m, 1H), 2.80 (dd, J = 13.5, 6.1 Hz, 1H), 2.71 (dd, J = 13.5, 7.3 Hz, 1H), 2.26 (s, 3H), 1.73 - 1.61 (m, 1H), 1,58 - 1.16 (m, 11H). |
| 24 | 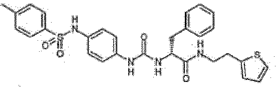 | 563 | 1H NMR (400 MHz, DMSO-d6) δ 9.81 (s, 1H), 8.56 (s, 1H), 8.22 (t, J = 5.7 Hz, 1H), 7.62 - 7.51 (m, 2H), 7.34 - 7.29 (m, 3H), 7.25 (tt, J = 7.1, 1.2 Hz, 2H), 7.21 - 7.12 (m, 5H), 6.98 - 6.83 (m, 4H), 6.26 (d, J = 8.3 Hz, 1H), 4.48 - 4.33 (m, 1 H), 3.31 - 3.19 (m, 2H), 2.98 - 2.83 (m, 3H), 2.82 - 2.71 (m, 1H), 2.32 (s, 3H). |
| 25 | 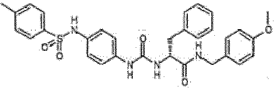 | 573 | 1H NMR (400 MHz, DMSO-d6) δ 9.81 (s, 1H), 8.56 (s, 1H), 8.50 (t, J = 5.9 Hz, 1H), 7.61 - 7.51 (m, 2H), 7.34 - 7.29 (m, 2H), 7.28 - 7.13 (m, 7H), 7.12 - 7.04 (m, 2H), 6.92 - 6.87 (m, 2H), 6.87 - 6.81 (m, 2H), 6.30 (d, J = 8.3 Hz, 1H), 4.52 - 4.41 (m, 1H), 4.26 - 4.11 (m, 2H), 3.72 (s, 3H), 2.95 (dd, J = 13.7, 5.7 Hz, 1H), 2.81 (dd, J = 13.7, 7.9 Hz, 1H), 2.32 (s, 3H). |

**[Table 3-6]**

| comp. No. | structure | MS (ESI) m/z (M+H)⁺ | NMR |
|---|---|---|---|
| 26 | 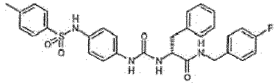 | 561 | 1H NMR (400 MHz, DWO-d6) δ 9.82 (s, 1H), 8,64 - 8.53 (m, 2H), 7.58 - 7.52 (m, 2H), 7.33 - 7.29 (m, 2H), 7.29 - 7.05 (m, 11H), 6.95 - 6.86 (m, 2H), 6.32 (d, J = 8.2 Hz, 1H), 4.51 - 4.43 (m, 1H), 4.31 - 4.16 (m, 2H), 2.96 (dd, J = 13.6, 5.9 Hz, 1H), 2.83 (dd, J = 13.6, 7.7 Hz, 1H), 2.32 (s, 3H). |
| 27 | 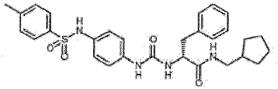 | 535 | 1H NMR (400 MHz, DMSO-d6) δ 9.81 (s, 1H), 8.56 (s, 1H), 8.03 (t, J = 5.7 Hz, 1H), 7.60 - 7.51 (m, 2H), 7.33 - 7.29 (m, 2H), 7.28 - 7.22 (m, 2H), 7.21 - 7.14 (m, 5H), 6.96 - 6.83 (m, 2H), 6.27 (d, J = 8.4 Hz, 1H), 4.49 - 4.37 (m, 1H), 3.06 - 2.96 (m, 1H), 2.95 - 2.84 (m, 2H), 2.79 (dd, J = 13.6, 7.6 Hz, 1H), 2.32 (s, 3H), 1.96 - 1.84 (m, 1H), 164 - 1.38 (m, 6H), 1.09 (t, J = 12.0 Hz, 2H). |
| 28 | 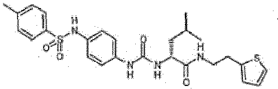 | 529 | 1H NMR (400 MHz, DMSO-d6) δ 9.82 (s, 1H), 8.49 (s, 1H), 8.20 (t, J = 5,6 Hz, 1H), 7.60 - 7.51 (m, 2H), 7.35 - 7.26 (m, 3H), 7.23 - 7.16 (m, 2H), 6.93 - 6.89 (m, 3H), 6.86 (dd, J = 3.4, 1.2 Hz, 1H), 6.23 (d, J = 8.5 Hz, 1H), 4.22 - 4.11 (m, 1H), 3.33 - 3.18 (m, 2H), 2.91 (t, J = 7.1 Hz, 2H), 2.32 (s, 3H), 1.54 (m, 1H), 1.47 -1.21 (m, 2H), 0.94 - 0.80 (m, 6H). |
| 29 | 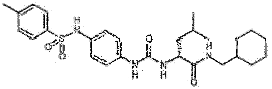 | 515 | 1H NMR (400 MHz, DMSO-d6) δ 9.81 (s, 1H), 8.49 (s, 1H), 8.01 (t, J = 5.8 Hz, 1H), 7.62 - 7.51 (m, 2H), 7.31 (dd, J = 8.2, 0.8 Hz, 2H), 7.23 - 7.16 (m, 2H), 6.96 - 6.85 (m, 2H), 6.22 (d, J = 8.6 Hz, 1H), 4.28 - 4.09 (m, 1H), 2.99 - 2.89 (m, 1H), 2.89 -2.79 (m, 1H), 2.32 (s, 3H), 1.69 - 1.52 (m, 6H), 1.45 -1.30 (m, 3H), 1.08 (d, J = 37.8 Hz, 3H), 0.87 (dd, J = 6.6, 4.6 Hz, 8H). |
| 30 | 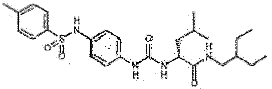 | 503 | 1H NMR (400 MHz, DMSO-d6) δ 9.81 (s, 1H), 8.49 (s, 1H), 7.96 (t, J = 5.8 Hz, 1H), 7.62 - 7.48 (m, 2H), 7.41 - 7.27 (m, 2H), 7.24 - 7.15 (m, 2H), 6.96 - 6.83 (m, 2H), 6.22 (d, J = 8.5 Hz, 1H), 4.31 - 4.10 (m, 1H), 3,10 - 3.00 (m, 1H), 2,97 - 2.87 (m, 1H), 2.33 (s, 3H), 1.65 - 1.50 (m, 1H), 1.46 - 1.15 (m, 7H), 0.89 (d, J = 4.7 Hz, 3H), 0.87 (d, J = 4.6 Hz, 3H), 0.81 (t, J = 7.3 Hz, 6H). |

**[Table 3-7]**

| comp. No. | structure | MS (ESI) m/z (M+H)⁺ | NMR |
|---|---|---|---|
| 31 | 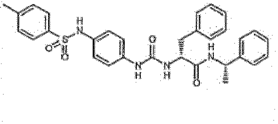 | 557 | not measured |
| 32 | 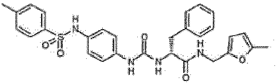 | 547 | not measured |
| 33 | 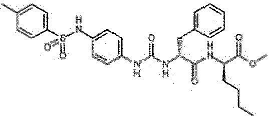 | 581 | not measured |
| 34 | 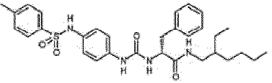 | 565 | 1H NMR (400 MHz, DMSO-d6) δ 9.80 (s, 1H), 8.55 (s, 1H), 7.92 (t, J = 5.8 Hz, 1H), 7.59 - 7.50 (m, 2H), 7.34 - 7.28 (m, 2H), 7.28 - 7.22 (m, 2H), 7.21 - 7.13 (m, 5H), 6.94 - 6.85 (m, 2H), 6.27 (d, J = 8.3 Hz, 1H), 4.50 - 4.37 (m, 1H), 3.08 - 2.96 (m, 1H), 2.96 - 2.86 (m, 2H), 2.78 (dd, J = 13.7, 7.7 Hz, 1H), 2.32 (s, 3H), 1.37 - 1.07 (m, 9H), 0.84 (td, J = 6.9, 1.8 Hz, 3H), 078 (t, J = 7.3 Hz, 3H). |
| 35 | 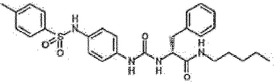 | 523 | 1H NMR (400 MHz, DMSO-d6) δ 9.81 (s, 1H), 8.56 (s, 1H), 7.99 (t, J = 5.7 Hz, 1H), 7.62 - 7.51 (m, 2H), 7.33 - 7.29 (m, 2H), 7.28 - 7.22 (m, 2H), 7.21 - 7.14 (m, 5H), 6.96 - 6.85 (m, 2H), 6.27 (d, J = 8.3 Hz, 1H), 4.53 - 4.27 (m, 1H), 3,12 - 2.87 (m, 3H), 2.79 (dd, J = 13.6, 7.5 Hz, 1H), 2.32 (s, 3H), 1.40-1.09 (m, 6H), 0.83 (t, J = 7.2 Hz, 3H). |

**[Table 3-8]**

| comp. No. | structure | MS (ESI) m/z (M+H)⁺ | NMR |
|---|---|---|---|
| 36 | 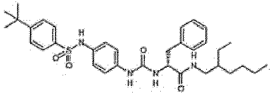 | 607 | 1H NMR (400 MHz, DMSO-d6) δ 9.89 (s, 1H), 8.55 (s, 1H), 7.92 (t, J = 5.8 Hz, 1H), 7.69 - 7.59 (m, 2H), 7.57 - 7.48 (m, 2H), 7.28 - 7.21 (m, 2H), 7.21 - 7.14 (m, 5H), 6.98 - 6.89 (m, 2H), 6.34 - 6.20 (m, 1H), 4.50 - 4.38 (m, 1H), 3.08 - 2.96 (m, 1H), 2.95 - 2.85 (m, 2H), 2.78 (dd, J = 13.7, 7.7 Hz, 1H), 1.38 - 1.07 (m, 18H), 0.83 (td, J = 6.9, 1.9 Hz, 3H), 0.78 (t, J = 7.4 Hz, 3H). |
| 37 | 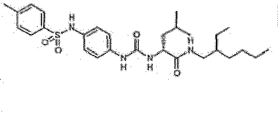 | 531 | not measured |
| 38 | 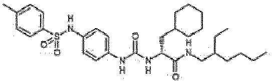 | 571 | 1H NMR (400 MHz, DMSO-d6) δ 9.81 (s, 1H), 8.49 (s, 1H), 7.94 (t, J = 5.8 Hz, 1H), 7.61 - 7.49 (m, 2H), 7.39 - 7.27 (m, 2H), 7.23 - 7.15 (m, 2H), 6.97 - 6.85 (m, 2H), 6.21 (d, J = 8.5 Hz, 1H), 4.27 - 4.15 (m, 1H), 3.14 - 3.03 (m, 1H), 2.94 - 2.81 (m, 1H), 2.32 (s, 3H), 1.80 - 1.53 (m, 5H), 1.50 - 1.02 (m, 16H), 0.94 - 0.76 (m, 7H). |
| 39 | 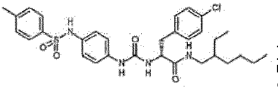 | 600 | 1H NMR (400 MHz, DMSO-d6) δ 9.81 (s, 1H), 8.54 (d, J = 1.5 Hz, 1H), 7.94 (t, J = 5.8 Hz, 1H), 7.60 - 7.51 (m, 2H), 7.36 - 7.28 (m, 4H), 7.22 - 7.12 (m, 4H), 6.94 - 6.83 (m, 2H), 6.30 (dd, J = 8.5, 2.0 Hz, 1H), 4.50 - 4.38 (m, 1H), 3.07 - 2.95 (m, 1H), 2.96 - 2.83 (m, 2H), 2.79 (dd, J = 13.7, 7.5 Hz, 1H), 2.32 (s, 3H), 1.37 - 1.06 (m, 9H), 0.84 (td, J = 6.9, 1.9 Hz, 3H), 0.78 (td, J = 7.5, 1.3 Hz, 3H). |
| 40 | 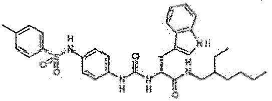 | 604 | 1H NMR (400 MHz, DMSO-d6) δ 10.80 (s, 1H), 9.80 (s, 1H), 8.57 (d, J = 1,2 Hz, 1H), 7.89 (t, J = 5.8 Hz, 1H), 7.58 - 7.50 (m, 3H), 7.34 - 7.27 (m, 3H), 7.21 - 7.14 (m, 2H), 7.09 - 7.00 (m, 2H), 6.97 - 6.31 (m, 1H), 6.91 - 6.86 (m, 2H), 6.26 (d, J = 8.1 Hz, 1H), 4.53 - 4.41 (m, 1H), 3.05 (dd, J = 14.5, 5.8 Hz, 1H), 3.00 - 2.87 (m, 3H), 2.32 (s, 3H), 1.36 - 1,05 (m, 9H), 0.82 (td, J = 6.9, 2.8 Hz, 3H), 0.79 - 0.74 (m, 3H). |

**[Table 3-9]**

| comp. No. | structure | MS (ESI) m/z (M+H)⁺ | NMR |
|---|---|---|---|
| 41 | 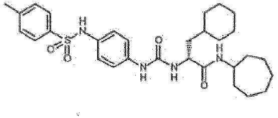 | 555 | 1H NMR (400 MHz, DMSO-d6) δ 9.81 (s, 1H), 8.50 (s, 1H), 7.96 (d, J = 7.9 Hz, 1H), 7.62 - 7.50 (m, 2H), 7.31 (dd, J = 8.3, 0.9 Hz, 2H), 7.24 - 7.14 (m, 2H), 6.95 - 6.86 (m, 2H), 6.19 (d, J = 8.5 Hz, 1H), 4.23 - 4.13 (m, 1H), 3.77 - 3.66 (m, 1H), 2.33 (s, 3H), 1.83 - 1.03 (m, 23H), 0.93 - 0.79 (m, 2H). |
| 42 | 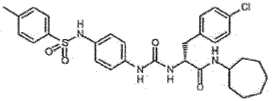 | 584 | 1H NMR (400 MHz, DMSO-d6) δ 9.81 (s, 1H), 8.57 (s, 1H), 7.95 (d, J = 7.8 Hz, 1H), 7.61 - 7.51 (m, 2H), 7.37 - 7.28 (m, 4H), 7.23 - 7.12 (m, 4H), 6.96 - 6.84 (m, 2H), 6.27 (d, J = 8.4 Hz, 1H), 4.46 - 4.33 (m, 1H), 3.75 - 3.61 (m, 1H), 2.91 - 2.72 (m, 2H), 2.32 (s, 3H), 1.82 - 1.19 (m, 12H). |
| 43 | 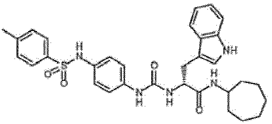 | 588 | 1H NMR (400 MHz, DMSO-d6) δ 10.79 (s, 1H), 9.80 (s, 1H), 8.59 (s, 1H), 7.86 (d, J = 7.9 Hz, 1H), 7.60 - 7.51 (m, 3H), 7.30 (ddt, J = 8.0, 4.9, 0.9 Hz, 3H), 7.21 - 7.13 (m, 2H), 7.11 - 6.98 (m, 2H), 6.97 - 6.84 (m, 3H), 6.25 (d, J = 8.1 Hz, 1H), 4.47 - 4.36 (m, 1H), 3.66 (s, 1H), 3.01 (dd, J = 14.5, 6.2 Hz, 1H), 2.93 (dd, J = 14.5, 6.8 Hz, 1H), 2.32 (s, 3H), 1.77 - 1.65 (m, 1H), 1.61 - 1.22 (m, 11H). |
| 44 | 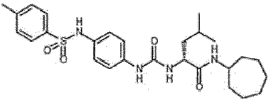 | 515 | 1H NMR (400 MHz, DMSO-d6) δ 9.81 (s, 1H), 8.50 (s, 1H), 7.98 (d, J = 7.9 Hz, 1H), 7.61 - 7.52 (m, 2H), 7.36 - 7.27 (m, 2H), 7.25 - 7.14 (m, 2H), 6.95 - 6.86 (m, 2H), 6.20 (d, J = 8.6 Hz, 1H), 4.23 - 4.13 (m, 1H), 3.76 - 3.65 (m, 1H), 2.33 (s, 3H), 1.79 - 1.30 (m, 15H), 0.88 (d, J = 3.5 Hz, 3H), 0.86 (d, J = 3.4 Hz, 3H). |
| 45 | 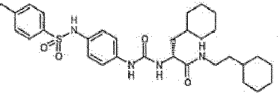 | 569 | 1H NMR (400 MHz, DMSO-d6) δ 9.81 (s, 1H), 8.48 (s, 1H), 7.98 (t, J = 5.6 Hz, 1H), 7.61 - 7.50 (m, 2H), 7.36 - 7.27 (m, 2H), 7.24 - 7.16 (m, 2H), 6.96 - 6.85 (m, 2H), 6.21 (d, J = 8.4 Hz, 1H), 4.21 - 4,10 (m, 1H), 3.21 - 3.06 (m, 1H), 3.05 - 2.92 (m, 1H), 2.32 (s, 3H), 1.78 - 1.51 (m, 10H), 1.50 - 1.02 (m, 12H), 0.96 - 0.77 (m, 4H). |

**[Table 3-10]**

| comp. No. | structure | MS (ESI) m/z (M+H)⁺ | NMR |
|---|---|---|---|
| 46 | 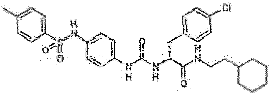 | 598 | 1H NMR (400 MHz, DMSO-d6) δ 9.81 (s, 1H), 8.55 (s, 1H), 7.98 (t, J = 5.6 Hz, 1H), 7.59 - 7.49 (m, 2H), 7.36 - 7.27 (m, 4H), 7.21 - 7.13 (m, 4H), 6.95 - 6.85 (m, 2H), 6.29 (d, J = 8.3 Hz, 1H), 4.43 - 4.32 (m, 1H), 3.15 - 2.93 (m, 2H), 2.88 (dd, J = 13.6, 6.1 Hz, 1H), 2.79 (dd, J = 13.6, 7.4 Hz, 1H), 2.32 (s, 3H), 1.70 - 11.53 (m, 5H), 1.28 - 1.08 (m, 6H), 0.89 - 0.74 (m, 2H). |
| 47 | 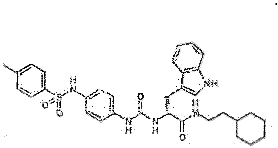 | 602 | 1H NMR (400 MHz, DMSO-d6) δ 10.81 (s, 1H), 9.80 (s, 1H), 8.58 (s, 1H), 7.91 (t, J = 5.6 Hz, 1H), 7.61 - 7.50 (m, 3H), 7.37 - 7.27 (m, 3H), 7.22 - 7.14 (m, 2H), 7.09 - 7.00 (m, 2H), 6.98 - 6.85 (m, 3H), 6.25 (d, J = 8.1 Hz, 1H), 4.47 - 4.35 (m, 1H), 3.10 - 2.99 (m, 3H), 2.94 (dd, J = 14.6, 7.0 Hz, 1H), 2.32 (s, 3H), 1.67 - 1.52 (m, 5H), 1.26 - 1.05 (m, 6H), 0.87 - 0.73 (m, 2H). |
| 48 | 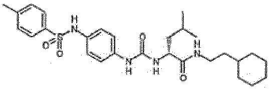 | 529 | 1H NMR (400 MHz, DMSO-d6) δ 9.81 (s, 1H), 8.48 (s, 1H), 7.99 (t, J = 5.6 Hz, 1H), 7.60 - 7.52 (m, 2H), 7.37 - 7.28 (m, 2H), 7.23 - 7.15 (m, 2H), 6.95 - 6.85 (m, 2H), 6.22 (d, J = 8.5 Hz, 1H), 4.21 - 4.10 (m, 1H), 3.14 - 2.99 (m, 2H), 2.32 (s, 3H), 1.70 - 1.49 (m, 7H), 1.46 - 1.02 (m, 8H), 0.87 (t, J = 6.6 Hz, 6H), 0.85 - 0.76 (m, 1H). |
| 49 | 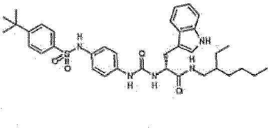 | 646 | not measured |
| 50 | 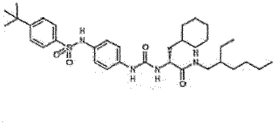 | 613 | 1H NMR (400 MHz, DMSO-d6) δ 9.89 (s, 1H), 8.49 (s, 1H), 7.94 (t, J = 5,8 Hz, 1H), 7.66 - 7.59 (m, 2H), 7.58 - 7.51 (m, 2H), 7.25 - 7.17 (m, 2H), 7.00 - 6.88 (m, 2H), 6.21 (d, J = 8.4 Hz, 1H), 4.27-4.14 (m, 1H), 3.14 - 3.01 (m, 1H), 2.93 - 2.82 (m, 1H), 1.80 -1.53 (m, 5H), 1.50 - 1.00 (m, 24H), 0.95 - 0.75 (m, 8H). |

**[Table 3-11]**

| comp. No. | structure | MS (ESI) m/z (M+H)⁺ | NMR |
|---|---|---|---|
| 51 | 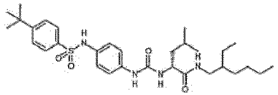 | 573 | 1H NMR (400 MHz, DMSO-d6) δ 9.89 (s, 1H), 8.49 (s, 1H), 7.95 (t, J = 5.8 Hz, 1H), 7.67 - 7.60 (m, 2H), 7.59 - 7.51 (m, 2H), 7.27 - 7.14 (m, 2H), 6.98 - 6.90 (m, 2H), 6.22 (d, J = 8.5 Hz, 1H), 4.27 - 4.13 (m, 1H), 3.10 - 2.99 (m, 1H), 2.97 - 2.85 (m, 1H), 1.64 - 1.50 (m, 1H), 1.46 - 1.29 (m, 3H), 1.29 - 1.11 (m, 17H), 0.93 - 0.74 (m, 12H). |
| 52 | 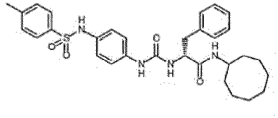 | 563 | 1H NMR (400 MHz, DMSO-d6) δ 9.74 (s, 1H), 8.51 (s, 1H), 7.84 (d, J = 7.9 Hz, 1H), 7.53 - 7.45 (m, 2H), 7.27 - 7.22 (m, 2H), 7.18(tt, J = 7.1, 1.1 Hz, 2H), 7.13-7.07 (m, 5H), 6.88 - 6.78 (m, 2H), 6.18 (d, J = 8.3 Hz, 1H), 4.40 - 4.28 (m, 1H), 3.64 (s, 1H), 2.80 (dd, J = 13.5, 6.1 Hz, 1H), 2.71 (dd, J = 13.5, 7.4 Hz, 1H), 2.25 (s, 3H), 1.62 - 1.24 (m, 14H). |
| 53 | 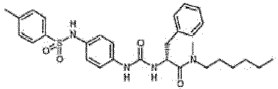 | 551 | not measured |
| 54 | 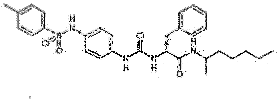 | 551 | 1 H NMR (400 MHz, DMSO-d6) δ 9.81 (s, 1H), 8.57 (d, J = 4.8 Hz, 1 H), 7.91 - 7.72 (m, 1H), 7.64 - 7.49 (m, 2H), 7.34 - 7.29 (m, 2H), 7.28 - 7.21 (m, 2H), 7.21 -7.14 (m, 5H), 6.95 - 6.83 (m, 2H), 6.33 - 6.22 (m, 1H), 4,45 - 4.35 (m, 1H), 3.75 - 3.62 (m, 1 H), 2.97 - 2.85 (m, 1H), 2.84 - 2.73 (m, 1H), 2.32 (s, 3H), 1.40 - 1.05 (m, 8H), 1.02 - 0.96 (m, 1.5H), 0.94 - 0.88 (m, 1.5H), 0.88 - 0.76 (m, 3H). |
| 55 | 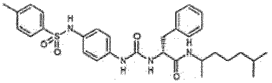 | 565 | 1H NMR (400 MHz₁ DMSO-d6) δ 9.80 (d, J = 1.6 Hz, 1H), 8.56 (d, J = 2.3 Hz, 1H), 7.85 (d, J = 8.2 Hz, 0.5H), 7.79 (d, J = 8.3 Hz, 0.5H), 7.60 - 7.51 (m, 2H), 7.33 - 7.29 (m, 2H), 7.28 - 7.21 (m, 2H), 7.20 - 7.14 (m, 5H), 6.93 - 6.84 (m, 2H), 6.26 (dd, J = 8.3, 3.2 Hz, 1H), 4.45 - 4.34 (m, 1H), 3.75 - 3.62 (m, 1H), 2.96 - 2.84 (m, 1H), 2.84 - 2.74 (m, 1 H), 2.32 (s, 3H), 1.52 - 1.40 (m, 1H), 1.34 - 1.02 (m, 6H), 1.00 (d, J = 6.6 Hz, 1.5H), 0.90 (d, J = 6.6 Hz, 1.5H), 0.86 - 0.76 (m, 6H). |

**[Table 3-12]**

| comp. No. | structure | MS (ESI) m/z (M+H)⁺ | NMR |
|---|---|---|---|
| 56 | 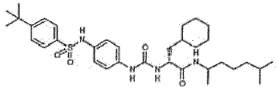 | 613 | 1H NMR (400 MHz, DMSO-d6) δ 9.82 (d, J = 3.2 Hz, 1H), 8.43 (d, J = 9.1 Hz, 1H), 7.76 (dd, J = 16.1, 8.3 Hz, 1H), 7.60 - 7.52 (m, 2H), 7.51 - 7.44 (m, 2H), 7.19 - 7.09 (m, 2H), 6.93 - 6.80 (m, 2H), 6.13 (dd, J = 8.5, 4.5 Hz, 1H), 4.16 - 4,04 (m, 1H), 3.73 - 3.59 (m, 111), 1.73 - 1,46 (m, 6H), 1.46 - 0.96 (m, 20H), 0.93 (t, J = 6.8 Hz, 3H), 0.86 - 0.74 (m, 6H), 0.72 (dd, J = 6.6, 1.8 Hz, 3H). |
| 57 | 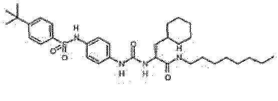 | 613 | 1H NMR (400 MHz, DMSO-d6) δ 9.90 (s, 1H), 8.49 (s, 1H), 8.00 (t, J = 5.6 Hz, 1H), 7.67 - 7.61 (m, 2H), 7.59 - 7.51 (m, 2H), 7.26 - 7.16 (m, 2H), 7.00 - 6.90 (m, 2H), 6.22 (d, J = 8.4 Hz, 1H), 4.25 - 4.10 (m, 1H), 3.16 - 3.02 (m, 1H), 3.03 - 2.90 (m, 1H), 1.82 - 1.53 (m, 5H), 1.49 - 1.00 (m, 27H), 0.95 - 0.79 (m, 5H). |
| 58 | 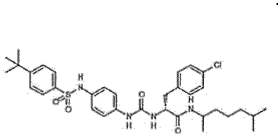 | 642 | 1H NMR (400 MHz, DMSO-d6) δ 9.83 (d, J = 2.9 Hz, 1H), 8.49 (d, J = 4.7 Hz, 1H), 7.78 (dd, J = 20.3, 8.2 Hz, 1H), 7.60 - 7.51 (m, 2H), 7.51 - 7.45 (m, 2H), 7.28 - 7.20 (m, 2H), 7.16 - 7.07 (m, 4H), 6.91 - 6.83 (m, 2H), 6.22 (dd, J = 8.4, 4.6 Hz, 1H), 4.38 - 4.26 (m, 1H), 3.69 - 3.54 (m, 1H), 2.87 - 2.76 (m, 1H), 2.76 - 2.66 (m, 1H), 1.46 - 1.33 (m, 1H), 1.29 - 0.95 (m, 15H), 0.92 (d, J = 6.6 Hz, 1.5H), 0.85 (d, J = 6.6 Hz, 1.5H), 0.78 - 0.70 (m, 6H). |
| 59 | 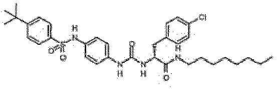 | 642 | not measured |
| 60 | 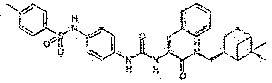 | 589 | 1H NMR (400 MHz, DMSO-d6) δ 9.81 (s, 1H), 8.56 (s, 1H), 8.01 (q, J = 5.5 Hz, 1H), 7.60 - 7.50 (m, 2H), 7.34 - 7.28 (m, 2H), 7.28 - 7.21 (m, 2H), 7.21 - 7.13 (m, 5H), 6.95 - 6.85 (m, 2H), 6.26 (d, J = 8.3 Hz, 1H), 4.48 - 4.32 (m, 1H), 3.13 - 3.01 (m, 1H), 3.02 - 2.85 (m, 2H), 2.85 - 2.75 (m, 1H), 2.32 (s, 3H), 2.31 - 2.21 (m, 1H), 2.10 -1.96 (m, 1H), 1.90 - 1.66 (m, 5H), 1.42 - 1.21 (m, 1H), 1.12 (d, J = 9.8 Hz, 3H), 0.98 (d, J = 4.7 Hz, 3H), 0.84 - 0.74 (m, 1H). |

**[Table 3-13]**

| comp. No. | structure | MS (ESI) m/z (M+H)⁺ | NMR |
|---|---|---|---|
| 61 | 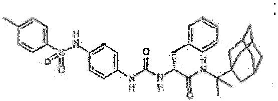 | 629 | 1H NMR (400 MHz, DMSO-d6) δ 9.81 (s, 1H), 8.59 (s, 1H), 7.58 - 7.52 (m, 2H), 7.31 (d, J = 8.1 Hz, 2H), 7.28 - 7.15 (m, 7H), 6.95 (s, 1H), 6.92 - 6.86 (m, 2H), 6.27 (d, J = 8.2 Hz, 1H), 4.57 - 4.43 (m, 1H), 2.89 (dd, J = 13.7, 6.2 Hz, 1H), 2.81 (dd, J = 13.6, 7.5 Hz, 1H), 2.32 (s, 3H), 1.87 (s, 3H), 1.63 - 1.43 (m, 12H), 1.18 (s, 3H), 1.11 (s, 3H). |
| 62 | 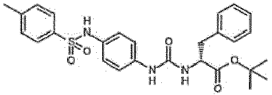 | 510 | 1H NMR (400 MHz, DMSO-d6) δ 9.84 (s, 1H), 8.60 (s, 1H), 7.60 - 7.52 (m, 2H), 7.34 - 7.26 (m, 4H), 7.26 - 7.17 (m, 5H), 6.96 - 6.88 (m, 2H), 6.32 (d, J = 8.0 Hz, 1H), 4.36 (m, 1H), 3.01 - 2.89 (m, 2H), 2.32 (s, 3H), 1.34 (s, 9H). |
| 63 | 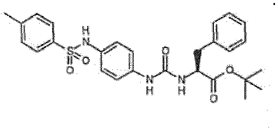 | 510 | 1H NMR (400 MHz, DMSO-d6) δ 9.84 (s, 1H), 8.60 (s, 1H), 7.60 - 7.52 (m, 2H), 7.34- 7.26 (m, 4H), 7.26 - 7.17 (m, 5H), 6.96 - 6.88 (m, 2H), 6.32 (d, J = 8.0 Hz, 1H), 4.36 (m, 1H), 3.01 - 2.89 (m, 2H), 2.32 (s, 3H), 1.34 (s, 9H). |
| 64 | 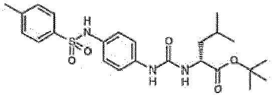 | 476 | 1H NMR (400 MHz, DMSO-d6) δ 9.83 (s, 1H), 8.46 (s, 1H), 7.66 - 7.52 (m, 2H), 7.38 - 7.28 (m, 2H). 7.26 - 7.14 (m, 2H), 6.99 - 6.87 (m, 2H), 6.33 (d, J = 8.3 Hz, 1H), 4.19 - 4.00 (m, 1H), 2,33 (s, 3H), 1.71 - 1.58 (m, 1H), 1.52 - 1.42 (m, 2H), 1.40 (s, 9H), 0.90 (d, J = 6.6 Hz, 3H), 0.87 (d, J = 6.5 Hz, 3H). |
| 65 | 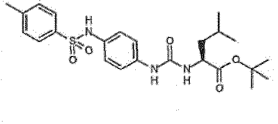 | 476 | 1H NMR (400 MHz, DMSO-d) δ 9.83 (s, 1H), 8.46 (s, 1H), 7.66 - 7.52 (m, 2H), 7.38 - 7.28 (m, 2H), 7.26 - 7.14 (m, 2H), 6.99 - 6.67 (m, 2H), 6.33 (d, J = 6.3 Hz, 1H), 4.19 - 4.00 (m, 1H), 2.33 (s, 3H), 1.71 -1.58 (m, 1H), 1.52 - 1,42 (m, 2H), 1.40 (s, 9H), 0.90 (d, J = 6.6 Hz, 3H), 0.87 (d, J = 6.5 Hz, 3H). |

**[Table 3-14]**

| comp. No. | structure | MS (ESI) m/z (M+H)⁺ | NMR |
|---|---|---|---|
| 66 | 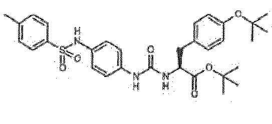 | 582 | 1H NMR (400 MHz, DMSO-d6) δ 9.84 (s, 1H), 8.59 (s, 1H), 7.63 - 7.52 (m, 2H), 7.37 - 7.28 (m, 2H), 7.25 - 7.16 (m, 2H), 7.16 - 7.06 (m, 2H), 6.97 - 6.82 (m, 4H), 6.35 (d, J = 7.9 Hz, 1H), 4.33 (dt, J = 7.9, 6.7 Hz, 1H), 2.97 - 2.79 (m, 2H), 2.33 (s, 3H), 1.30 (s, 9H), 1.25 (s, 9H). |
| 67 | 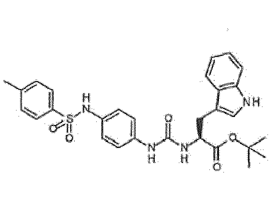 | 549 | 1H NMR (400 MHz, DMSO-d6) δ 10.88 (d, J = 2.4 Hz, 1H), 9.83 (s, 1H), 8.60 (s, 1H), 7.59 - 7.53 (m, 2H), 7.53 - 7.48 (m, 1H), 7,36 - 7.28 (m, 3H), 7.22 - 7.16 (m, 2H), 7.12 (d, J = 2.4 Hz, 1H), 7.09 - 7.03 (m, 1H), 7.00 - 6.94 (m, 1H), 6.93 - 6.88 (m, 2H), 6.31 (d, J = 7.9 Hz, 1H), 4.50 - 4.34 (m, 1H), 3.16 - 2.98 (m, 2H), 2.32 (s, 3H), 1.30 (s, 9H). |
| 68 | 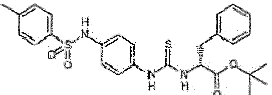 | 526 | 1H NMR (400 MHz, DMSO-d6) δ 10.15 (s, 1H), 9.69 (s, 1H), 7.70 - 7.57 (m, 3H), 7.37 - 7,32 (m, 2H), 7.32 - 7.20 (m, 5H), 7.20 - 7.16 (m, 2H), 7.04 - 6.95 (m, 2H), 5.11 - 4.94 (m, 1H), 3.07 (d, J = 6.6 Hz, 2H), 2.33 (s, 3H), 1.34 (s, 9H). |
| 69 | 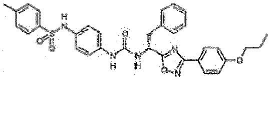 | 612 | 1H NMR (400 MHz, DMSO-d6) δ 9.85 (s, 1H), 8.61 (s, 1H), 7.97 - 7.86 (m, 2H), 7.62 - 7.50 (m, 2H), 7.33 - 7.25 (m, 4H), 7.25 - 7.21 (m, 3H), 7.21 - 7.16 (m, 2H), 7.14 - 7.04 (m, 2H), 6.95 - 6.84 (m, 3H), 5.37 - 5.25 (m, 1H), 4.01 (t, J = 6.5 Hz, 2H), 3.31 - 3.19 (m, 2H), 2.31 (s, 3H), 1.75 (q, J = 7.0 Hz, 2H), 0.99 (t, J = 7.4 Hz, 3H). |
| 70 | 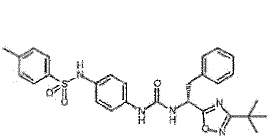 | 534 | 1H NMR (400 MHz, DMSO-d6) δ 9.86 (s, 1H), 8.58 (s, 1H), 7.62 - 7.50 (m, 2H), 7.33 - 7.10 (m, 9H), 6.96 - 6.88 (m, 2H), 6.83 (d, J = 8.1 Hz, 1 H), 5.33 - 5.19 (m, 1H), 3.17 (d, J = 7.2 Hz, 2H), 2.32 (s, 3H), 1.27 (s, 9H). |

**[Table 3-15]**

| comp. No. | structure | MS (ESI) m/z (M+H)⁺ | NMR |
|---|---|---|---|
| 71 | 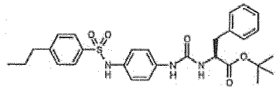 | 538 | not measured |
| 72 | 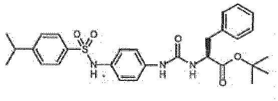 | 538 | not measured |
| 73 | 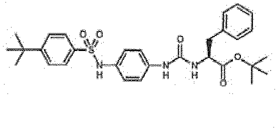 | 552 | not measured |
| 74 | 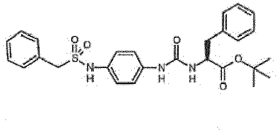 | 510 | not measured |
| 75 | 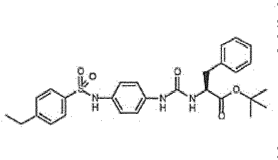 | 524 | not measured |

**[Table 3-16]**

| comp. No. | structure | MS (ESI) m/z (M+H)⁺ | NMR |
|---|---|---|---|
| 76 | 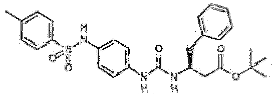 | 524 | 1H NMR (400 MHz, DMSO-d6) δ 9.81 (s, 1H), 8.38 (s, 1H), 7.61 - 749 (m, 2H), 7.36 - 7.25 (m, 4H), 7.25 - 7.11 (m, 5H), 6.96 - 6.83 (m, 2H), 6.06 (d, J = 8.6 Hz, 1H), 4.22 - 4.07 (m, 1H), 2.75 (d, J = 6.8 Hz, 2H), 2.41 - 2.23 (m, 5H), 1.37 (s, 9H). |
| 77 | 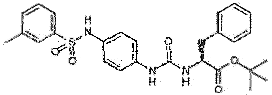 | 510 | 1H NMR (400 MHz, DMSO-d6) δ 9.88 (s, 1H), 8.60 (s, 1H), 7.54 - 7.50 (m, 1H), 7.50 - 7.43 (m, 1 H), 7.42 - 7.37 (m, 2H), 7.34- 7.26 (m, 2H), 7.27 - 7.15 (m, 5H), 6.96 - 6.87 (m, 2H), 6.32 (d, J = 8.0 Hz, 1H), 4.41 - 4.31 (m, 1H), 2.95 (d, J = 7.4 Hz, 2H), 2.33 (s, 3H), 1.33 (s, 9H). |
| 78 | 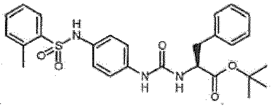 | 510 | not measured |
| 79 | 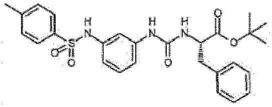 | 510 | 1H NMR (400 MHz, DMSO-d6) δ 10.13 (s, 1H), 8.70 (s, 1H), 7.70 - 7.59 (m, 2H), 7.36 - 7.28 (m, 4H), 7,27 - 7.16 (m, 4H), 7.12 - 6.98 (m, 2H), 6.69 - 6.56 (m, 1H), 6.29 (d, J = 8.0 Hz, 1H), 4.45 - 4.31 (m, 1H), 3.00 - 2.93 (m, 2H), 2.32 (s, 3H), 1.35 (s, 9H). |
| 80 | 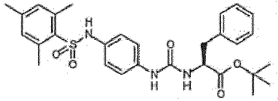 | 538 | 1H NMR (400 MHz, DMSO-d6) δ 9.72 (s, 1H), 8.58 (s, 1H), 7.32 - 7.26 (m, 2H), 7.25 - 7.16 (m, 5H), 7.01 - 6.93 (m, 2H), 6.87 - 6.79 (m, 2H), 6.32 (d, J = 8.0 Hz, 1H), 4.43 - 4.29 (m, 1H), 2.94 (d, J = 6.7 Hz, 2H), 2.47 (s, 6H), 2.21 (s, 3H), 1.33 (s, 9H). |

**[Table 3-17]**

| comp. No. | structure | NS (ESI) m/z (M+H)⁺ | NMR |
|---|---|---|---|
| 81 | 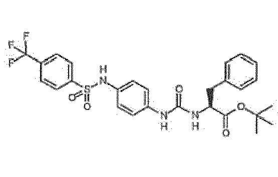 | 564 | 1H NMR (400 MHz, DMSO-D6) δ 10.15 (s, 1H), 8.65 (s, 1H), 7.94 (d, J = 8.5 Hz, 2H), 7.87 (d, J = 8.3 Hz, 2H), 7.33 - 7.26 (m, 2H), 7.26 - 7.17 (m, 5H), 6,99 - 6.84 (m, 2H), 6.33 (d, J = 8.0 Hz, 1H), 4.47 - 4.29 (m, 1H), 2.97 - 2.93 (m, 2H), 1.34 (s, 9H). |
| 82 | 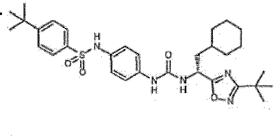 | 582 | 1H NMR (400 MHz, DMSO-d6) δ 9.95 (s, 1H), 8.50 (s, 1H), 7.68 - 7.61 (m, 2H), 7.59 - 7.51 (m, 2H), 7.27 - 7.19 (m, 2H), 7.01 - 6.93 (m, 2H), 6.86 (d, J = 8.1 Hz, 1H), 5.10 - 4.98 (m, 1H), 1.84 - 1.54 (m, 6H), 1.29 (s, 9H), 1.26 (s, 9H), 1.39 - 1.04 (m, 5H), 1.03 - 0.83 (m, 2H). |
| 83 | 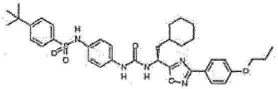 | 660 | 1H NMR (400 MHz, DMSO-d6) δ 9.94 (s, 1H), 8.55 (s, 1H), 7.98 - 7.85 (m, 2H), 7.69 - 7.60 (m, 2H), 7.59 - 7.48 (m, 2H), 7.30 - 7.19 (m, 2H), 7.13 - 7.03 (m, 2H), 7.00 - 6.88 (m, 3H), 5.14 - 5.02 (m, 1H), 4.00 (t, J = 6.5 Hz, 2H), 1.90 - 1.55 (m, 9H), 1.44 - 1.32 (m, 1H), 1.25 (s, 9H), 1.22 - 1.07 (m, 3H), 1.05 - 0.88 (m, 5H). |
| 84 | 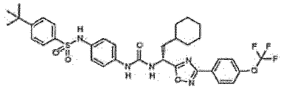 | 686 | 1H NMR (400 MHz, DMSO-d6) δ 9.95 (s, 1H), 8.58 (s, 1H), 8.17 - 8.07 (m, 2H), 7.6B - 7.60 (m, 2H), 7.59 - 7.52 (m, 4H), 7.30 - 7.21 (m, 2H), 6.97 (dd, J = 8.4, 3.4 Hz, 3H), 5.18 - 5.07 (m, 1H), 191 - 1.73 (m, 3H), 1.72 - 1.56 (m, 4H), 1.47 - 1.33 (m, 1H), 1.25 (s, 9H), 1.21 - 1.07 (m, 3H), 1.07 - 0.84 (m, 2H). |
| 85 | 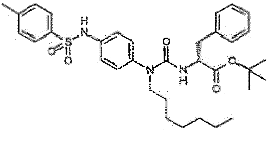 | 608 | 1H NMR (400 MHz, DMSO-d6) δ 8.74 (s, 1H), 7.38-7.33 (m, 2H), 7.33 - 7.28 (m, 2H), 7.28 - 7.20 (m, 4H), 7.20 - 7.13 (m, 3H), 6.83 - 6.75 (m, 2H), 6.35 (d, J = 8.0 Hz, 1H), 4.38 - 4.28 (m, 1H), 2.97 - 2.85 (m, 2H), 2.32 (s, 3H), 1.28 (s, 9H), 1.25 - 1.04 (m, 12H), 0.76 (t, J = 6.9 Hz, 3H). |

**[Table 3-18]**

| comp. No. | structure | MS (ESI) m/z (M+H)⁺ | NMR |
|---|---|---|---|
| 86 | 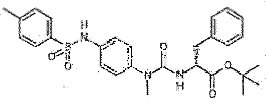 | 524 | 1H NMR (400 MHz, DMSO-d6) δ 8.79 (s, 1H), 7.38 (s, 4H), 7.35 - 7.18 (m, 7H), 6.94 - 6.88 (m, 2H), 6.40 (d, J = 8.0 Hz, 1H), 4.44 - 4.34 (m, 1H), 3.05 (s, 3H), 3.01 - 2.92 (m, 2H), 2.39 (s, 3H), 1.35 (s, 9H). |
| 87 | 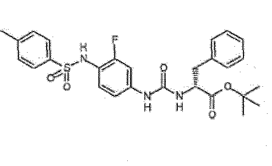 | 528 | 1H NMR (400 MHz, DMSO-d6) δ 9.75 (s, 1H), 8.90 (s, 1H), 7.61 - 7.50 (m, 2H), 7.39 - 7.28 (m, 5H), 7.28 - 7.15 (m, 3H), 7.02 (t, J = 8.8 Hz, 1H), 6.94 - 6.84 (m, 1H), 6.45 (d, J = 7.9 Hz, 1H), 4.45 - 4.31 (m, 1H), 3.01 - 2.93 (m, 2H), 2.36 (s, 3H), 1.35 (s, 9H). |
| 88 | 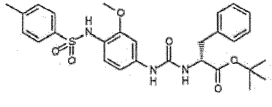 | 540 | not measured |
| 89 | 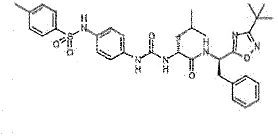 | 647 | not measured |
| 90 | 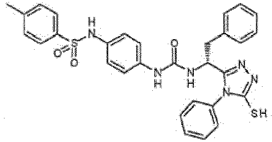 | 585 | not measured |

**[Table 3-19]**

| compound | structure | MS (ESI) m/z (M+H)+ | NMR |
|---|---|---|---|
| 91 | 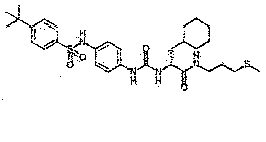 | 589 | 1H NMR (400 MHz, DMSO-d6) δ 9.90 (s, 1 H), 8.49 (s, 1H), 8.07 (t, J = 5.7 Hz, 1H), 7.67 - 7.59 (m, 2H), 7.58 - 7.51 (m, 2H), 7.24 - 7.18 (m, 2H), 6.98 - 6.91 (m, 2H), 6.23 (d, J = 8.4 Hz, 1H), 4.16 (m, 1H), 3.23 - 3.01 (m, 2H), 2.44 (t, J = 7.3 Hz, 2H), 2.01 (s, 3H), 1.97 - 1.54 (m, 8H), 1.50 - 1.30 (m, 2H), 1.26 (s, 9H), 1.20 - 1.06 (m, 3H), 0.94 - 0.79 (m, 2H). |
| 92 | 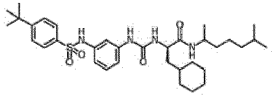 | 613 | not measured |
| 93 | 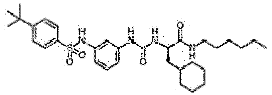 | 585 | not measured |
| 94 | 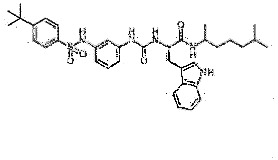 | 646 | 1H NMR (400 MHz, DMSO-d6) δ 10.81 (d, J = 2.9 Hz, 1H), 10.17 (s, 1H), 8.69 (d. J = 5.4 Hz, 1H), 7.80 (dd, J = 26.6, 8.2 Hz, 1H), 7.73 - 7.68 (m, 2H), 7.59 - 7.52 (m, 3H), 7.34 - 7.28 (m, 1H), 7.23 - 7.18 (m, 1H), 7.11 - 6.99 (m, 4H), 6.97 - 6.90 (m, 1H), 6.69 - 6.60 (m, 1H), 6.25 (t, J = 7.7 Hz, 1H), 4.52 - 4.38 (m, 1H), 3.78 - 3.62 (m, 1H), 3.12 - 3.01 (m, 1 H), 2.99 - 2.86 (m, 1H), 1.52 - 1.37 (m, 1H), 1.35 - 1.03 (m, 15H), 1.00 (d, J = 6.6 Hz, 1.5H), 0.89 (d, J = 6.6 Hz, 1.5H), 0.80 (dd, J = 6.6, 5.5 Hz, 6H). |
| 95 | 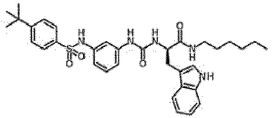 | 618 | 1H NMR (400 MHz, DMSO-d6) δ 10.82 (d, J = 2.4 Hz, 1H), 10.17 (s, 1H), 8.70 (s, 1H), 7.97 (t, J = 5.6 Hz, 1H), 7.77 - 7.66 (m, 2H), 7.63 - 7.50 (m, 3H), 7.39 - 7.28 (m, 1H), 7.24 - 7.18 (m, 1H), 7.10 - 6.99 (m, 4H), 6.97 - 6.91 (m, 1H), 6.67 - 6.57 (m, 1H), 6.25 (d, J = 8.0 Hz, 1H), 4.51 - 4.38 (m, 1H), 3.12 - 2.90 (m, 4H), 1.25 (s, 17H), 0.83 (t, J = 6.9 Hz, 3H). |

**[Table 3-20]**

| compound | structure | MS (ESI) m/z (M+H)+ | NMR |
|---|---|---|---|
| 96 | 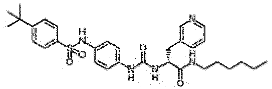 | 580 | 1H NMR (400 MHz, DMSO-d6) δ 9.93 (s, 1H), 8.82 - 8.67 (m, 3H), 8.36 - 8.23 (m, 1H), 8.15 (t, J = 5.7 Hz, 1H), 7.93 - 7.84 (m, 1H), 7.62 (d, J = 8.2 Hz, 2H), 7.54 (d, J = 8.3 Hz, 2H), 7.14 (d, J = 8.4 Hz, 2H), 6.93 (d, J = 8.4 Hz, 2H), 6.55 (d, J = 8.4 Hz, 1H), 4.59 - 4.42 (m, 1H), 3.14 (dd, J = 13.8, 5.4 Hz, 1H), 3.07 - 2.92 (m, 3H), 1.46 - 1.09 (m, 17H), 0.84 (t, J = 6.7 Hz, 3H). |
| 97 | 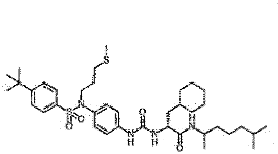 | 702 | 1H NMR (400 MHz, DMSO-d6) δ 8.73 (d, J = 10.1 Hz, 1H), 7.87 (dd, J = 15.4, 8.4 Hz, 1H), 7.65 - 7.55 (m, 2H), 7.51 - 7.44 (m, 2H), 7.37 - 7.29 (m, 2H), 6.87 (dd, J = 8.8, 4.0 Hz, 2H), 6.31 (dd, J = 8.6, 4.4 Hz, 1H), 4.26 - 4.14 (m, 1H), 3.81 - 3.68 (m, 1H), 3.60 - 3.41 (m, 2H), 2.43 (t, J = 7.2 Hz, 2H), 1.95 (d, J = 0.6 Hz, 3H), 1.82 - 1.08 (m, 29H). 1.07 - 0.96 (m, 3H), 0.94 - 0.76 (m, 8H). |
| 98 | 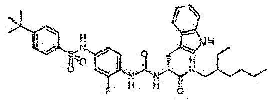 | 664 | 1H NMR (400 MHz, DMSO-d6) δ 10.80 (d, J = 2.3 Hz, 1H), 10.17 (s, 1H), 8.43 - 8.35 (m, 1H), 7.97 - 7.80 (m, 2H), 7.71 - 7.60 (m, 2H), 7.60 - 7.50 (m, 3H), 7.30 (d, J = 8.0 Hz, 1H), 7.08 (d, J = 2.3 Hz, 1H), 7.07 - 7.01 (m, 1H), 6.94 (t, J = 7.6 Hz, 1H), 6.90 (dd, J = 12.8, 2.4 Hz, 1H), 6.82 (d, J = 8.0 Hz, 1H), 6.76 (dd, J = 9.3, 2.3 Hz, 1H), 4.54 - 4.41 (m, 1H), 3.15 - 2.85 (m, 4H), 1.41 - 1.05 (m, 18H), 0.88 - 0.70 (m, 6H). |
| 99 | 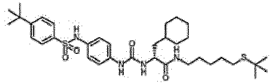 | 659 | not measured |
| 100 | 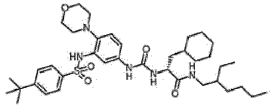 | 698 | not measured |

**[Table 3-21]**

| compound | structure | MS (ESI) m/z (M+H)+ | NMR |
|---|---|---|---|
| 101 | 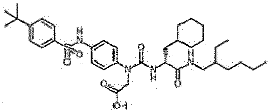 | 671 | 1H NMR (400 MHz, DMSO-d6) δ 12.80 (s, 1H), 8.68 (s, 1H), 7.97 (t, J = 5.8 Hz, 1H), 7.70 - 7.50 (m, 4H), 7.31 - 7.25 (m, 2H), 7.02 - 6.96 (m, 2H), 6.31 (d, J = 8.4 Hz, 1H), 4.31 (s, 2H), 4.28 - 4.18 (m, 1H), 3.16 - 3.04 (m, 1H), 2.94 - 2.83 (m, 1H), 1.81 - 1.53 (m, 5H), 1.50 - 1.33 (m, 3H), 1.32 - 1.07 (m, 21H), 0.96 - 0.77 (m, 8H). |
| 102 | 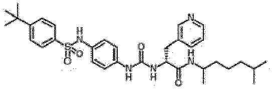 | 608 | 1H NMR (400 MHz, DMSO-d6) δ 9.93 (s, 1H), 8.74 - 8.62 (m, 3H), 8.17 (d, J = 8.0 Hz, 1H), 7.97 (dd, J = 23.7, 8.1 Hz, 1H), 7.84 - 7.75 (m, 1H), 7.66 - 7.59 (m, 2H), 7.58 - 7.51 (m, 2H), 7.20 - 7.11 (m, 2H), 6.97 - 6.90 (m, 2H), 6.51 (d, J = 8.4 Hz, 1H), 4.57 - 4.41 (m, 1H), 3.78 - 3.64 (m, 1H), 3.15 - 3.03 (m, 1H), 3.01 - 2.88 (m, 1H), 1.54 - 1.38 (m, 1H), 1.26 (s, 15H), 1.02 (d, J = 6.6 Hz, 1.5H), 0.93 (d, J = 6.6 Hz, 1.5H), 0.83 (d, J = 6.5 Hz, 3H), 0.79 (m, 3H). |
| 103 | 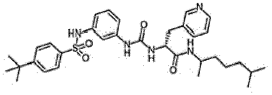 | 608 | 1H NMR (400 MHz, DMSO-d6) δ 10.19 (s, 1H), 8.79 (d, J = 3.7 Hz, 1H), 8.75 - 8.62 (m, 2H), 8.18 (d, J = 7.9 Hz, 1H), 7.99 (dd, J = 22.7, 8.2 Hz, 1H), 7.89 - 7,76 (m, 1H), 7.75 - 7,66 (m, 2H), 7.61 - 7.52 (m, 2H), 7.21 - 7.14 (m, 1H), 7.09 - 6.95 (m, 2H), 6.68 - 6.59 (m, 1H), 6.49 (d, J = 8.4 Hz, 1H), 4.59 - 4.44 (m, 1H), 3.77 - 3.64 (m, 1H), 3.17 - 3.04 (m, 1H), 3.03 - 2.90 (m, 1H), 1.57 - 1.38 (m, 1H), 1.37 - 1.05 (m, 15H), 1.03 (d, J = 6.6 Hz, 1.5H), 0.94 (d, J = 6.6 Hz, 1.5H), 0.86 - 0.82 (m, 3H), 0.82 - 0.77 (m, 3H). |
| 104 | 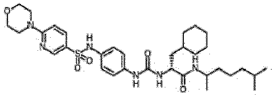 | 643 | 1H NMR (400 MHz, DMSO-d6) δ 9.75 (d, J = 1.6 Hz, 1H), 8.59 (d, J = 8.0 Hz, 1H), 8.28 (d, J = 2.5 Hz, 1H), 7.83 (dd, J = 16.7, 8.3 Hz, 1H), 7.66 (dd, J = 9.1, 2.6 Hz, 1H), 7.26 - 7.16 (m, 2H), 6.97 - 6.91 (m, 2H), 6.87 (d, J = 9.2 Hz, 1H), 6.25 (d, J = 8.5 Hz, 1H), 4.23 - 4.08 (m, 1H), 3.80 - 3.67 (m, 1H), 3.61 - 350 (m, 4H), 1.80 - 1.54 (m, 6H), 1.52 - 1.03 (m, 16H), 1.03 - 0.95 (m, 3H), 0.94 - 0.73 (m, 8H). |
| 105 | 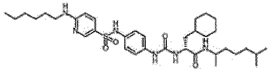 | 657 | 1H NMR (400 MHz, DMSO-d6) δ 9.64 (d, J = 1.8 Hz, 1H), 8.52 (d, J = 9.1 Hz, 1H), 8.14 (d, J = 2.5 Hz, 1H), 7.83 (dd, J = 15.9, 8.3 Hz, 1H), 7.59 - 7.45 (m, 2H), 7.28 - 7.17 (m, 2H), 6.98 - 6.88 (m, 2H), 6.48 (dd, J = 9.2, 1.2 Hz, 1H), 6.21 (dd, J = 8.4, 3.8 Hz, 1H), 4.24 - 4.12 (m, 1H), 3.78 - 3.67 (m, 1H), 3.27 - 3.16 (m, 2H), 1.82 - 1.53 (m, 6H), 1.53 - 1.04 (m, 22H), 1.00 (t, J = 7.0 Hz, 3H), 0.91 - 0.81 (m, 6H), 0.79 (d, J = 1.7 Hz, 1.5H), 0.78 (d, J = 1.7 Hz, 1.5H). |

**[Table 3-22]**

| compound | structure | MS (ESI) m/z (M+H)+ | NMR |
|---|---|---|---|
| 106 | 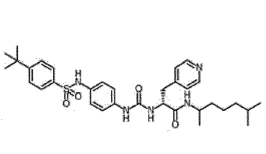 | 608 | 1H NMR (400 MHz, DMSO-d6) δ 9.93 (d, J = 1.5 Hz, 1H), 8.84 - 8.74 (m, 2H), 8.68 (s, 1H), 8.01 (dd, J = 21.3, 8.2 Hz, 1H), 7.84 - 7.75 (m, 2H), 7.66 - 7.60 (m, 2H), 7.57 - 7.51 (m, 2H), 7.20 - 7.10 (m, 2H), 6.96 - 6.90 (m, 2H), 6.52 (dd, J = 8.6, 1.7 Hz, 1H), 4.65 - 4.47 (m, 1H), 3.79 - 3.64 (m, 1H), 3.23 - 3.12 (m, 1H), 3.09 - 2.99 (m, 1H), 1.53 - 1.05 (m, 16H), 1.03 (d, J = 6.6 Hz, 1.5H), 0.95 (d, J = 6.6 Hz, 1.5H), 0.82 (dd, J = 6.6, 0.8 Hz, 3H), 0.79 (dd, J = 6.6, 1.2 Hz, 3H). |
| 107 | 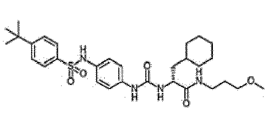 | 573 | 1H NMR (400 MHz, DMSO-d6) δ 9.90 (s, 1H), 8.49 (s, 1H), 8.02 (t, J = 5.6 Hz, 1H), 7.67 - 7.59 (m, 2H), 7.59 - 7.50 (m, 2H), 7.25 - 7.14 (m, 2H), 6.99 - 6.89 (m, 2H), 6.22 (d, J = 8.4 Hz, 1H), 4.24 - 4.08 (m, 1H), 3.29 (t, J = 5.6 Hz, 2H), 3.19 (s, 3H), 3.17 - 2.96 (m, 2H), 1.80 - 1.54 (m, 8H), 1.49 - 1.31 (m, 2H), 1.26 (s, 9H), 1.22 - 1.02 (m, 3H), 0.97 - 0.78 (m, 2H). |
| 108 | 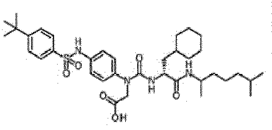 | 671 | not measured |
| 109 | 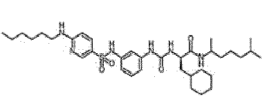 | 657 | 1H NMR (400 MHz, DMSO-d6) δ 9.98 (d, J = 1.6 Hz, 1H), 8.64 (d, J = 5.5 Hz, 1H), 8.26 (d, J = 2.4 Hz, 1H), 7.86 (dd, J = 19.4, 8.3 Hz, 1H), 7.66 (brs, 1H), 7.62 - 7.56 (m, 1H), 7.19 (dt, J = 12.6, 1.9 Hz, 1H), 7.11 - 6.97 (m, 2H), 6.61 (dt, J = 6.8, 2.1 Hz, 1H), 6.52 (d, J = 9.1 Hz, 1H), 6.22 (d, J = 8.5 Hz, 1H), 4.29 - 4.09 (m, 1H), 3.83 - 3.87 (m, 1H), 3.30 - 3.19 (m, 2H), 1.81 - 1.54 (m, 6H), 1.53 - 1.05 (m, 21H), 1.01 (dd, J = 8.9, 6.6 Hz, 3H), 0.94 - 0.75 (m, 11H). |
| 110 | 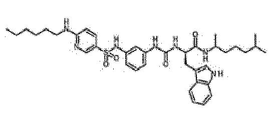 | 690 | not measured |

**[Table 3-23]**

| compound | structure | MS (ESI) m/z (M+H)+ | NMR |
|---|---|---|---|
| 111 | 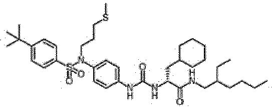 | 702 | 1H NMR (400 MHz, DMSO-d6) δ 8.75 (s, 1H), 7.97 (t, J = 5.9 Hz, 1H), 7.64 - 7.56 (m, 2H), 7.52 - 7.45 (m, 2H), 7.38 - 7.29 (m, 2H), 6.93 - 6.83 (m, 2H), 6.34 (d, J = 8.4 Hz, 1H), 4.32 - 4.17 (m, 1H), 3.61 - 3.50 (m, 2H), 3.16 - 3.05 (m, 1H), 2.98 - 2.84 (m, 1H), 2.44 (t, J = 7.2 Hz, 2H), 1.95 (s, 3H), 1.80 - 1.50 (m, 7H), 1.50 - 1.01 (m, 24H), 0.96 - 0.78 (m, 8H). |
| 112 | 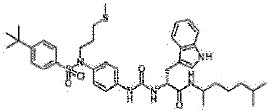 | 735 | 1 H NMR (400 MHz, DMSO-d6) δ 10.82 (d, J = 2.4 Hz, 1H), 8.81 (s, 1H), 7.82 (dd, J = 20.5, 8.3 Hz, 1H), 7.63 - 752 (m, 3H), 7.52 - 7.42 (m, 2H), 7.37 - 7.26 (m, 3H), 7.10 (d, J = 2.4 Hz, 1H), 7.04 (ddd, J = 8.1, 7.0, 1.2 Hz, 1H), 6.98 - 6.91 (m, 1H), 6.90 - 6.83 (m, 2H), 6.41 - 6.32 (m, 1 H), 4,53 - 4.38 (m, 1H), 3.79 - 3.64 (m, 1H), 3.59 - 3.49 (m, 2H), 3.15 - 3.01 (m, 1H), 3.00 - 2.90 (m, 1H), 2.43 (t, J = 7.2 Hz, 2H), 1.94 (s, 3H), 1.61 - 1.50 (m, 2H), 1.49 - 1.38 (m, 1H), 1.35 - 1.02 (m, 15H), 1.00 (d, J = 6.6 Hz, 1.5H), 0.90 (d, J = 6.6 Hz, 1.5H), 0.83 - 0.77 (m, 6H). |
| 113 | 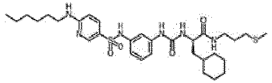 | 633 | 1H NMR (400 MHz, DMSO-d6) δ 10.00 (s, 1H), 8.64 (s, 1H), 8.25 (d, J = 2.4 Hz, 1H), 8.10 (t, J = 5.7 Hz, 1H), 7.73 (brs, 1H), 7.61 (d, J = 9.3 Hz, 1H), 7.20 (t, J = 1.9 Hz, 1H), 7.12 - 7.00 (m, 2H), 6.62 (dt, J = 7.0, 2.0 Hz, 1H), 6.55 (d, J = 9.1 Hz, 1H), 6.25 (d, J = 8.4 Hz, 1H), 4.25 - 4.12 (m, 1H), 3.29 - 3.13 (m, 3H), 3.13 - 3.03 (m, 1H), 2.44 (t, J = 7.3 Hz, 2H), 2.01 (s, 3H), 1.78 - 1.54 (m, 7H), 1.54 - 1.07 (m, 14H), 0.97 - 0.79 (m, 5H). |
| 114 | 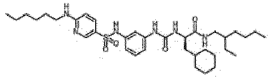 | 657 | 1H NMR (400 MHz, DMSO-d6) δ 9.97 (s, 1H), 8.63 (s, 1H), 8.26 (d, J = 2.5 Hz, 1H), 7.97 (t, J = 5.9 Hz, 1H), 7.73 - 7.54 (m, 2H), 7.20 (t, J = 1.9 Hz, 1H), 7.12 - 6.99 (m, 2H), 6.62 (dt, J = 7.3, 1.9 Hz, 1H), 6.52 (d, J = 9.0 Hz, 1H), 6.23 (d, J = 8.4 Hz, 1H), 4.29 - 4.19 (m, 1H), 3.30 - 3.18 (m, 2H), 3.16 - 3.04 (m, 1H), 2.97 - 2.83 (m, 1H), 1.80 - 1.54 (m, 5H), 1.53 - 1.07 (m, 23H), 0.95 - 0.77 (m, 11H). |

Formulation Example 1 (production of capsule)

| | |
|---|---|
| 1) Example compound | 10 mg |
| 2) fine powder cellulose | 10 mg |
| 3) lactose | 19 mg |
| 4) magnesium stearate | 1 mg |
| total | 40 mg |

1), 2), 3) and 4) are mixed and filled in a gelatin capsule.

Formulation Example 2 (production of tablet)

| | |
|---|---|
| 1) Example compound | 10 g |
| 2) lactose | 50 g |
| 3) cornstarch | 15 g |
| 4) carboxymethylcellulose calcium | 44 g |
| 5) magnesium stearate | 1 g |
| 1000 tablets total | 120 g |

The total amount of 1), 2), 3) and 30 g of 4) are kneaded with water, vacuum dried and sieved. The sieved powder is mixed with 14 g of 4) and 1 g of 5), and the mixture is punched by a tableting machine. In this way, 1000 tablets containing 10 mg of the Example compound per tablet are obtained.

Test Example 1: GLP-1 receptor in vitro calcium assay (intracellular free calcium concentration change induced via GLP-1 receptor)

PEAK^{RAPID}-derived Gα15-trans48 LD stably expressing cells (PRG48) maintained in DMEM/Ham's F-12 (Nacalai Tesque) medium containing 10% Fetal bovine serum (NICHIREI CORPORATION) and 1% Pen Strep (GIBCO) were washed with D-PBS(-) (Nacalai Tesque), and the cells were recovered using 0.25% Trypsin EDTA (GIBCO) from a 15 cm dish (CORING). The supernatant was removed by centrifugation (1,200 rpm, 3 min), and the cells were suspended at 4×10⁶ cells/mL in 5% FBS DMEM/Ham's F-12. 1.0 mL thereof was seeded in a 15 cm dish and cultured (37°C, 5% CO₂) overnight. The next day, the medium of the cultured PRG48 cells was replaced with 5% FBS DMEM/Ham's F-12 medium (30 mL), human GLP-1R gene mixture solution (GLP-1R/pc.DNA3.1(+) 60 µg) prepared using 6.0 mL of Opti-MEM (invitrogen) and 120 µL of Lipofectamin 2000 (invitrogen) was slowly added to the cells, and cultured (37°C, 5% CO₂) for 6 hr to introduce the gene. After gene transfer, the cells were washed with D-PBS(-) and recovered by peeling the cells from the flask with 0.25% Trypsin-EDTA (GIBCO). The cells were counted, suspended at 0.5×10⁶ cells/mL in 5% FBS DMEM/Ham's F-12 medium, and seeded in each well of D-Lysine coat 96 well plate (BD Bioscience) at 7.0×10⁴ cells. After static adhesion at room temperature for 1 hr, the cells were cultured overnight (37°C, 5% CO₂). The medium in the Cultured 96 well was entirely discarded, 150 µL of intracellular calcium measurement staining solution Calcium 5 assay kit Express (Molecular Device) diluted 80-fold with Assay buffer (20 mM HEPES, 146 mM NaCl, 1 mM MgSO₄, 1.39 mM glucose, 1 mM CaCl₂, 2.5 mM Probenecid, 0.05% Casein Enzymatic Hydrolysate) was added, and the mixture was stood at 37°C for 30 min, and then at room temperature for 45 min to perform staining. After staining, 250pM GLP-1 and the compound were added and Ca²⁺ response was observed by using FDSSµCELL (Hamamatsu Photonics K.K.). Each evaluation compound was dissolved in DMSO, further diluted with Assay buffer and used.

For evaluation, the fluorescence values (Ex480:Em540) before and after addition were measured, and changes in the intracellular free calcium concentration produced via GLP-1R receptor by the addition were quantitatively examined. For the measurement and analysis of the fluorescence values, a soft (FDSS7000EX) attached to the FDSSµCELL was used, and ΔF/F value ((maximum fluorescence value after stimulation - minimum fluorescence value after stimulation)/fluorescence value before stimulation) was calculated. Using the obtained ΔF/F values, EC₅₀ value was calculated by Prism5.

### Test Example 2: Activity evaluation of the compound of the present invention using isolated rat islets of Langerhans

The islets of Langerhans were isolated from the pancreas of male Wistar rats at around 10 weeks of age, according to a conventional method and using collagenase and Ficoll-Conray solution (Cryobiology, 2006, 52:90-8), and used for the activity evaluation of the compound.

The isolated islets were precultured in glucose-free Krebs Ringer Bicarbonate-Hepes buffer (KRBH:129 mM NaCl, 4.7 mM KCl, 5.0 mM NaHCO₃, 1.2 mM MgSO₄, 12 mM KH₂PO₄, 2.5 mM CaCl₂, 10 mM HEPES, pH 7.4) under the conditions of 37°C, 5% CO₂ for 30 min and seeded in each well of a 24 well plate by 5 islets. After seeding, KRBH containing 2.8 mM or 8.3 mM glucose, 1.0 nM GLP-1 analogue (exendin-4), and 30 µM compound 50 was added, and the mixture was cultured for 1 hr (37°C, 5% CO₂). The culture supernatant was recovered from each well after culturing for 1 hr, and the concentration of insulin secreted in the supernatant was measured using a commercially available measurement kit (manufactured by Morinaga Institute of Biological Science, Inc.). The insulin secretion amounts under respective stimulation conditions were calculated as secretion amounts relative to the secretion amount at 8.3 mM glucose.

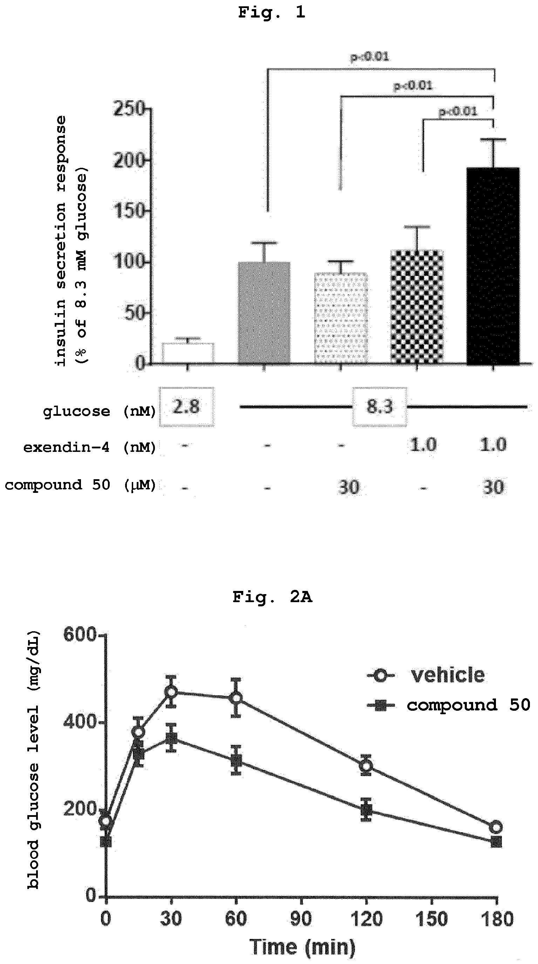

When the glucose concentration in the buffer was raised from 2.8 mM to 8.3 mM, the isolated islets of Langerhans showed about a 5-fold increase in insulin secretion as compared to that at 2.8 mM, whereby it was confirmed that the glucose responsiveness was maintained. Addition of 30 µM compound 50 or GLP-1 analogue exendin-4 (1 nM) alone to a buffer containing 8.3 mM glucose did not affect insulin secretion. However, simultaneous addition of 30 µM compound 50 and 1 nM exendin-4 resulted in an about 2-fold increase in the insulin secretion amount as compared to that with 8.3 mM glucose alone (Fig. 1). From the above, it was confirmed that compound 50 has an activity enhancing the glucose-stimulated insulin secretion promoting action by GLP-1.

The calcium assay results (EC₅₀ (µM) value) of the Example compounds of the present invention and the intensity of GLP-1 receptor action enhancing activity of the Example compounds of the present invention compared to BETP described in non-patent document 1 (activity of BETP as 1) are shown in the following Table 4-1 to Table 4-4.

**[Table 4-1]**

| Compound number (compound name) | Ca EC₅₀ value (µM) | GLP-1 receptor action enhancing activity (vs BETP) |
|---|---|---|
| 1 | 0.0043 | 5817 |
| 2 | 0.68 | 37 |
| 3 | 1.7 | 15 |
| 4 | 1.9 | 13 |
| 5 | 0.051 | 490 |
| 6 | 0.13 | 187 |
| 7 | 0.050 | 505 |
| 8 | 0.50 | 50 |
| 9 | 0.65 | 39 |
| 10 | 0.38 | 67 |
| 11 | 0.49 | 52 |
| 12 | 0.54 | 47 |
| 13 | 1.7 | 15 |
| 14 | 1.8 | 14 |
| 15 | 0.042 | 599 |
| 16 | 0.52 | 49 |
| 17 | 0.52 | 48 |
| 18 | 0.36 | 71 |
| 19 | 0.16 | 154 |
| 20 | 1.5 | 17 |
| 21 | 0.47 | 53 |
| 22 | 0.22 | 116 |
| 23 | 0.37 | 68 |
| 24 | 0.85 | 30 |
| 25 | 1.4 | 18 |
| 26 | 1.1 | 24 |
| 27 | 1.1 | 23 |
| 28 | 1.8 | 14 |
| 29 | 0.94 | 27 |
| 30 | 1.2 | 21 |

**[Table 4-2]**

| Compound number (compound name) | Ca EC₅₀ value (µM) | GLP-1 receptor action enhancing activity (vs BETP) |
|---|---|---|
| 31 | 0.48 | 52 |
| 32 | 0.82 | 31 |
| 33 | 1.5 | 17 |
| 34 | 0.064 | 391 |
| 35 | 0.66 | 38 |
| 36 | 0.018 | 1425 |
| 37 | 0.18 | 140 |
| 38 | 0.030 | 852 |
| 39 | 0.023 | 1104 |
| 40 | 0.13 | 199 |
| 41 | 0.076 | 333 |
| 42 | 0.092 | 274 |
| 43 | 0.16 | 160 |
| 44 | 1.3 | 19 |
| 45 | 0.022 | 1149 |
| 46 | 0.016 | 1553 |
| 47 | 0.12 | 208 |
| 48 | 0.28 | 92 |
| 49 | 0.010 | 2534 |
| 50 | 0.0068 | 3720 |
| 51 | 0.0090 | 2786 |
| 52 | 0.17 | 144 |
| 53 | 0.28 | 91 |
| 54 | 0.18 | 137 |
| 55 | 0.045 | 558 |
| 56 | 0.0038 | 6569 |
| 57 | 0.0034 | 7462 |
| 58 | 0.0046 | 5436 |
| 59 | 0.0057 | 4398 |
| 60 | 0.045 | 560 |

**[Table 4-3]**

| Compound number (compound name) | Ca EC₅₀ value (µM) | GLP-1 receptor action enhancing activity (vs BETP) |
|---|---|---|
| 61 | 0.015 | 1710 |
| 62 | 0.93 | 27 |
| 63 | 1.9 | 13 |
| 64 | 1.6 | 15 |
| 65 | 2.0 | 13 |
| 66 | 0.74 | 34 |
| 67 | 0.66 | 38 |
| 68 | 1.2 | 22 |
| 69 | 0.024 | 1068 |
| 70 | 1.3 | 20 |
| 71 | 0.40 | 64 |
| 72 | 0.68 | 37 |
| 73 | 0.31 | 83 |
| 74 | 1.9 | 14 |
| 75 | 0.62 | 41 |
| 76 | 1.6 | 16 |
| 77 | 1.6 | 16 |
| 78 | 1.7 | 15 |
| 79 | 1.5 | 17 |
| 80 | 0.38 | 67 |
| 81 | 1.0 | 24 |
| 82 | 0.016 | 1606 |
| 83 | 0.0043 | 5794 |
| 84 | 0.0034 | 7358 |
| 85 | 0.0070 | 3578 |
| 86 | 0.43 | 58 |
| 87 | 1.1 | 23 |
| 88 | 1.3 | 20 |
| 89 | 0.26 | 98 |
| 90 | 1.4 | 18 |
| BETP (Comparative Example) | 25 | 1 |

**[Table 4-4]**

| Compound number (compound name) | Ca EC₅₀ value (µM) | GLP-1 receptor action enhancing activity (vs BETP) |
|---|---|---|
| 91 | 0.056 | 446 |
| 92 | 0.024 | 1028 |
| 93 | 0.045 | 555 |
| 94 | 0.18 | 140 |
| 95 | 0.25 | 101 |
| 96 | 0.60 | 42 |
| 97 | 0.063 | 399 |
| 98 | 0.072 | 350 |
| 99 | 0.15 | 169 |
| 100 | 0.050 | 503 |
| 101 | 0.020 | 1272 |
| 102 | 0.13 | 193 |
| 103 | 0.13 | 195 |
| 104 | 0.48 | 53 |
| 105 | 0.44 | 56 |
| 106 | 0.39 | 64 |
| 107 | 0.72 | 35 |
| 108 | 0.0015 | 17107 |
| 109 | 0.011 | 2311 |
| 110 | 0.0044 | 5731 |
| 111 | 0.0033 | 7694 |
| 112 | 0.011 | 2206 |
| 113 | 0.028 | 898 |
| 114 | 0.030 | 850 |
| BETP (Comparative Example) | 25 | 1 |

### Test Example 3: Blood glucose increase suppressive effect of the compound of the present invention

23-week-old male KKAy mice fasted overnight were randomly divided into 2 groups, and whey protein was orally administered to each mouse at 3 g/kg to induce endogenous GLP-1 secretion. At 30 min after whey protein administration, the compound of the present invention (compound 50) or administration medium (vehicle) was intraperitoneally administered to each mouse at 3 mg/kg, glucose was orally administered immediately thereafter at 2 g/kg, blood samples were collected from the tail vein over time, and the blood glucose level was measured.

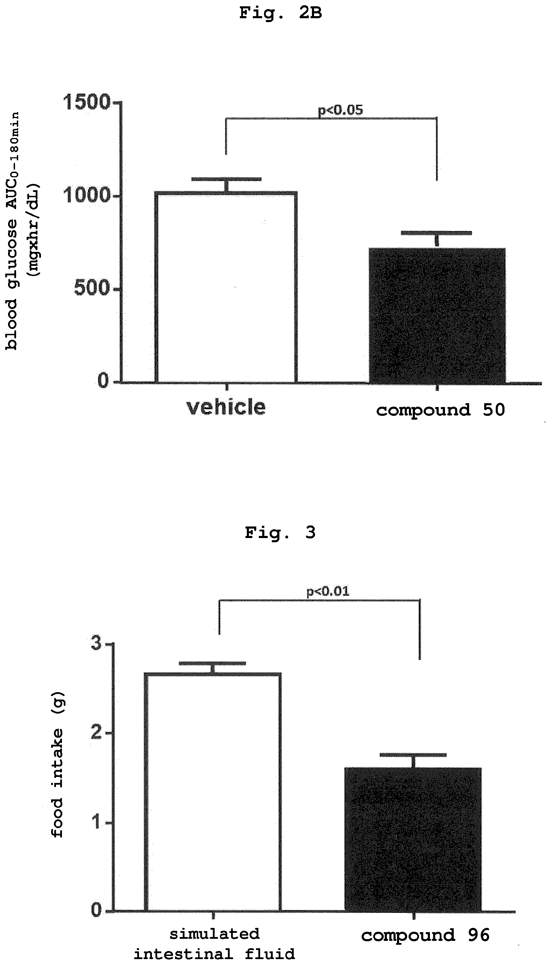

The mouse administered with vehicle showed blood glucose variation with a peak (470.4±34.9 mg/dL) appearing at 30 min after glucose administration. In contrast, the mouse administered with the compound of the present invention (compound 50) showed suppression of blood glucose increase at the time of peak (Fig. 2A), and a significant decrease in the blood glucose AUC value at 180 min after glucose administration (administration vehicle group: 1016±74.8 mg.hr/dL, compound 50 administration group: 734.3±65.9 mg.hr/dL, p<0.05) (Fig. 2B).

From the above results, it was confirmed that the compound of the present invention (compound 50) having a GLP-1 receptor action enhancing activity has an action to suppress blood glucose increase.

### Test Example 4: Food intake-suppressive effect of the compound of the present invention

11-week-old male KKAy mice were randomly divided into 2 groups. After fasting overnight, simulated intestinal fluid (containing 10 mM taurocholic acid, 2 mM lecithin, 55 mM maleic acid, 81.6 mM sodium hydroxide, 125 mM sodium chloride), or the compound of the present invention (compound 96) dissolved in the simulated intestinal fluid was orally administered (3 mg/kg), a commercially available feed (CRF-1, manufactured by Oriental Yeast Co., ltd.) was given and changes in the food intake was monitored.

Cumulative food consumption was measured for 4 hr after administration of compound 96. The compound 96 administration group showed significant suppression as compared to the simulated intestinal fluid administration group (Fig. 3). Therefrom it was confirmed that the compound of the present invention (compound 96) having a GLP-1 receptor action enhancing activity shows an anorectic action by oral administration.

### [Industrial Applicability]

The compound of the present invention has a superior GLP-1 receptor action enhancing activity and permits oral administration. Therefore, the compound is extremely useful as a prophylactic or therapeutic agent for diabetes and obesity, as well as diseases related thereto (e.g., diabetic complication etc.).

This application is based on a patent application No. 2016-067130 filed in Japan, the contents of which are incorporated in full herein.

## Claims

1. A compound represented by the formula (I): 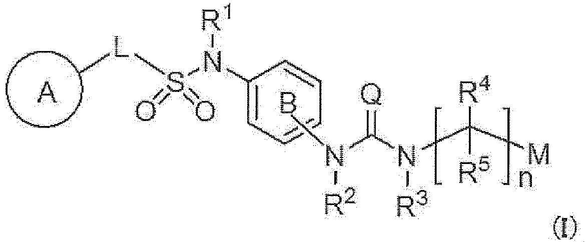 wherein
A is an optionally further substituted cyclic group;
B is an optionally further substituted benzene ring;
L is a bond or an optionally substituted C₁₋₆ alkylene group;
R¹ is a hydrogen atom, a hydroxy group, an optionally substituted C₁₋₆ alkoxy group, an optionally substituted amino group or an optionally substituted C₁₋₆ alkyl group;
R² and R³ are the same or different and are each independently a hydrogen atom, a carboxy group, a hydroxy group, an optionally substituted C₁₋₁₀ alkoxy group, an optionally substituted amino group or an optionally substituted C₁₋₁₀ alkyl group;
Q is an oxygen atom or a sulfur atom;
R⁴ and R⁵ are the same or different and are each independently a hydrogen atom or an optionally substituted C₁₋₆ alkyl group, or R⁴ and R⁵ form a ring together with the carbon atom bonded thereto;
n is an integer of 1 - 6; and
M is an optionally substituted C₁₋₁₂ alkoxy-carbonyl group, an optionally substituted carbamoyl group or an optionally substituted heterocyclic group, excluding 3-(3-(3-(phenylsulfonamide)phenyl)ureido)-4-(trimethylammonio)butanoate, or a salt thereof.

2. The compound according to claim 1, wherein
A is a C₆₋₁₄ aryl group or a 5- or 6-membered monocyclic aromatic heterocyclic group, each of which is optionally further substituted,
L is a bond or a methylene group,
Q is an oxygen atom,
R⁴ and R⁵ are the same or different and are each independently a hydrogen atom or an optionally substituted C₁₋₆ alkyl group, and
n is 1 or 2, or a salt thereof.

3. The compound according to claim 1, wherein
A is a phenyl group optionally further substituted by the same or different 1 to 5 substituents selected from the group consisting of a halogen atom, a hydroxy group, a carboxy group, a sulfanyl group, an optionally substituted C₁₋₆ alkyl group, an optionally substituted C₁₋₆ alkoxy group, an optionally substituted amino group, a C₁₋₆ alkylthio group optionally substituted by a halogen atom, and a C₁₋₃ alkylenedioxy group, or a pyridyl group optionally further substituted by the same or different 1 to 4 substituents selected from the group consisting of a halogen atom, a hydroxy group, a carboxy group, a sulfanyl group, an optionally substituted C₁₋₆ alkyl group, an optionally substituted C₁₋₆ alkoxy group, an optionally substituted amino group, a 4- to 7-membered monocyclic non-aromatic heterocyclic group, a C₁₋₆ alkylthio group optionally substituted by a halogen atom, and a C₁₋₃ alkylenedioxy group,
B is a benzene ring optionally further substituted by the same or different 1 to 4 substituents selected from the group consisting of a halogen atom, a hydroxy group, a sulfanyl group, an optionally substituted C₁₋₆ alkyl group, an optionally substituted C₁₋₆ alkoxy group, an optionally substituted amino group, a C₁₋₆ alkylthio group optionally substituted by a halogen atom, a C₁₋₃ alkylenedioxy group, and a 4- to 7-membered monocyclic non-aromatic heterocyclic group,
L is a bond or a methylene group,
R¹ is a hydrogen atom, or a C₁₋₆ alkyl group optionally substituted by a substituent selected from the group consisting of a halogen atom, a hydroxy group, a sulfanyl group, a carboxy group, a C₁₋₆ alkoxy group, an amino group optionally mono- or di-substituted by a C₁₋₆ alkyl group, and a C₁₋₆ alkylthio group optionally substituted by a halogen atom,
R² and R³ are the same or different and are each independently a hydrogen atom; a carboxy group; a hydroxy group; or a C₁₋₁₀ alkyl group optionally substituted by a substituent selected from the group consisting of a halogen atom, a hydroxy group, a sulfanyl group, a carboxy group, a C₁₋₆ alkoxy group, an amino group optionally mono- or di-substituted by a C₁₋₆ alkyl group, and a C₁₋₆ alkylthio group optionally substituted by a halogen atom,
Q is an oxygen atom,
R⁴ is a hydrogen atom,
R⁵ is a C₁₋₆ alkyl group optionally substituted by a substituent selected from the group consisting of a halogen atom, a hydroxy group, a carboxy group, a guanidino group, a cyano group, a sulfanyl group, an optionally substituted amino group, an optionally substituted carbamoyl group, a C₁₋₆ alkoxy-carbonyl group optionally substituted by a halogen atom, an optionally substituted C₁₋₆ alkoxy group, a C₁₋₆ alkylthio group optionally substituted by a halogen atom, an optionally substituted C₃₋₁₀ cycloalkyl group, and an optionally substituted aromatic ring group,
n is 1, and
M is an optionally substituted C₁₋₁₂ alkoxy-carbonyl group, an optionally substituted carbamoyl group or an optionally substituted 5- or 6-membered monocyclic aromatic heterocyclic group, or a salt thereof.

4. The compound according to claim 1, wherein
A is a phenyl group optionally further substituted by a C₁₋₆ alkyl group optionally substituted by 1 to 3 substituents selected from the group consisting of a halogen atom, a carboxy group, a hydroxy group, a C₁₋₆ alkoxy-carbonyl group, a C₁₋₆ alkoxy group, an amino group optionally mono- or di-substituted by a C₁₋₆ alkyl group, and a cyano group, or a pyridyl group optionally further substituted by the same or different 1 to 3 substituents selected from the group consisting of an amino group optionally mono- or di-substituted by a C₁₋₆ alkyl group and a 4- to 7-membered monocyclic non-aromatic nitrogen-containing heterocyclic group,
B is a benzene ring optionally further substituted by the same or different 1 to 4 substituents selected from the group consisting of a halogen atom, a C₁₋₆ alkoxy group, and a 4- to 7-membered monocyclic non-aromatic heterocyclic group,
L is a bond,
R¹ is a hydrogen atom, or a C₁₋₆ alkyl group substituted by a C₁₋₆ alkylthio group optionally substituted by a halogen atom,
R² and R³ are the same or different and are each independently a hydrogen atom, or a C₁₋₁₀ alkyl group optionally substituted by a carboxy group,
Q is an oxygen atom,
R⁴ is a hydrogen atom,
R⁵ is a C₁₋₆ alkyl group optionally substituted by a substituent selected from the group consisting of a halogen atom, a hydroxy group, a carboxy group, a guanidino group, a cyano group, a sulfanyl group, an optionally substituted amino group, an optionally substituted carbamoyl group, a C₁₋₆ alkoxy-carbonyl group, an optionally substituted C₁₋₆ alkoxy group, a C₁₋₆ alkylthio group optionally substituted by a halogen atom, an optionally substituted C₃₋₁₀ cycloalkyl group, and an optionally substituted aromatic ring group,
n is 1, and
M is an optionally substituted C₁₋₁₂ alkoxy-carbonyl group; a carbamoyl group optionally mono- or di-substituted by a substituent selected from the group consisting of an optionally substituted C₁₋₈ alkyl group and a C₃₋₁₀ cycloalkyl group; or an optionally substituted 5- or 6-membered monocyclic aromatic heterocyclic group, or a salt thereof.

5. The compound according to claim 1, wherein
A is a phenyl group optionally further substituted by a C₁₋₆ alkyl group optionally substituted by 1 to 3 halogen atoms,
B is a benzene ring without a further substituent,
L is a bond,
R¹ is a hydrogen atom,
R² and R³ are the same or different and are each independently a hydrogen atom or a C₁₋₁₀ alkyl group,
Q is an oxygen atom,
R⁴ is a hydrogen atom,
R⁵ is a C₁₋₄ alkyl group optionally substituted by a substituent selected from the group consisting of an optionally substituted C₃₋₁₀ cycloalkyl group, an optionally substituted phenyl group,
a C₁₋₆ alkylthio group, a 5- or 6-membered monocyclic aromatic nitrogen-containing heterocyclic group, and a 8- to 14-membered fused aromatic nitrogen-containing heterocyclic group,
n is 1, and
M is an optionally substituted C₁₋₁₂ alkoxy-carbonyl group, an optionally substituted carbamoyl group, an optionally substituted triazolyl group, an optionally substituted isoxazolyl group, an optionally substituted oxazolyl group, an optionally substituted oxadiazolyl group or an optionally substituted tetrazolyl group, or a salt thereof.

6. The compound according to claim 1, wherein
A is a phenyl group optionally further substituted by 1 to 3 C₁₋₆ alkyl groups,
B is a benzene ring without a further substituent,
L is a bond,
R¹ is a hydrogen atom,
R² and R³ are the same or different and are each independently a hydrogen atom or a C₁₋₁₀ alkyl group,
Q is an oxygen atom,
R⁴ is a hydrogen atom,
R⁵ is a C₁₋₄ alkyl group optionally substituted by a substituent selected from the group consisting of an optionally substituted C₃₋₁₀ cycloalkyl group, an optionally substituted phenyl group,
a C₁₋₆ alkylthio group, a 5- or 6-membered monocyclic aromatic nitrogen-containing heterocyclic group, and a 8- to 14-membered fused aromatic nitrogen-containing heterocyclic group,
n is 1, and
M is a carbamoyl group mono-substituted by a C₁₋₁₂ alkyl group or an optionally substituted oxadiazolyl group, or a salt thereof.

7. The compound according to any one of claims 1 to 6, wherein the group -N(R²)- binds to B at a para-position relative to the binding position of the group -N(R¹)-, or a salt thereof.

8. A glucagon-like peptide-1 receptor action enhancer comprising the compound according to any one of claims 1 to 7 or a salt thereof.

9. A pharmaceutical composition comprising the compound according to any one of claims 1 to 7 or a salt thereof, and a pharmaceutically acceptable carrier.

10. The pharmaceutical composition according to claim 9, wherein the composition is a prophylactic or therapeutic agent for diabetes, obesity and/or a complication thereof.

11. The pharmaceutical composition according to claim 10, further comprising at least one kind of medicament selected from the group consisting of the dipeptidyl peptidase-4 inhibitor, insulin secretagogue, a-glucosidase inhibitor, insulin resistance improving agent, sodium.glucose conjugated transporter-2 inhibitor, glucagon-like peptide-1 receptor agonist and lipase inhibitor.
